# EUROPEAN PATENT APPLICATION

(11) **EP 4 265 641 A1**
(43) Date of publication of application: **25.10.2023**
(21) Application number: 21905760.1
(22) Date of filing: 15.12.2021
(51) Int. Cl.: C07K 16/40, C07K 16/46, C12N 15/13, C12N 15/63, A61K 39/395

(54) **DEVELOPMENT AND APPLICATION OF COMPLEMENT INHIBITOR**

(30) Priority: 16.12.2020 CN 202011485792
(71) Applicant: Keymed Biosciences Co.,Ltd., Chengdu, Sichuan 610219 (CN)
(72) Inventor: CHEN, Bo, Chengdu, Sichuan 610219 (CN); XU, Gang, Chengdu, Sichuan 610219 (CN); WANG, Changyu, Chengdu, Sichuan 610219 (CN); ZHANG, Guohui, Chengdu, Sichuan 610219 (CN)
(74) Representative: Elzaburu S.L.P.
(86) International application number: PCT/CN2021/138447
(87) International publication number: WO 2022/127834

(57) **Abstract**

Provided are an antibody or antigen-binding portion thereof that binds to MASP-2, and a use of the antibody or antigen-binding portion thereof in the preparation of a drug or kit for diagnosing, treating or preventing diseases associated with lectin pathway of complement activation. The antibody can specifically recognize enzymatic activity domains CCP1-CCP2-SP of human, monkey, mouse and rat MASP-2, and effectively inhibit the production of a complement component C4b and a C3 convertase C4b2a.

## Description

### Technical Field

The present disclosure relates to a complement inhibitor and applications thereof, in particular, to a MASP-2 inhibitory antibody and applications thereof.

### Background Art

Mannose-binding lectin-associated serine protease 2 (MASP-2) belongs to the serine protease family and is a key enzyme in the complement lectin activation pathway. MASP-2 contains six domains, namely N-terminal CUB1, EGF, CUB2, CCP1, CCP2, and C-terminal SP enzymatic activity domain, with a molecular weight of about 75 kDa. MASP-2 in the blood binds the lectin pathway recognition molecules mannose-binding lectin (MBL), collectin-11 (CL-K1), and ficolin (Ficolin-1, Ficolin-2, and Ficolin-3) via N-terminal CUB1-EGF-CUB2 three domains and forms a complex. When MBL, CL-K1 or ficolin bind to pathogen-associated molecular PAMPs such as D-mannose, N-acetyl-D-glucosamine or acetyl, MASP-1 and MASP-2 are activated sequentially. The activated MASP-2 cleaves C4 and C2, and the fragment C4b formed by cleavage interacts with C2a to produce a C3 convertase (C4b2a). The C3 convertase (C4b2a) activates C3, resulting in the production of a C5 convertase (C4b2a3b) and the formation of a membrane attack complex (C5b-9) capable of causing microbial lysis. Activated forms of C3 and C4 (C3b and C4b) are covalently deposited on the surface of foreign targets, which are recognized by complement receptors on various phagocytes, resulting in phagocytosis of the corresponding pathogen. The lectin pathway plays an important role in the innate immunity of the body against pathogenic microorganisms such as bacteria, yeasts, and viruses.

Under certain pathological conditions, glycoproteins with incomplete internal glycosylation modifications (containing high mannose "precursor" glycan clusters) are released after cell injury and rupture, which can be recognized by MBL with high affinity and initiate complement activation (Maynard et al., J. Biol. Chem. 257: 3788-3794, 1982); similarly, immune complexes formed by IgA antibody with galactose modification defects are deposited on the inner wall of glomerular blood vessels, etc. and activate the complement lectin pathway through MBL-MASP-2, causing an inflammatory response. The granzyme released by overactivated inflammatory cells may cause damage to the nearby tissues and organs. In addition, activated complement components are deposited on the nearby cells, which may lead to the generation of the complement membrane attack complex and cell lysis. The complement system is associated with the pathogenesis of a variety of acute or chronic disease conditions, such as IgA nephropathy (IgAN), ischemia-reperfusion injury, transplant rejection, rheumatoid arthritis, myocardial infarction, vascular remodeling following stroke, ARDS, septic shock, capillary leak following thermal burns, inflammation following cardiopulmonary bypass, multiple sclerosis, myasthenia gravis, and Alzheimer's disease, etc. Recent studies have shown that various tissue damage such as lung and kidney caused by novel coronavirus COVID-2019 infection is associated with the complement lectin pathway (Magro C et al., Transl Res. 2020 Jun; 220: 1-13), and inhibition of MASP-2 activity can greatly reduce mortality in severe patients with COVID-2019 (Rambaldi A et al., Immunobiology. 2020 Aug 9: 152001).

MASP-2 is a unique C4-cleaving protease in the lectin pathway, and its concentration in healthy human blood is much lower than that of MASP-1 and other complement components in the lectin pathway. Kidmose R et al. showed that MASP-2 binds to C4 via the CCP2 domain, resulting in the latter being allosteric and exposing restriction sites thereof. The mutation of the CCP2 domain can completely inhibit the activity of MASP-2 (PNAS September 18, 2012, 109 (38) 15425-15430).

### Summary of the Invention

The present disclosure relates to anti-MASP-2 inhibitory antibodies for inhibiting the undesirable effects of MASP-2 dependent complement activation.

In one aspect, the present disclosure provides an antibody or antigen-binding portion thereof that binds to MASP-2.

In one aspect, the present disclosure provides a bispecific antibody or antigen-binding portion thereof.

In one aspect, the present disclosure provides a nucleic acid encoding the aforementioned antibody or antigen-binding portion thereof that binds to MASP-2, or the bispecific antibody or antigen-binding portion thereof.

In one aspect, the present disclosure provides a vector comprising the aforementioned nucleic acid.

In one aspect, the present disclosure provides a cell comprising the aforementioned vector. In one aspect, the present disclosure provides a composition or kit comprising the aforementioned antibody or antigen-binding portion thereof, encoding nucleic acid, vector, and cell.

In one aspect, the present disclosure provides a method of treating diseases associated with MASP-2 dependent complement activation, comprising the steps of administering to the subject a therapeutically effective amount of the antibody or antigen-binding fragment thereof, nucleic acid, vector, cell and/or composition of any of the preceding aspects.

In one aspect, the present disclosure provides a use of the antibody or antigen-binding fragment thereof, nucleic acid, vector, cell and/or pharmaceutical composition of any of the preceding aspects in the preparation of a drug or kit for treating diseases associated with MASP-2 dependent complement activation in a subject.

### Brief Description of the Drawings

FIG. 1 shows SDS-PAGE results for MASP-2 D4-6 recombinant proteins.
FIG. 2 shows the inhibition of C4b deposition by hybridoma supernatant.
FIG. 3 shows the inhibition of C4b deposition by hybridoma clonal chimeric antibodies.
FIG. 4 shows the inhibition of C3b deposition by hybridoma clonal chimeric antibodies.
FIG. 5 shows ELISA detection of candidate chimeric antibodies binding to different species of MASP-2 D4-6.
FIG. 6 shows the inhibition of C4b deposition by Fab candidate clones from the phage display library.
FIG. 7 shows the inhibition of C4b deposition after the purification of Fab candidate clones from the phage display library.
FIG. 8 shows the inhibition of C4b deposition after 28O14 clone mutation. Among them, Ser102 represents the wild-type antibody, with serine at position 102. Gly102, Tyr102, Ala102, Glu102, Gln102, Val102, Leu102, and His102 represent mutant antibodies in which serine is mutated at position 102 to glycine, tyrosine, alanine, glutamic acid, glutamine, valine, leucine, and histidine, respectively.
FIGs. 9A and 9B show the inhibition of C4b deposition by humanized MASP-2 antibodies.
FIGs. 10A, 10B, and 10C show the inhibition of C3b deposition by humanized MASP-2 antibodies.
FIG. 11 shows ELISA detection of humanized MASP-2 antibodies binding to different species of antigens.
FIG. 12 shows the efficacy of humanized MASP-2 antibody in a mouse hemolytic uremic syndrome model.

### Detailed Description of Embodiments

### I. Definitions

In the present disclosure, the scientific and technical terms used herein have the meanings commonly understood by those skilled in the art, unless otherwise specified. Also, the terms and laboratory procedures related to protein and nucleic acid chemistry, molecular biology, cell and tissue culture, microbiology, and immunology used herein are terms and routine procedures widely used in the corresponding fields. Meanwhile, to better understand the present disclosure, definitions and explanations of related terms are provided below.

Provided herein are antibodies (e.g., monoclonal antibodies) that specifically bind to MASP-2 and antigen-binding fragments thereof. In a specific aspect, provided herein are anti-MASP-2 monoclonal antibodies that specifically bind to MASP-2, wherein the anti-MASP-2 antibodies include variants of the parent antibody. In a specific aspect, provided herein are antibodies that specifically bind to MASP-2 (e.g., human MASP-2). In a particular aspect, provided herein are anti-MASP-2 antibodies comprising modifications in one or more amino acid residues (e.g., 5-13 amino acid substitutions in the framework region of the heavy chain variable region), which retain affinity to antigens compared to the parent antibody without the modifications. The term "MASP-2" refers to any MASP-2 molecule known to those skilled in the art. For example, the MASP-2 may be from mammals, e.g., the MASP-2 may be from humans.

As used herein, the term "MASP-2 dependent complement activation" comprises MASP-2 dependent activation of the lectin pathway, which occurs under physiological conditions (i.e., in the presence of Ca²⁺), resulting in the formation of a lectin pathway C3 convertase C4b2a, and resulting in the formation of a C5 convertase C4b2a(C3b)n after accumulation of the C3 cleavage product C3b.

As used herein, the term "MASP-2 inhibitory antibody" refers to any anti-MASP-2 antibody or MASP-2 binding fragment thereof that binds to or directly interacts with MASP-2 and effectively inhibits MASP-2 dependent complement activation. MASP-2 inhibitory antibodies used in the disclosed method may reduce MASP-2 dependent complement activation by greater than 20%, e.g., greater than 30%, greater than 40%, greater than 50%, greater than 60%, greater than 70%, greater than 80%, greater than 90%, or greater than 95%.

The term "lectin pathway" refers to complement activation by specific binding of serum and non-serum carbohydrate-binding proteins, including mannan-binding lectin (MBL) and ficolin.

As used herein, the term "about" or "approximately" refers to within plus or minus 10% of a given value or range, unless otherwise specified. Where integers are required, the term refers to integers within plus or minus 10% of a given value or range, rounded up or down to the nearest integer.

The phrase "substantially identical" with respect to an antibody chain polypeptide sequence may be construed as an antibody chain exhibiting at least 60%, 65%, 70%, 75%, 80%, 85%, 90%, 95%, 96%, 97%, 98%, 99% or more sequence identity to a reference polypeptide sequence. The term with respect to a nucleotide sequence may be construed as a nucleotide sequence exhibiting at least greater than 60%, 65%, 70%, 75%, 80%, 85%, 90%, 95%, 96%, 97%, 98%, 99% or more sequence identity to the reference nucleotide sequence.

Sequence "identity" has a recognized meaning in this field and the percentage of sequence identity between two nucleic acid or polypeptide molecules or regions can be calculated using published techniques. Sequence identity can be measured along the entire length of a polynucleotide or polypeptide or a region of the molecule. While there are many methods of measuring identity between two polynucleotides or polypeptides, the term "identity" is well-known to those skilled in the art (Carrillo, H. & Lipman, D. SIAM J Applied Math 48: 1073 (1988)).

"Substitution" variants are those in which at least one amino acid residue in the native sequence has been removed and inserted by a different amino acid at the same position. The substitution may be single, with only one amino acid in the molecule being substituted; or may be multiple, with two or more amino acids being substituted in the same molecule. Multiple substitutions may be at consecutive positions. Likewise, an amino acid may be substituted with multiple residues, wherein such variants include both substitutions and insertions. "Insertion" variants are those in which one or more amino acids are inserted immediately adjacent to an amino acid at a particular position in a native sequence. By immediately adjacent amino acid is meant attached to the α-carboxy or α-amino functional group of the amino acid. "Deletion" variants are those in which one or more amino acids in the native amino acid sequence have been removed. Typically, one or two amino acids are deleted in a particular region of the molecules of deletion variants.

With respect to the variable domains of antibodies, the term "variable" refers to certain portions of related molecules that differ widely in sequence between antibodies and are used for specifically recognizing and binding to a specific target of a particular antibody. However, the variability is not uniformly distributed throughout the variable domain of the antibody. Variability is concentrated on three segments called complementarity determining regions (CDRs; i.e., CDR1, CDR2, and CDR3) or hypervariable regions, all of which are located within the variable domains of the light and heavy chains. The more conserved portions within the variable domains are referred to as framework (FR) regions or framework sequences. Each variable domain of native heavy and light chains comprises four FR regions, predominantly in a β -sheet configuration, connected by three CDRs, which form loops connecting and in some cases forming part of the β -sheet structure. The CDRs of each chain are typically joined together by adjacent FR regions and aid in the formation of antibody target binding sites (epitopes or determinants) by means of CDR from other chains. As used herein, the numbering of immunoglobulin amino acid residues is based on Kabat et al.'s immunoglobulin amino acid residues numbering scheme, unless otherwise indicated. One CDR may have the ability to specifically bind to an associated epitope.

As used herein, the "antibody fragment" or "antigen-binding fragment" of an antibody refers to any portion of the full-length antibody, which is less than full-length, but comprises at least a portion of the variable region (e.g., one or more CDRs and/or one or more antibody binding sites) of the antibody that binds to an antigen, and thus retaining binding specificity as well as at least a portion of the specific binding capacity of the full-length antibody. Thus, an antigen-binding fragment refers to an antibody fragment that comprises an antigen-binding portion that binds the same antigen as the antibody from which the antibody fragment was derived. Antibody fragments include antibody derivatives produced by enzymatic treatment of full-length antibodies, as well as synthetically produced derivatives, e.g., recombinantly produced derivatives. Antibodies include antibody fragments. Examples of the antibody fragments include, but are not limited to, Fab, Fab', F(ab')₂, single chain Fv (scFv), Fv, dsFv, diabodies, Fd, and Fd' fragments and other fragments, including modified fragments (see, e.g., Methods in Molecular Biology, Vol 207: Recombinant Antibodies for Cancer Therapy Methods and Protocols (2003); Chapter 1; p 3-25, Kipriyanov). The fragments may comprise multiple chains linked together, for example, by disulfide bonds and/or by peptide linkers. Antibody fragments generally comprise at least or about 50 amino acids, and typically at least or about 200 amino acids. Antigen-binding fragments include any antibody fragment that, when inserted into an antibody framework (e.g., by displacement of the corresponding region), results in the antibody that immunospecifically binds to an antigen (i.e., exhibiting a Ka of at least or at least about 10⁷-10⁸M⁻¹). The "functional fragment" or "analog of an anti-MASP-2 antibody" is a fragment or analog that may prevent or substantially reduce the ability of the receptor to bind a ligand or initiate signal transduction. As used herein, the functional fragments generally have the same meaning as "antibody fragments" and, in the case of antibodies, may refer to fragments that may prevent or substantially reduce the ability of the receptor to bind to a ligand or initiate signal transduction, e.g., Fv, Fab, F(ab')₂, and the like. The "Fv" fragment consists of a dimer (V_{H}-V_{L} dimer) formed by the non-covalent binding of a variable domain of a heavy chain and a variable domain of a light chain. In this configuration, the three CDRs of each variable domain interact to determine the target binding sites on the surface of the V_{H}-V_{L} dimer, as is the case with intact antibodies. The six CDRs together confer target binding specificity to the intact antibody. However, even a single variable domain (or half of an Fv comprising only three target-specific CDRs) may still have the ability to recognize and bind to targets.

As used herein, the term "bispecific antibody (BsAb)" refers to an antibody and/or antigen-binding molecule capable of specifically binding to two different antigenic determinants. Generally, a bispecific antibody and/or antigen-binding molecule comprises two antigen-binding sites, each of which is specific for different antigenic determinants. In some embodiments, the bispecific antibody and/or antigen-binding molecule is capable of binding to two antigenic determinants simultaneously, particularly two antigenic determinants expressed on two different cells.

As used herein, the "monoclonal antibody" refers to a population of identical antibodies, meaning that each individual antibody molecule in the population of monoclonal antibodies is identical to another antibody molecule. This property is in contrast to the property of a population of polyclonal antibodies comprising antibodies having a plurality of different sequences. Monoclonal antibodies can be prepared by a number of well-known methods (Smith et al., (2004) J. Clin. Pathol. 57, 912-917; and Nelson et al., J Clin Pathol (2000), 53, 111-117). For example, monoclonal antibodies can be prepared by immortalizing B cells, for example, by fusing with myeloma cells to generate hybridoma cell lines or by infecting B cells with a virus such as EBV Recombinant techniques can also be used to prepare antibodies from clonal populations of host cells in vitro by transforming host cells with plasmids carrying artificial sequences encoding the nucleotides of the antibodies.

As used herein, the term "hybridoma" or "hybridoma cell" refers to a cell or cell line (typically a myeloma or lymphoma cell) resulting from the fusion of antibody-producing lymphocytes and non-antibody-producing cancer cells. As known to those of ordinary skill in the art, the hybridoma may be propagated and continuously supplied to produce a particular monoclonal antibody. Methods for producing hybridomas are known in the art. When referring to the term "hybridoma" or "hybridoma cell", it also comprises subclones and progeny cells of the hybridoma.

As used herein, a full-length antibody is an antibody having two full-length heavy chains (e.g., VH-CH1-CH2-CH3 or VH-CH1-CH2-CH3-CH4) and two full-length light chains (VL-CL) and a hinge region, e.g., an antibody naturally produced by an antibody-secreting B cell as well as a synthetically produced antibody having the same domain.

The term "chimeric antibody" refers to an antibody in which the variable region sequences are derived from one species and the constant region sequences are derived from another species, such as an antibody in which the variable region sequences are derived from a mouse antibody and the constant region sequences are derived from a human antibody. The "humanized" antibody refers to a form of non-human (e.g., mouse) antibody that is a chimeric immunoglobulin, immunoglobulin chain or fragment thereof (e.g., Fv, Fab, Fab', F(ab')₂ or other antigen-binding subsequences of the antibody), containing minimal sequence derived from non-human immunoglobulin. Preferably, the humanized antibody is a human immunoglobulin (recipient antibody) in which residues from a complementarity determining region (CDR) of the recipient antibody are replaced by CDR residues from a non-human species (donor antibody) such as mouse, rat, or rabbit having the desired specificity, affinity, and capacity.

Furthermore, in humanization it is also possible to mutate amino acid residues within the CDR1, CDR2 and/or CDR3 regions of VH and/or VL, thereby improving one or more binding properties (e.g., affinity) of the antibody. Mutations may be introduced, e.g., by PCR-mediated mutagenesis, and their effects on antibody binding or other functional properties can be assessed using the in vitro or in vivo assays described herein. Typically, conservative mutations are introduced. Such mutations may be amino acid substitutions, additions, or deletions. In addition, mutations within CDR typically do not exceed one or two. Thus, humanized antibodies of the present disclosure also encompass antibodies comprising 1 or 2 amino acid mutations within CDR.

As used herein, the term "CDR" refers to a complementarity-determining region, where it is known that each heavy and light chain of antibody molecules has three CDRs. CDR is also known as a hypervariable region and is present in the variable region of each of the heavy and light chains of an antibody with a very high site of variability in the primary structure of CDR. In the present specification, the CDR of the heavy chain is represented by CDR1, CDR2 and CDR3 from the amino terminus of the amino-terminal sequence of the heavy chain, and the CDR of the light chain is represented by CDR1, CDR2 and CDR3 from the amino terminus of the amino-terminal sequence of the light chain. These sites are adjacent to each other in the tertiary structure and determine the specificity of the antigen to which the antibody binds.

As used herein, the term "epitope" refers to any antigenic determinant on an antigen to which the paratope of an antibody binds. Epitopic determinants usually comprise chemically active surface types of molecules, such as amino acids or sugar side chains, and usually have specific three-dimensional structural characteristics as well as specific charge characteristics.

As used herein, the "specific binding" or "immunospecifically binding" with respect to an antibody or antigen-binding fragment thereof is used interchangeably herein and refers to the ability of an antibody or antigen-binding fragment to form one or more non-covalent bonds with the same antigen through non-covalent interactions between the antibody and the antibody binding site of the antigen. The antigen may be an isolated antigen or present in a tumor cell. Typically, the antibody that immunospecifically binds (or specifically binds) to the antigen is the one that binds to the antigen with an affinity constant Ka of about or 1×10⁷M⁻¹ or 1×10⁸M⁻¹ or more (or a dissociation constant (Kd) of 1×10⁻⁷M or 1×10⁻⁸M or less). Affinity constant can be determined by standard kinetic methods for antibody reactions, e.g., immunoassays, surface plasmon resonance (SPR) (Rich and Myszka (2000) Curr. Opin. Biotechnol 11:54; Englebienne (1998) Analyst. 123:1599), isothermal titration calorimetry (ITC), or other kinetic interaction assays known in the art; see also U.S. Patent No. 7, 229, 619 describing exemplary SPR and ITC method for calculating the binding affinity of an antibody. Instruments and methods for real-time detection and monitoring of the binding rate are known and commercially available (see, Malmqvist (2000) Biochem.Soc.Trans. 27:335).

As used herein, the terms "polynucleotide" and "nucleic acid molecule" refer to an oligomer or polymer comprising at least two linked nucleotides or nucleotide derivatives, including deoxyribonucleic acid (DNA) and ribonucleic acid (RNA), typically linked together by phosphodiester bonds. As used herein, the term "nucleic acid molecule" is intended to include DNA molecules and RNA molecules. The nucleic acid molecule may be single-stranded or double-stranded, and may be cDNA.

As used herein, an isolated nucleic acid molecule is a nucleic acid molecule isolated from other nucleic acid molecules present in the natural source of the nucleic acid molecule. The "isolated" nucleic acid molecule, such as a cDNA molecule, can be substantially free of other cellular material or culture medium when prepared by recombinant techniques, or substantially free of chemical precursors or other chemical components when chemically synthesized. Exemplary isolated nucleic acid molecules provided herein comprise isolated nucleic acid molecules encoding provided antibodies or antigen-binding fragments.

As used herein, the "operably linked" with respect to a nucleotide sequence, region, element or domain means that the nucleic acid regions are functionally related to one another. For example, a promoter may be operably linked to a nucleic acid encoding a polypeptide such that the promoter regulates or mediates transcription of the nucleic acid. Also provided are "conservative sequence modifications" of the sequences set forth in the sequence listings described herein, i.e., nucleotide and amino acid sequence modifications that do not eliminate the binding ability of the antibody encoded by the nucleotide sequence or containing an amino acid sequence to an antigen. These conservative sequence modifications comprise conservative nucleotide and amino acid substitutions as well as nucleotide and amino acid additions and deletions. For example, modifications can be introduced into the sequence listings described herein by standard techniques known in the art, such as site-directed mutagenesis and PCR-mediated mutagenesis. Conservative sequence modifications comprise conservative amino acid substitutions in which an amino acid residue is replaced with an amino acid residue having a similar side chain. Families of amino acid residues having similar side chains have been defined in the art. These families include amino acids containing basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Thus, a predicted nonessential amino acid residue in an anti-MASP-2 antibody is preferably replaced with another amino acid residue from the same side chain family. Methods for identifying conservative substitutions of nucleotides and amino acids that do not eliminate antigen binding are well-known in the art (see, e.g., Brummell et al., Biochem. 32: 1180-1187 (1993); Kobayashi et al., Protein Eng. 12(10): 879-884 (1999); Burks et al., Proc.Natl.Acad.Sci.USA 94: 412-417 (1997)).

Alternatively, in another embodiment, mutations can be randomly introduced along all or a portion of the anti-MASP-2 antibody coding sequence, e.g., by saturation mutagenesis, and the resulting modified anti-MASP-2 antibodies can be screened for improved binding activity.

As used herein, the "expression" refers to a process of producing a polypeptide by transcription and translation of a polynucleotide. The level of expression of a polypeptide can be assessed using any method known in the art, including, for example, methods of determining the amount of polypeptide produced from a host cell. Such methods may include, but are not limited to, quantitation of polypeptides in cell lysates by ELISA, gel electrophoresis followed by Coomassie blue staining, Lowry protein assay, and Bradford protein assay.

As used herein, the "host cell" is a cell for receiving, maintaining, replicating, and expanding a vector. The host cell may also be used to express a polypeptide encoded by the vector. The nucleic acid contained in the vector replicates during the host cell division, thereby amplifying the nucleic acid. The host cell may be a eukaryotic cell or a prokaryotic cell. Suitable host cells include, but are not limited to, CHO cells, various COS cells, HeLa cells, and HEK cells such as HEK 293 cells.

As used herein, the "vector" is a replicable nucleic acid from which one or more heterologous proteins can be expressed when the vector is transformed into an appropriate host cell. References to vectors include those into which a nucleic acid encoding a polypeptide or fragment thereof may be introduced, typically by restriction digestion and ligation. References to vectors also include those comprising a nucleic acid encoding a polypeptide. Vectors are used to introduce a nucleic acid encoding a polypeptide into a host cell, to amplify the nucleic acid, or to express/display the polypeptide encoded by the nucleic acid. The vector generally remains free but can be designed to allow the integration of a gene or portion thereof into the chromosome of the genome. Also contemplated are vectors for artificial chromosomes, such as yeast artificial vectors and mammalian artificial chromosomes. The selection and use of such vehicles are well-known to those skilled in the art.

As used herein, the vector also comprises a "virus vector" or a "viral vector". The viral vector is an engineered virus that is operably linked to an exogenous gene to transfer (as a vehicle or shuttle) the exogenous gene into a cell.

As used herein, the "expression vector" comprises a vector capable of expressing a DNA operably linked to regulatory sequences, such as a promoter region, capable of affecting the expression of such DNA fragments. Such additional fragments may comprise promoter and terminator sequences, and optionally may comprise one or more origins of replication, one or more selectable markers, enhancers, polyadenylation signals, etc. Expression vectors are typically derived from plasmid or viral DNA, or may contain elements of both. Thus, the expression vector refers to a recombinant DNA or RNA construct, such as a plasmid, phage, recombinant virus, or other vectors, which results in the expression of a cloned DNA when introduced into an appropriate host cell. Appropriate expression vectors are well-known to those skilled in the art and comprise expression vectors that are replicable in eukaryotic and/or prokaryotic cells as well as expression vectors that remain free or that are integrated into the host cell genome.

As used herein, "treating" individuals with a disease or condition means that the individual symptoms are partially or completely alleviated or remain unchanged after the treatment. Thus, the treatment comprises prevention, treatment, and/or cure. Prevention refers to preventing the underlying disease and/or preventing the worsening of symptoms or the progression of the disease. The treatment also comprises any pharmaceutical use of any antibody or antigen-binding fragment thereof provided and the composition provided herein.

As used herein, the "therapeutic effect" refers to an effect resulting from the treatment of an individual that alters, typically improves or ameliorates the symptoms of a disease or condition, or cures a disease or condition.

As used herein, the "therapeutically effective amount" or "therapeutically effective dose" refers to an amount of a substance, compound, material, or composition comprising the compound that is at least sufficient to produce a therapeutic effect after being administered to the subject. Thus, it is an amount necessary to prevent, cure, ameliorate, block, or partially block the symptoms of a disease or disorder.

As used herein, the "prophylactically effective amount" or "prophylactically effective dose" refers to an amount of a substance, compound, material, or composition comprising the compound that, when administered to a subject, has the desired prophylactic effect, e.g., preventing or delaying the occurrence or recurrence of a disease or condition, reducing the likelihood of the occurrence or recurrence of a disease or condition. A fully prophylactically effective dose need not occur by administration of one dose, and may occur only after administration of a series of doses. Thus, the prophylactically effective amount may be administered in one or more administrations.

As used herein, the term "patient" or "subject" refers to a mammal, such as a human.

### II. Detailed description of specific embodiments

In one aspect, the present disclosure provides an antibody or antigen-binding portion thereof that binds to MASP-2, comprising heavy chain CDRs selected from the group consisting of amino acid sequences SEQ ID NOs: 7, 8, 9, 17, 18, 26, 27, 28, 36, 37, 38, 46, 47, 60, 65, 66, 67, 79, 85, 86, 94, 95, 96, 104, 105, 113, 114, 126, 127, 128, 136, 137, 138, 141, 149, 150, 151, 165, 183, 192 or any variant thereof, and/or light chain CDRs selected from the group consisting of amino acid sequences SEQ ID NOs: 12, 13, 14, 21, 22, 23, 31, 32, 33, 41, 42, 43, 50, 51, 52, 57, 70, 71, 72, 82, 91, 108, 109, 110, 117, 118, 123, 131, 132, 133, 144, 145, 146, 154, 155, 156, 161, 162, 168, 173, 174, 201, 202, 207, 216 or any variant thereof.

The antibody or antigen-binding portion thereof according to the previous aspect, comprising a heavy chain CDR1 selected from the group consisting of amino acid sequences SEQ ID NOs: 7, 26, 36, 65, 94, 126, 136, 149 or any variant thereof, a heavy chain CDR2 selected from the group consisting of amino acid sequences SEQ ID NOs: 8, 17, 27, 37, 46, 66, 85, 95, 104, 113, 127, 137, 150, 165, 183, 192 or any variant thereof, and a heavy chain CDR3 selected from the group consisting of amino acid sequences SEQ ID NOs: 9, 18, 28, 38, 47, 60, 67, 79, 86, 96, 105, 114, 128, 138, 141, 151 or any variant thereof; and/or a light chain CDR1 selected from the group consisting of amino acid sequences SEQ ID NOs: 12, 21, 31, 41, 50, 70, 99, 108, 117, 123, 131, 144, 154, 161, 201, 216 or any variant thereof, a light chain CDR2 selected from the group consisting of amino acid sequences SEQ ID NOs: 13, 22, 32, 42, 51, 57, 71, 100, 109, 118, 132, 145, 155, 162, 168, 173, 202, 207 or any variant thereof, and a light chain CDR3 selected from the group consisting of amino acid sequences SEQ ID NOs: 14, 23, 33, 43, 52, 72, 82, 91, 101, 110, 133, 146, 156, 174 or any variant thereof.

The antibody or antigen-binding portion thereof according to the previous aspect, comprising a combination of heavy and light chain CDRs selected from the group consisting of:
(1) the heavy chain CDR1, CDR2 and CDR3 sequences comprising SEQ ID NOs: 7, 8 and 9, respectively, and/or the light chain CDR1, CDR2 and CDR3 sequences comprising SEQ ID NOs: 12, 13 and 14, respectively;
(2) the heavy chain CDR1, CDR2 and CDR3 sequences comprising SEQ ID NOs: 7, 17 and 18, respectively, and/or the light chain CDR1, CDR2 and CDR3 sequences comprising SEQ ID NOs: 21, 22 and 23, respectively;
(3) the heavy chain CDR1, CDR2 and CDR3 sequences comprising SEQ ID NOs: 26, 27 and 28, respectively, and/or the light chain CDR1, CDR2 and CDR3 sequences comprising SEQ ID NOs: 31, 32 and 33, respectively;
(4) the heavy chain CDR1, CDR2 and CDR3 sequences comprising SEQ ID NOs: 36, 37 and 38, respectively, and/or the light chain CDR1, CDR2 and CDR3 sequences comprising SEQ ID NOs: 41, 42 and 43, respectively;
(5) the heavy chain CDR1, CDR2 and CDR3 sequences comprising SEQ ID NOs: 7, 46 and 47, respectively, and/or the light chain CDR1, CDR2 and CDR3 sequences comprising SEQ ID NOs: 50, 51 and 52, respectively;
(6) the heavy chain CDR1, CDR2 and CDR3 sequences comprising SEQ ID NOs: 7, 17 and 18, respectively, and/or the light chain CDR1, CDR2 and CDR3 sequences comprising SEQ ID NOs: 21, 57 and 23, respectively;
(7) the heavy chain CDR1, CDR2 and CDR3 sequences comprising SEQ ID NOs: 7, 17 and 60, respectively, and/or the light chain CDR1, CDR2 and CDR3 sequences comprising SEQ ID NOs: 50, 51 and 52, respectively;
(8) the heavy chain CDR1, CDR2 and CDR3 sequences comprising SEQ ID NOs: 65, 66 and 67, respectively, and/or the light chain CDR1, CDR2 and CDR3 sequences comprising SEQ ID NOs: 70, 71 and 72, respectively;
(9) the heavy chain CDR1, CDR2 and CDR3 sequences comprising SEQ ID NOs: 7, 17 and 47, respectively, and/or the light chain CDR1, CDR2 and CDR3 sequences comprising SEQ ID NOs: 50, 51 and 52, respectively;
(10) the heavy chain CDR1, CDR2 and CDR3 sequences comprising SEQ ID NOs: 7, 17 and 79, respectively, and/or the light chain CDR1, CDR2 and CDR3 sequences comprising SEQ ID NOs: 50, 51 and 82, respectively;
(11) the heavy chain CDR1, CDR2 and CDR3 sequences comprising SEQ ID NOs: 36, 85 and 86, respectively, and/or the light chain CDR1, CDR2 and CDR3 sequences comprising SEQ ID NOs: 41, 42 and 43, respectively;
(12) the heavy chain CDR1, CDR2 and CDR3 sequences comprising SEQ ID NOs: 36, 85 and 86, respectively, and/or the light chain CDR1, CDR2 and CDR3 sequences comprising SEQ ID NOs: 41, 42 and 91, respectively;
(13) the heavy chain CDR1, CDR2 and CDR3 sequences comprising SEQ ID NOs: 94, 95 and 96, respectively, and/or the light chain CDR1, CDR2 and CDR3 sequences comprising SEQ ID NOs: 99, 100 and 101, respectively;
(14) the heavy chain CDR1, CDR2 and CDR3 sequences comprising SEQ ID NOs: 7, 104 and 105, respectively, and/or the light chain CDR1, CDR2 and CDR3 sequences comprising SEQ ID NOs: 108, 109 and 110, respectively;
(15) the heavy chain CDR1, CDR2 and CDR3 sequences comprising SEQ ID NOs: 26, 113 and 114, respectively, and/or the light chain CDR1, CDR2 and CDR3 sequences comprising SEQ ID NOs: 117, 118 and 33, respectively;
(16) the heavy chain CDR1, CDR2 and CDR3 sequences comprising SEQ ID NOs: 94, 95 and 96, respectively, and/or the light chain CDR1, CDR2 and CDR3 sequences comprising SEQ ID NOs: 123, 100 and 101, respectively;
(17) the heavy chain CDR1, CDR2 and CDR3 sequences comprising SEQ ID NOs: 126, 127 and 128, respectively, and/or the light chain CDR1, CDR2 and CDR3 sequences comprising SEQ ID NOs: 131, 132 and 133, respectively;
(18) the heavy chain CDR1, CDR2 and CDR3 sequences comprising SEQ ID NOs: 136, 137 and 138, respectively, and/or the light chain CDR1, CDR2 and CDR3 sequences comprising SEQ ID NOs: 131, 132 and 133, respectively;
(19) the heavy chain CDR1, CDR2 and CDR3 sequences comprising SEQ ID NOs: 7, 17 and 141, respectively, and/or the light chain CDR1, CDR2 and CDR3 sequences comprising SEQ ID NOs: 144, 145 and 146, respectively;
(20) the heavy chain CDR1, CDR2 and CDR3 sequences comprising SEQ ID NOs: 149, 150 and 151, respectively, and/or the light chain CDR1, CDR2 and CDR3 sequences comprising SEQ ID NOs: 154, 155 and 156, respectively;
(21) the heavy chain CDR1, CDR2 and CDR3 sequences comprising SEQ ID NOs: 7, 17 and 9, respectively, and/or the light chain CDR1, CDR2 and CDR3 sequences comprising SEQ ID NOs: 161, 162 and 14, respectively;
(22) the heavy chain CDR1, CDR2 and CDR3 sequences comprising SEQ ID NOs: 7, 165 and 9, respectively, and/or the light chain CDR1, CDR2 and CDR3 sequences comprising SEQ ID NOs: 144, 168 and 14, respectively;
(23) the heavy chain CDR1, CDR2 and CDR3 sequences comprising SEQ ID NOs: 7, 17 and 9, respectively, and/or the light chain CDR1, CDR2 and CDR3 sequences comprising SEQ ID NOs: 144, 173 and 174, respectively;
(24) the heavy chain CDR1, CDR2 and CDR3 sequences comprising SEQ ID NOs: 136, 183 and 138, respectively, and/or the light chain CDR1, CDR2 and CDR3 sequences comprising SEQ ID NOs: 216, 132 and 133, respectively;
(25) the heavy chain CDR1, CDR2 and CDR3 sequences comprising SEQ ID NOs: 126, 192 and 128, respectively, and/or the light chain CDR1, CDR2 and CDR3 sequences comprising SEQ ID NOs: 216, 132 and 133, respectively;
(26) the heavy chain CDR1, CDR2 and CDR3 sequences comprising SEQ ID NOs: 7, 17 and 141, respectively, and/or the light chain CDR1, CDR2 and CDR3 sequences comprising SEQ ID NOs: 201, 202 and 146, respectively;
(27) the heavy chain CDR1, CDR2 and CDR3 sequences comprising SEQ ID NOs: 7, 17 and 141, respectively, and/or the light chain CDR1, CDR2 and CDR3 sequences comprising SEQ ID NOs: 201, 207 and 146, respectively.

The antibody or antigen-binding portion thereof according to the previous aspect, comprising a heavy chain variable region selected from the group consisting of amino acid sequences SEQ ID NOs: 5, 15, 24, 34, 44, 53, 58, 63, 73, 77, 83, 92, 102, 111, 119, 124, 134, 139, 147, 157, 163, 169, 175, 177, 179, 181, 184, 186, 188, 190, 193, 195, 197 or any variant thereof, and/or a light chain variable region selected from the group consisting of amino acid sequences SEQ ID NOs: 10, 19, 29, 39, 48, 55, 61, 68, 75, 80, 87, 89, 97, 106, 115, 121, 129, 142, 152, 159, 166, 171, 199, 203, 205, 208, 210, 212, 214 or any variant thereof;
in some preferred embodiments, the antibody or antigen-binding portion thereof comprises the heavy chain variable region of the amino acid sequence SEQ ID NO: 5 or any variant thereof, and the light chain variable region of the amino acid sequence SEQ ID NO: 10 or any variant thereof;
in some preferred embodiments, the antibody or antigen-binding portion thereof comprises the heavy chain variable region of the amino acid sequence SEQ ID NO: 15 or any variant thereof, and the light chain variable region of the amino acid sequence SEQ ID NO: 19 or any variant thereof;
in some preferred embodiments, the antibody or antigen-binding portion thereof comprises the heavy chain variable region of the amino acid sequence SEQ ID NO: 24 or any variant thereof, and the light chain variable region of the amino acid sequence SEQ ID NO: 29 or any variant thereof;
in some preferred embodiments, the antibody or antigen-binding portion thereof comprises the heavy chain variable region of the amino acid sequence SEQ ID NO: 34 or any variant thereof, and the light chain variable region of the amino acid sequence SEQ ID NO: 39 or any variant thereof;
in some preferred embodiments, the antibody or antigen-binding portion thereof comprises the heavy chain variable region of the amino acid sequence SEQ ID NO: 44 or any variant thereof, and the light chain variable region of the amino acid sequence SEQ ID NO: 48 or any variant thereof;
in some preferred embodiments, the antibody or antigen-binding portion thereof comprises the heavy chain variable region of the amino acid sequence SEQ ID NO: 53 or any variant thereof, and the light chain variable region of the amino acid sequence SEQ ID NO: 55 or any variant thereof;
in some preferred embodiments, the antibody or antigen-binding portion thereof comprises the heavy chain variable region of the amino acid sequence SEQ ID NO: 58 or any variant thereof, and the light chain variable region of the amino acid sequence SEQ ID NO: 61 or any variant thereof;
in some preferred embodiments, the antibody or antigen-binding portion thereof comprises the heavy chain variable region of the amino acid sequence SEQ ID NO: 63 or any variant thereof, and the light chain variable region of the amino acid sequence SEQ ID NO: 68 or any variant thereof;
in some preferred embodiments, the antibody or antigen-binding portion thereof comprises the heavy chain variable region of the amino acid sequence SEQ ID NO: 73 or any variant thereof, and the light chain variable region of the amino acid sequence SEQ ID NO: 75 or any variant thereof;
in some preferred embodiments, the antibody or antigen-binding portion thereof comprises the heavy chain variable region of the amino acid sequence SEQ ID NO: 77 or any variant thereof, and the light chain variable region of the amino acid sequence SEQ ID NO: 80 or any variant thereof;
in some preferred embodiments, the antibody or antigen-binding portion thereof comprises the heavy chain variable region of the amino acid sequence SEQ ID NO: 83 or any variant thereof, and the light chain variable region of the amino acid sequence SEQ ID NO: 87 or any variant thereof;
in some preferred embodiments, the antibody or antigen-binding portion thereof comprises the heavy chain variable region of the amino acid sequence SEQ ID NO: 83 or any variant thereof, and the light chain variable region of the amino acid sequence SEQ ID NO: 89 or any variant thereof;
in some preferred embodiments, the antibody or antigen-binding portion thereof comprises the heavy chain variable region of the amino acid sequence SEQ ID NO: 92 or any variant thereof, and the light chain variable region of the amino acid sequence SEQ ID NO: 97 or any variant thereof;
in some preferred embodiments, the antibody or antigen-binding portion thereof comprises the heavy chain variable region of the amino acid sequence SEQ ID NO: 102 or any variant thereof, and the light chain variable region of the amino acid sequence SEQ ID NO: 106 or any variant thereof;
in some preferred embodiments, the antibody or antigen-binding portion thereof comprises the heavy chain variable region of the amino acid sequence SEQ ID NO: 111 or any variant thereof, and the light chain variable region of the amino acid sequence SEQ ID NO: 115 or any variant thereof;
in some preferred embodiments, the antibody or antigen-binding portion thereof comprises the heavy chain variable region of the amino acid sequence SEQ ID NO: 119 or any variant thereof, and the light chain variable region of the amino acid sequence SEQ ID NO: 121 or any variant thereof;
in some preferred embodiments, the antibody or antigen-binding portion thereof comprises the heavy chain variable region of the amino acid sequence SEQ ID NO: 124 or any variant thereof, and the light chain variable region of the amino acid sequence SEQ ID NO: 129 or any variant thereof;
in some preferred embodiments, the antibody or antigen-binding portion thereof comprises the heavy chain variable region of the amino acid sequence SEQ ID NO: 134 or any variant thereof, and the light chain variable region of the amino acid sequence SEQ ID NO: 129 or any variant thereof;
in some preferred embodiments, the antibody or antigen-binding portion thereof comprises the heavy chain variable region of the amino acid sequence SEQ ID NO: 139 or any variant thereof, and the light chain variable region of the amino acid sequence SEQ ID NO: 142 or any variant thereof;
in some preferred embodiments, the antibody or antigen-binding portion thereof comprises the heavy chain variable region of the amino acid sequence SEQ ID NO: 147 or any variant thereof, and the light chain variable region of the amino acid sequence SEQ ID NO: 152 or any variant thereof;
in some preferred embodiments, the antibody or antigen-binding portion thereof comprises the heavy chain variable region of the amino acid sequence SEQ ID NO: 157 or any variant thereof, and the light chain variable region of the amino acid sequence SEQ ID NO: 159 or any variant thereof;
in some preferred embodiments, the antibody or antigen-binding portion thereof comprises the heavy chain variable region of the amino acid sequence SEQ ID NO: 163 or any variant thereof, and the light chain variable region of the amino acid sequence SEQ ID NO: 166 or any variant thereof;
in some preferred embodiments, the antibody or antigen-binding portion thereof comprises the heavy chain variable region of the amino acid sequence SEQ ID NO: 169 or any variant thereof, and the light chain variable region of the amino acid sequence SEQ ID NO: 171 or any variant thereof;
in some preferred embodiments, the antibody or antigen-binding portion thereof comprises the heavy chain variable region of the amino acid sequence SEQ ID NO: 181 or any variant thereof, and the light chain variable region of the amino acid sequence SEQ ID NO: 214 or any variant thereof;
in some preferred embodiments, the antibody or antigen-binding portion thereof comprises the heavy chain variable region of the amino acid sequence SEQ ID NO: 184 or any variant thereof, and the light chain variable region of the amino acid sequence SEQ ID NO: 214 or any variant thereof;
in some preferred embodiments, the antibody or antigen-binding portion thereof comprises the heavy chain variable region of the amino acid sequence SEQ ID NO: 186 or any variant thereof, and the light chain variable region of the amino acid sequence SEQ ID NO: 214 or any variant thereof;
in some preferred embodiments, the antibody or antigen-binding portion thereof comprises the heavy chain variable region of the amino acid sequence SEQ ID NO: 188 or any variant thereof, and the light chain variable region of the amino acid sequence SEQ ID NO: 214 or any variant thereof;
in some preferred embodiments, the antibody or antigen-binding portion thereof comprises the heavy chain variable region of the amino acid sequence SEQ ID NO: 190 or any variant thereof, and the light chain variable region of the amino acid sequence SEQ ID NO: 214 or any variant thereof;
in some preferred embodiments, the antibody or antigen-binding portion thereof comprises the heavy chain variable region of the amino acid sequence SEQ ID NO: 193 or any variant thereof, and the light chain variable region of the amino acid sequence SEQ ID NO: 214 or any variant thereof;
in some preferred embodiments, the antibody or antigen-binding portion thereof comprises the heavy chain variable region of the amino acid sequence SEQ ID NO: 195 or any variant thereof, and the light chain variable region of the amino acid sequence SEQ ID NO: 214 or any variant thereof;
in some preferred embodiments, the antibody or antigen-binding portion thereof comprises the heavy chain variable region of the amino acid sequence SEQ ID NO: 197 or any variant thereof, and the light chain variable region of the amino acid sequence SEQ ID NO: 214 or any variant thereof;
in some preferred embodiments, the antibody or antigen-binding portion thereof comprises the heavy chain variable region of the amino acid sequence SEQ ID NO: 175 or any variant thereof, and the light chain variable region of the amino acid sequence SEQ ID NO: 199 or any variant thereof;
in some preferred embodiments, the antibody or antigen-binding portion thereof comprises the heavy chain variable region of the amino acid sequence SEQ ID NO: 175 or any variant thereof, and the light chain variable region of the amino acid sequence SEQ ID NO: 203 or any variant thereof;
in some preferred embodiments, the antibody or antigen-binding portion thereof comprises the heavy chain variable region of the amino acid sequence SEQ ID NO: 175 or any variant thereof, and the light chain variable region of the amino acid sequence SEQ ID NO: 205 or any variant thereof;
in some preferred embodiments, the antibody or antigen-binding portion thereof comprises the heavy chain variable region of the amino acid sequence SEQ ID NO: 177 or any variant thereof, and the light chain variable region of the amino acid sequence SEQ ID NO: 205 or any variant thereof;
in some preferred embodiments, the antibody or antigen-binding portion thereof comprises the heavy chain variable region of the amino acid sequence SEQ ID NO: 177 or any variant thereof, and the light chain variable region of the amino acid sequence SEQ ID NO: 199 or any variant thereof;
in some preferred embodiments, the antibody or antigen-binding portion thereof comprises the heavy chain variable region of the amino acid sequence SEQ ID NO: 177 or any variant thereof, and the light chain variable region of the amino acid sequence SEQ ID NO: 203 or any variant thereof;
in some preferred embodiments, the antibody or antigen-binding portion thereof comprises the heavy chain variable region of the amino acid sequence SEQ ID NO: 179 or any variant thereof, and the light chain variable region of the amino acid sequence SEQ ID NO: 199 or any variant thereof;
in some preferred embodiments, the antibody or antigen-binding portion thereof comprises the heavy chain variable region of the amino acid sequence SEQ ID NO: 179 or any variant thereof, and the light chain variable region of the amino acid sequence SEQ ID NO: 203 or any variant thereof;
in some preferred embodiments, the antibody or antigen-binding portion thereof comprises the heavy chain variable region of the amino acid sequence SEQ ID NO: 179 or any variant thereof, and the light chain variable region of the amino acid sequence SEQ ID NO: 205 or any variant thereof;
in some preferred embodiments, the antibody or antigen-binding portion thereof comprises the heavy chain variable region of the amino acid sequence SEQ ID NO: 175 or any variant thereof, and the light chain variable region of the amino acid sequence SEQ ID NO: 208 or any variant thereof;
in some preferred embodiments, the antibody or antigen-binding portion thereof comprises the heavy chain variable region of the amino acid sequence SEQ ID NO: 175 or any variant thereof, and the light chain variable region of the amino acid sequence SEQ ID NO: 210 or any variant thereof;
in some preferred embodiments, the antibody or antigen-binding portion thereof comprises the heavy chain variable region of the amino acid sequence SEQ ID NO: 175 or any variant thereof, and the light chain variable region of the amino acid sequence SEQ ID NO: 212 or any variant thereof;
in some preferred embodiments, the antibody or antigen-binding portion thereof comprises the heavy chain variable region of the amino acid sequence SEQ ID NO: 177 or any variant thereof, and the light chain variable region of the amino acid sequence SEQ ID NO: 208 or any variant thereof;
in some preferred embodiments, the antibody or antigen-binding portion thereof comprises the heavy chain variable region of the amino acid sequence SEQ ID NO: 177 or any variant thereof, and the light chain variable region of the amino acid sequence SEQ ID NO: 210 or any variant thereof;
in some preferred embodiments, the antibody or antigen-binding portion thereof comprises the heavy chain variable region of the amino acid sequence SEQ ID NO: 177 or any variant thereof, and the light chain variable region of the amino acid sequence SEQ ID NO: 212 or any variant thereof;
in some preferred embodiments, the antibody or antigen-binding portion thereof comprises the heavy chain variable region of the amino acid sequence SEQ ID NO: 179 or any variant thereof, and the light chain variable region of the amino acid sequence SEQ ID NO: 208 or any variant thereof;
in some preferred embodiments, the antibody or antigen-binding portion thereof comprises the heavy chain variable region of the amino acid sequence SEQ ID NO: 179 or any variant thereof, and the light chain variable region of the amino acid sequence SEQ ID NO: 210 or any variant thereof;
in some preferred embodiments, the antibody or antigen-binding portion thereof comprises the heavy chain variable region of the amino acid sequence SEQ ID NO: 179 or any variant thereof, and the light chain variable region of the amino acid sequence SEQ ID NO: 212 or any variant thereof.

In one aspect, the present disclosure provides a bispecific or multispecific molecule comprising the antibody or antigen-binding portion thereof of any of the preceding aspects.

In some preferred embodiments of the present disclosure, the antibody or antigen-binding portion thereof is humanized.

In one aspect, the present disclosure provides a nucleic acid molecule encoding the antibody or antigen-binding portion thereof or the bispecific or multispecific molecule according to any of the preceding aspects.

In some preferred embodiments, the nucleic acid comprises an antibody heavy chain variable region nucleotide sequence selected from the group consisting of SEQ ID NOs: 6, 16, 25, 35, 45, 54, 59, 64, 74, 78, 84, 93, 103, 112, 120, 125, 135, 140, 148, 158, 164, 170, 176, 178, 180, 182, 185, 187, 189, 191, 194, 196, 198 or any variant thereof, and/or an antibody light chain variable region nucleotide sequence selected from the group consisting of SEQ ID NOs: 11, 20, 30, 40, 49, 56, 62, 69, 76, 81, 88, 90, 98, 107, 116, 122, 130, 143, 153, 160, 167, 172, 200, 204, 206, 209, 211, 213, 215 or any variant thereof;
in some preferred embodiments, the nucleic acid comprises the heavy chain variable region nucleotide sequence of the nucleotide sequence SEQ ID NO: 6 or any variant thereof, and the light chain variable region nucleotide sequence of the nucleotide sequence SEQ ID NO: 11 or any variant thereof;
in some preferred embodiments, the nucleic acid comprises the heavy chain variable region nucleotide sequence of the nucleotide sequence SEQ ID NO: 16 or any variant thereof, and the light chain variable region nucleotide sequence of the nucleotide sequence SEQ ID NO: 20 or any variant thereof;
in some preferred embodiments, the nucleic acid comprises the heavy chain variable region nucleotide sequence of the nucleotide sequence SEQ ID NO: 25 or any variant thereof, and the light chain variable region nucleotide sequence of the nucleotide sequence SEQ ID NO: 30 or any variant thereof;
in some preferred embodiments, the nucleic acid comprises the heavy chain variable region nucleotide sequence of the nucleotide sequence SEQ ID NO: 35 or any variant thereof, and the light chain variable region nucleotide sequence of the nucleotide sequence SEQ ID NO: 40 or any variant thereof;
in some preferred embodiments, the nucleic acid comprises the heavy chain variable region nucleotide sequence of the nucleotide sequence SEQ ID NO: 45 or any variant thereof, and the light chain variable region nucleotide sequence of the nucleotide sequence SEQ ID NO: 49 or any variant thereof;
in some preferred embodiments, the nucleic acid comprises the heavy chain variable region nucleotide sequence of the nucleotide sequence SEQ ID NO: 54 or any variant thereof, and the light chain variable region nucleotide sequence of the nucleotide sequence SEQ ID NO: 56 or any variant thereof;
in some preferred embodiments, the nucleic acid comprises the heavy chain variable region nucleotide sequence of the nucleotide sequence SEQ ID NO: 59 or any variant thereof, and the light chain variable region nucleotide sequence of the nucleotide sequence SEQ ID NO: 62 or any variant thereof;
in some preferred embodiments, the nucleic acid comprises the heavy chain variable region nucleotide sequence of the nucleotide sequence SEQ ID NO: 64 or any variant thereof, and the light chain variable region nucleotide sequence of the nucleotide sequence SEQ ID NO: 69 or any variant thereof;
in some preferred embodiments, the nucleic acid comprises the heavy chain variable region nucleotide sequence of the nucleotide sequence SEQ ID NO: 74 or any variant thereof, and the light chain variable region nucleotide sequence of the nucleotide sequence SEQ ID NO: 76 or any variant thereof;
in some preferred embodiments, the nucleic acid comprises the heavy chain variable region nucleotide sequence of the nucleotide sequence SEQ ID NO: 78 or any variant thereof, and the light chain variable region nucleotide sequence of the nucleotide sequence SEQ ID NO: 81 or any variant thereof;
in some preferred embodiments, the nucleic acid comprises the heavy chain variable region nucleotide sequence of the nucleotide sequence SEQ ID NO: 84 or any variant thereof, and the light chain variable region nucleotide sequence of the nucleotide sequence SEQ ID NO: 88 or any variant thereof;
in some preferred embodiments, the nucleic acid comprises the heavy chain variable region nucleotide sequence of the nucleotide sequence SEQ ID NO: 84 or any variant thereof, and the light chain variable region nucleotide sequence of the nucleotide sequence SEQ ID NO: 90 or any variant thereof;
in some preferred embodiments, the nucleic acid comprises the heavy chain variable region nucleotide sequence of the nucleotide sequence SEQ ID NO: 93 or any variant thereof, and the light chain variable region nucleotide sequence of the nucleotide sequence SEQ ID NO: 98 or any variant thereof;
in some preferred embodiments, the nucleic acid comprises the heavy chain variable region nucleotide sequence of the nucleotide sequence SEQ ID NO: 103 or any variant thereof, and the light chain variable region nucleotide sequence of the nucleotide sequence SEQ ID NO: 107 or any variant thereof;
in some preferred embodiments, the nucleic acid comprises the heavy chain variable region nucleotide sequence of the nucleotide sequence SEQ ID NO: 112 or any variant thereof, and the light chain variable region nucleotide sequence of the nucleotide sequence SEQ ID NO: 116 or any variant thereof;
in some preferred embodiments, the nucleic acid comprises the heavy chain variable region nucleotide sequence of the nucleotide sequence SEQ ID NO: 120 or any variant thereof, and the light chain variable region nucleotide sequence of the nucleotide sequence SEQ ID NO: 122 or any variant thereof;
in some preferred embodiments, the nucleic acid comprises the heavy chain variable region nucleotide sequence of the nucleotide sequence SEQ ID NO: 125 or any variant thereof, and the light chain variable region nucleotide sequence of the nucleotide sequence SEQ ID NO: 130 or any variant thereof;
in some preferred embodiments, the nucleic acid comprises the heavy chain variable region nucleotide sequence of the nucleotide sequence SEQ ID NO: 135 or any variant thereof, and the light chain variable region nucleotide sequence of the nucleotide sequence SEQ ID NO: 130 or any variant thereof;
in some preferred embodiments, the nucleic acid comprises the heavy chain variable region nucleotide sequence of the nucleotide sequence SEQ ID NO: 140 or any variant thereof, and the light chain variable region nucleotide sequence of the nucleotide sequence SEQ ID NO: 143 or any variant thereof;
in some preferred embodiments, the nucleic acid comprises the heavy chain variable region nucleotide sequence of the nucleotide sequence SEQ ID NO: 148 or any variant thereof, and the light chain variable region nucleotide sequence of the nucleotide sequence SEQ ID NO: 153 or any variant thereof;
in some preferred embodiments, the nucleic acid comprises the heavy chain variable region nucleotide sequence of the nucleotide sequence SEQ ID NO: 158 or any variant thereof, and the light chain variable region nucleotide sequence of the nucleotide sequence SEQ ID NO: 160 or any variant thereof;
in some preferred embodiments, the nucleic acid comprises the heavy chain variable region nucleotide sequence of the nucleotide sequence SEQ ID NO: 164 or any variant thereof, and the light chain variable region nucleotide sequence of the nucleotide sequence SEQ ID NO: 167 or any variant thereof;
in some preferred embodiments, the nucleic acid comprises the heavy chain variable region nucleotide sequence of the nucleotide sequence SEQ ID NO: 170 or any variant thereof, and the light chain variable region nucleotide sequence of the nucleotide sequence SEQ ID NO: 172 or any variant thereof;
in some preferred embodiments, the nucleic acid comprises the heavy chain variable region nucleotide sequence of the nucleotide sequence SEQ ID NO: 182 or any variant thereof, and the light chain variable region nucleotide sequence of the nucleotide sequence SEQ ID NO: 215 or any variant thereof;
in some preferred embodiments, the nucleic acid comprises the heavy chain variable region nucleotide sequence of the nucleotide sequence SEQ ID NO: 185 or any variant thereof, and the light chain variable region nucleotide sequence of the nucleotide sequence SEQ ID NO: 215 or any variant thereof;
in some preferred embodiments, the nucleic acid comprises the heavy chain variable region nucleotide sequence of the nucleotide sequence SEQ ID NO: 187 or any variant thereof, and the light chain variable region nucleotide sequence of the nucleotide sequence SEQ ID NO: 215 or any variant thereof;
in some preferred embodiments, the nucleic acid comprises the heavy chain variable region nucleotide sequence of the nucleotide sequence SEQ ID NO: 189 or any variant thereof, and the light chain variable region nucleotide sequence of the nucleotide sequence SEQ ID NO: 215 or any variant thereof;
in some preferred embodiments, the nucleic acid comprises the heavy chain variable region nucleotide sequence of the nucleotide sequence SEQ ID NO: 191 or any variant thereof, and the light chain variable region nucleotide sequence of the nucleotide sequence SEQ ID NO: 215 or any variant thereof;
in some preferred embodiments, the nucleic acid comprises the heavy chain variable region nucleotide sequence of the nucleotide sequence SEQ ID NO: 194 or any variant thereof, and the light chain variable region nucleotide sequence of the nucleotide sequence SEQ ID NO: 215 or any variant thereof;
in some preferred embodiments, the nucleic acid comprises the heavy chain variable region nucleotide sequence of the nucleotide sequence SEQ ID NO: 196 or any variant thereof, and the light chain variable region nucleotide sequence of the nucleotide sequence SEQ ID NO: 215 or any variant thereof;
in some preferred embodiments, the nucleic acid comprises the heavy chain variable region nucleotide sequence of the nucleotide sequence SEQ ID NO: 198 or any variant thereof, and the light chain variable region nucleotide sequence of the nucleotide sequence SEQ ID NO: 215 or any variant thereof;
in some preferred embodiments, the nucleic acid comprises the heavy chain variable region nucleotide sequence of the nucleotide sequence SEQ ID NO: 176 or any variant thereof, and the light chain variable region nucleotide sequence of the nucleotide sequence SEQ ID NO: 200 or any variant thereof;
in some preferred embodiments, the nucleic acid comprises the heavy chain variable region nucleotide sequence of the nucleotide sequence SEQ ID NO: 176 or any variant thereof, and the light chain variable region nucleotide sequence of the nucleotide sequence SEQ ID NO: 204 or any variant thereof;
in some preferred embodiments, the nucleic acid comprises the heavy chain variable region nucleotide sequence of the nucleotide sequence SEQ ID NO: 176 or any variant thereof, and the light chain variable region nucleotide sequence of the nucleotide sequence SEQ ID NO: 206 or any variant thereof;
in some preferred embodiments, the nucleic acid comprises the heavy chain variable region nucleotide sequence of the nucleotide sequence SEQ ID NO: 178 or any variant thereof, and the light chain variable region nucleotide sequence of the nucleotide sequence SEQ ID NO: 206 or any variant thereof;
in some preferred embodiments, the nucleic acid comprises the heavy chain variable region nucleotide sequence of the nucleotide sequence SEQ ID NO: 178 or any variant thereof, and the light chain variable region nucleotide sequence of the nucleotide sequence SEQ ID NO: 200 or any variant thereof;
in some preferred embodiments, the nucleic acid comprises the heavy chain variable region nucleotide sequence of the nucleotide sequence SEQ ID NO: 178 or any variant thereof, and the light chain variable region nucleotide sequence of the nucleotide sequence SEQ ID NO: 204 or any variant thereof;
in some preferred embodiments, the nucleic acid comprises the heavy chain variable region nucleotide sequence of the nucleotide sequence SEQ ID NO: 180 or any variant thereof, and the light chain variable region nucleotide sequence of the nucleotide sequence SEQ ID NO: 200 or any variant thereof;
in some preferred embodiments, the nucleic acid comprises the heavy chain variable region nucleotide sequence of the nucleotide sequence SEQ ID NO: 180 or any variant thereof, and the light chain variable region nucleotide sequence of the nucleotide sequence SEQ ID NO: 204 or any variant thereof;
in some preferred embodiments, the nucleic acid comprises the heavy chain variable region nucleotide sequence of the nucleotide sequence SEQ ID NO: 180 or any variant thereof, and the light chain variable region nucleotide sequence of the nucleotide sequence SEQ ID NO: 206 or any variant thereof;
in some preferred embodiments, the nucleic acid comprises the heavy chain variable region nucleotide sequence of the nucleotide sequence SEQ ID NO: 176 or any variant thereof, and the light chain variable region nucleotide sequence of the nucleotide sequence SEQ ID NO: 209 or any variant thereof;
in some preferred embodiments, the nucleic acid comprises the heavy chain variable region nucleotide sequence of the nucleotide sequence SEQ ID NO: 176 or any variant thereof, and the light chain variable region nucleotide sequence of the nucleotide sequence SEQ ID NO: 211 or any variant thereof;
in some preferred embodiments, the nucleic acid comprises the heavy chain variable region nucleotide sequence of the nucleotide sequence SEQ ID NO: 176 or any variant thereof, and the light chain variable region nucleotide sequence of the nucleotide sequence SEQ ID NO: 213 or any variant thereof;
in some preferred embodiments, the nucleic acid comprises the heavy chain variable region nucleotide sequence of the nucleotide sequence SEQ ID NO: 178 or any variant thereof, and the light chain variable region nucleotide sequence of the nucleotide sequence SEQ ID NO: 209 or any variant thereof;
in some preferred embodiments, the nucleic acid comprises the heavy chain variable region nucleotide sequence of the nucleotide sequence SEQ ID NO: 178 or any variant thereof, and the light chain variable region nucleotide sequence of the nucleotide sequence SEQ ID NO: 211 or any variant thereof;
in some preferred embodiments, the nucleic acid comprises the heavy chain variable region nucleotide sequence of the nucleotide sequence SEQ ID NO: 178 or any variant thereof, and the light chain variable region nucleotide sequence of the nucleotide sequence SEQ ID NO: 213 or any variant thereof;
in some preferred embodiments, the nucleic acid comprises the heavy chain variable region nucleotide sequence of the nucleotide sequence SEQ ID NO: 180 or any variant thereof, and the light chain variable region nucleotide sequence of the nucleotide sequence SEQ ID NO: 209 or any variant thereof;
in some preferred embodiments, the nucleic acid comprises the heavy chain variable region nucleotide sequence of the nucleotide sequence SEQ ID NO: 180 or any variant thereof, and the light chain variable region nucleotide sequence of the nucleotide sequence SEQ ID NO: 211 or any variant thereof;
in some preferred embodiments, the nucleic acid comprises the heavy chain variable region nucleotide sequence of the nucleotide sequence SEQ ID NO: 180 or any variant thereof, and the light chain variable region nucleotide sequence of the nucleotide sequence SEQ ID NO: 213 or any variant thereof.
in one aspect, the present disclosure provides an antibody or antigen-binding portion thereof that binds to MASP-2, having at least greater than 60%, 65%, 70%, 75%, 80%, 85%, 90%, 95%, 96%, 97%, 98%, 99%, or more sequence identity to the antibody or antigen-binding portion thereof of any of the preceding aspects.

In one aspect, the present disclosure provides a nucleic acid molecule encoding the antibody or antigen-binding portion thereof of any of the preceding aspects, or a nucleic acid molecule having at least greater than 60%, 65%, 70%, 75%, 80%, 85%, 90%, 95%, 96%, 97%, 98%, 99%, or more sequence identity thereto.

In one aspect, the present disclosure provides a vector comprising the nucleic acid of any of the preceding aspects.

In one aspect, the present disclosure provides a cell comprising the vector of any of the preceding aspects.

In one aspect, the present disclosure provides a composition comprising the aforementioned antibody or antigen-binding portion thereof, bispecific or multispecific molecule, nucleic acid, vector, and/or cell.

The antibodies of the present disclosure are useful as therapeutic or diagnostic tools in a variety of diseases in which MASP-2 is adversely expressed or found.

A method of treating diseases associated with MASP-2-dependent complement activation with the MASP-2 inhibitor of the present disclosure, comprising the steps of administering a therapeutically effective amount of the antibody or antigen-binding fragment thereof or nucleic acid molecule or vector or cell or pharmaceutical composition of any of the preceding aspects to a subject.

MASP-2 dependent complement activation has been indicated to contribute to the pathogenesis of a number of acute and chronic disease states, including MASP-2 dependent complement-mediated vascular conditions, ischemia-reperfusion injury, atherosclerosis, inflammatory gastrointestinal tract disorders, lung conditions, in vitro reperfusion processes, skeletal muscle conditions, kidney conditions, skin conditions, organ or tissue transplantation, neurological disorders or injuries, blood disorders, urogenital tract conditions, diabetes, chemotherapy or radiation therapy, malignancies, endocrine disorders, coagulation disorders, or ophthalmic conditions. Thus, the MASP-2 inhibitory antibodies of the present disclosure can be used to treat the diseases and conditions described above.

The clinical condition of a preferred embodiment of the present disclosure is a chronic inflammatory disease. Chronic inflammatory disease may be, for example, an autoimmune inflammatory condition.

Autoimmune inflammatory conditions (also referred to hereby as "autoimmune disorders ") can be loosely classified into diseases that are primarily limited to specific organs or tissues and that affect the entire body. Examples of organ-specific disorders (and affected organs) comprise multiple sclerosis (myelin coated on neurites), type I diabetes (pancreas), Hashimoto's thyroiditis (thyroid), pernicious anemia (stomach), Addison's disease (adrenal gland), myasthenia gravis (acetylcholine receptors on neuromuscular junctions), rheumatoid arthritis (joint lining), uveitis (eye), psoriasis (skin), Guillain-Barre syndrome (nerve cell) and Graves' disease (thyroid). Systemic autoimmune diseases comprise systemic lupus erythematosus, glomeronephritis, and dermatomyositis.

In one embodiment of the present disclosure, the preferred clinical condition is selected from the group consisting of rheumatoid arthritis or systemic lupus erythematosus.

Other examples of autoimmune disorders comprise asthma, eczema, atopicaldermatitis, contact dermatitis, other eczematous dermatitises, seborrheic dermatitis, rhinitis, lichen planus, pemplugus, bullous pemphigoid, epidermolysis bullosa, urticaria, angioedema, vasculitis, erythema, cutaneous eosinophilia, alopecia areata, arteriosclerosis, primary biliary cirrhosis and nephrotic syndrome. Related diseases comprise enteritis, such as coeliac disease, proctitis, eosinophilic gastroenteritis, mastocytosis, inflammatory bowel disease, Chrohn's disease, ulcerative colitis, and food-related allergies.

In another embodiment of the present disclosure, the clinical condition is characterized by massive cell loss, e.g., due to programmed cell death or necrosis. Necrosis or programmed cell death can be induced by a number of factors.

In a preferred embodiment of the present disclosure, the clinical condition is ischemic/reperfusion injury. Ischemia can be caused by a variety of causes, such as after stroke, myocardial infarction, major surgery, or organ transplantation. Thus, the clinical condition may be an ischemic/reperfusion injury caused by, for example, stroke, myocardial infarction, major surgery, or organ transplantation.

Thus, the clinical condition may be an ischemic/reperfusion injury, which is the result of PTCA (percutaneous transluminal coronary angioplasty) or CABG (coronary artery bypass grafting). Furthermore, the clinical condition may be ischemic/reperfusion injury, which is the result of acute myocardial infarction or cerebral ischemia.

The dosage of the agents comprising the MASP-2 inhibitor generally varies according to the age, body weight, height, gender, general disease condition and medical history of the subject to be treated. For example, the MASP-2 inhibitor, e.g., the MASP-2 antibody, can be administered in a dosage range of about 0.010-10.0 mg/kg, preferably 0.010-1.0 mg/kg, more preferably 0.010-0.1 mg/kg of the body weight of the subject to be treated. In certain embodiments, the composition comprises the combination of the anti-MASP-2 antibody and a MASP-2 inhibitory peptide.

Compositions and methods comprising the MASP-2 inhibitor may optionally comprise one or more additional therapeutic agents, which may enhance the activity of the MASP-2 inhibitor or provide relevant therapeutic functions in an additive or synergistic manner. For example, one or more MASP-2 inhibitors may be combined with one or more anti-inflammatory and/or analgesic agents. The amount and choice of additional agents can be determined to achieve the desired therapeutic results. Suitable anti-inflammatory and/or analgesic agents comprise: 5-hydroxytryptamine receptor antagonists; 5-hydroxytryptamine receptor agonists; histamine receptor antagonists; bradykinin receptor antagonists; kallikrein inhibitors; tachykinin receptor antagonists including neurokinin 1 and neurokinin 2 receptor subtype antagonists; calcitonin gene-related peptide (CGRP) receptor antagonists; interleukin receptor antagonists; inhibitors of active enzymes in the arachidonic acid metabolite synthesis pathway, including phospholipase inhibitors, including inhibitors of the PLA2 isoform and PLC gamma isoform, cyclooxygenase (COX) inhibitors (which may be inhibitors of COX-1, COX-2 or non-selective inhibitors of COX-1 and COX-2), lipoxygenase inhibitors; prostanoid receptor antagonists, including eicosanoid EP-I and EP-4 receptor subtype antagonists and thromboxane receptor subtype antagonists; leukotriene receptor antagonists, including leukotriene B4 receptor subtype antagonists and leukotriene D4 receptor subtype antagonists; opioid receptor agonists, including µ-opioid, δ-opioid and κ-opioid receptor subtype agonists; purine receptor agonists and antagonists, including P2x receptor antagonists and P2Y receptor agonists; adenosine triphosphate (ATP) sensitive potassium channel openers; MAP kinase inhibitors; nicotinic acetylcholine inhibitors; and alpha adrenergic receptor agonists (including alpha-1, alpha-2 and non-selective alpha-1 and alpha-2 agonists).

When used to inhibit or treat restenosis, the MASP-2 inhibitors of the present disclosure may be co-administered in combination with one or more anti-restenosis agents. Suitable anti-restenosis agents comprise antiplatelet agents, including thrombin inhibitors and receptor antagonists, adenosine diphosphate (ADP) receptor antagonists (also known as purinergic receptor 1 receptor antagonists), thromboxane inhibitors and receptor antagonists, and platelet membrane glycoprotein receptor antagonists; inhibitors of cell adhesion molecules, including selectin inhibitors and integrin free radicals; anti-chemotactic agents; interleukin receptor antagonists; and intracellular signal transduction inhibitors, including protein kinase C (PKC) inhibitors and protein tyrosine phosphatases, modulators of intracellular protein tyrosine kinase inhibitors, inhibitors of src homology 2 (SH2) domains, and calcium channel antagonists.

The MASP-2 inhibitors of the present disclosure may also be used in combination with one or more other complement inhibitors. Complement inhibitors have not been approved for use in the human body, but some agents have been shown to block complement in vivo. Many of such agents are also toxic, or only partial inhibitors, and their use is limited to being used as research tools. K76COOH and nafamostat mesylate are two agents that have certain therapeutic effects in animal models of transplantation. Studies have shown that low molecular weight heparin also effectively modulates complement activity. We believe that these small molecule inhibitors may be used as agents in combination with the MASP-2 inhibitors of the present disclosure.

Other naturally occurring complement inhibitors may be used in combination with the MASP-2 inhibitors of the present disclosure. The biological inhibitor of complement comprises soluble complement factor 1 (sCR1). This is a naturally occurring inhibitor that may be found on the outer membrane of human cells. Other membrane inhibitors include DAF, MCP, and CD59. Recombinant forms have been assayed for anti-complement activity in vitro and in vivo. sCR1 was shown to be effective in xenografts where the complement system (both alternative and classical) causes hyperreactive rejection syndrome within a few minutes of perfusion of blood into the newly transplanted organ. The use of sCR1 protects and extends the survival time of the transplanted organ, suggesting a complement pathway in organ survival mechanisms.

Other complement inhibitors suitable for use in combination with the compositions of the present disclosure also comprise, for example, the monoclonal antibody (MoAb) being developed by Alexion Pharmaceuticals, Inc. New Haven, Connecticut, and the anti-properdin monoclonal antibody (MoAb).

When used to treat arthritis (e.g., osteoarthritis and rheumatoid arthritis), the MASP-2 inhibitors of the present disclosure may be used in combination with one or more chondroprotective agents, which may include one or more cartilage anabolism promoters and/or one or more cartilage catabolism inhibitors, suitably including both an anabolic agent and a catabolic inhibitory agent administered simultaneously. Suitable chondroprotective agents that promote anabolism comprise interleukin (IL) receptor agonists, including IL-4, IL-10, IL-13, rhIL-4, rhIL-10 and rhIL-13 as well as chimeric IL-4, IL-10 or IL-13; transforming growth factor β superfamily agonists (including TGF-β, TGF-β1, TGF-β2, TGF-β3), bone morphogenetic proteins including BMP-2, BMP-4, BMP-5, BMP-6, BMP-7 (OP-1) and OP-2/BMP-8, growth differentiation factors including GDF-5, GDF-6 and GDF-7, recombinant TGF-β and BMP, and chimeric TGF-β and BMP; insulin-like growth factors, including IGF-1; and fibroblast growth factors, including bFGF. Suitable chondroprotective agents that inhibit catabolism comprise interleukin-1 (IL-1) receptor antagonists (IL-lra), including soluble human IL-1 receptor (shuIL-IR), rshuIL-IR, rhIL-lra, anti-IL1 antibodies, AF11567 and AF12198; tumor necrosis factor (TNF) receptor antagonists (TNF-alpha), soluble receptors including sTNFRI and sTNFRII, recombinant TNF soluble receptors and chimeric TNF soluble receptors including chimeric rhTNFR: Fc, Fc fusion soluble receptors and anti-TNF antibodies; cyclooxygenase-2 (COX-2 specific) inhibitors; including DuP 697, SC-58451, celecoxib, rofecoxib, nimesulide, diclofenac, meloxicam, piroxicam, NS-398, RS-57067, SC-57666, SC-58125, flosulide, etodolac, L-745, 337 and DFU-T-614; mitogen-activated protein kinase (MAPK) inhibitors, including inhibitors of ERKi, ERK2, SAPK1, SAPK2a, SAPK2b, SAPK2d, SAPK3, including SB 203580, SB 203580 iodine, SB202190, SB 242235, SB 220025, RWJ 67657, RWJ 68354, FR 133605, L-167307, PD 98059, PD 1693 16; nuclear factor kappa B (NPκB) inhibitors, including caffeic acid phenethyl ester (CAPE), DM-CAPE, SN-50 peptide, hymenialdisine, and pyrrolidone dithiocarbamate; nitric oxide synthase (NOS) inhibitors, including NG-monomethyl-L-arginine, 1400W, diphenylene iodide, S-methylisothiourea, S-(aminoethyl) isothiourea, L-N6-(l-iminoethyl) lysine, 1,3-PBITU, 2-ethyl-2-thiopseudourea, aminoguanidine, Nω-nitro-L-arginine, methyl Nω-nitro-L-arginine, inhibitors of matrix metalloproteinases (MMP), including inhibitors of MMP-1, MMP-2, MMP-3, MMP-7, MMP-8, MMP-9, MMP-10, MMP-11, MMP-12, MMP-13, MMP-14 and MMP-15, including U-24522, minocycline, 4-Abz-Gly-Pro-D-Leu-D-Ala-NHOH, Ac-Arg-Cys-Gly-Val-Pro-Asp-NH2, recombinant human TIMP1, recombinant human TIMP2 and phosphoramidon; cell adhesion molecules, including integrin agonists and antagonists, including αVβ3 MoAb LM 609 and echistatin; anti-chemotactic agents, including F-Met-Leu-Phe receptors, IL-8 receptors, MCP-1 receptors and MIP1-I/RANTES receptors; intracellular signal transduction inhibitors, including (a) protein kinase inhibitors, including (i) protein kinase C (PKC) inhibitors (isozymes) including calphostin C, G-6203 and GF109203 X and (ii) protein tyrosine kinase inhibitors; (b) intracellular protein tyrosine phosphatase (PTP enzyme) modulators; and (c) SH2 domain inhibitors (src homology 2 domains).

For some applications, it may be advantageous to combine the MASP-2 inhibitors of the present disclosure with a spasm inhibitor. For example, for urogenital applications, it may be advantageous to comprise at least one smooth muscle spasm inhibitor and/or at least one anti-inflammatory agent, and for vascular surgery, it may be beneficial to comprise at least one vasospasm inhibitor and/or at least one anti-inflammatory agent and/or at least one anti-restenosis agent. Suitable examples of the spasm inhibitor include 5-hydroxytryptamine 2 receptor subtype antagonists; tachykinin receptor antagonists; nitric oxide donors; ATP sensitive potassium channel openers; calcium channel antagonists; and endothelin receptor antagonists.

In one embodiment of the present disclosure, the antibody is administered in a single dose or multiple doses.

If the aforementioned antibody is administered in multiple doses according to the present disclosure, the antibody is preferably administered in at least 3 doses, at least 4 doses, at least 5 doses, at least 6 doses, at least 7 doses, at least 8 doses, at least 9 doses, or at least 10 doses and preferably at most 30 doses, 25 doses, 20 doses, 15 doses, or 10 doses. The dose of the aforementioned antibody is preferably administered at intervals of at least 7 days, at least 10 days, at least 14 days, or at least 20 days. Dose of the aforementioned antibody is preferably administered at intervals of 7 to 30 days, 10 to 20 days, and preferably about 14 days.

In one aspect, the present disclosure provides a use of the antibody or antigen-binding fragment thereof, nucleic acid molecule, vector, cell or pharmaceutical composition of any of the preceding aspects in the preparation of a drug for treating diseases associated with MASP-2 dependent complement activation in a subject.

In one aspect, the present disclosure provides a kit comprising the aforementioned antibody or antigen-binding portion thereof, bispecific or multispecific molecule, nucleic acid, vector, cell, and/or composition.

The disclosed kit comprises the antibodies, fragments, homologs, derivatives thereof, etc., of the present disclosure, e.g., the labeled or cytotoxic conjugates, as well as the instructions for use of the antibodies, the conjugates to kill a particular type of cell, and the like. The instructions may comprise directions for using the antibodies, conjugates, etc. in vitro, in vivo, or ex vivo. The antibodies may be in liquid form or solid form, usually lyophilized. The kit may comprise other suitable reagents, such as buffers, reconstitution solutions, and other necessary components for the intended use. Combinations of reagents packaged in predetermined amounts with instructions for their use, e.g., for therapeutic use or for conducting diagnostic assays, are contemplated. When the antibody is labeled, e.g., with an enzyme, then the kit may comprise a substrate and cofactors required for the enzyme (e.g., a substrate precursor providing a detectable chromophore or fluorophore). In addition, other additives, such as stabilizers, buffers (e.g., blocking buffers or lysis buffers), and the like, may also be included. The relative amounts of the various reagents can be varied to provide a concentrate of reagent solution, which provides user flexibility, and savings space and reagent. These reagents may also be provided in dry powder form, usually in lyophilized form, including excipients which, when dissolved, provide a reagent solution having the appropriate concentration.

In one aspect, the present disclosure provides a use of the aforementioned antibody or antigen-binding portion thereof, bispecific or multispecific molecule, nucleic acid molecule, vector, cell and/or composition in the preparation of a drug or kit for diagnosing, treating or preventing diseases associated with MASP-2 dependent complement activation.

In addition, the antibodies of the present disclosure may also be used in immunoassays, purification methods, and other methods using immunoglobulins or fragments thereof. Such uses are well-known in the art.

Accordingly, the present disclosure further provides a composition comprising the anti-MASP-2 antibody of the present disclosure, or fragment thereof, conveniently in combination with pharmaceutically acceptable carriers, diluents, or excipients, as is conventional in the art.

As used herein, the term "pharmaceutical composition" refers to formulations of various preparations. Formulations comprising therapeutically effective amounts of multivalent antibodies are in a sterile liquid solution, liquid suspension, or lyophilized form, optionally comprising stabilizers or excipients.

It will be appreciated that therapeutic agents according to the preceding embodiments will be administered with suitable pharmaceutically acceptable carriers, excipients, and other agents incorporated into formulations to provide improved transfer, delivery, tolerability, and the like. A large number of suitable formulations can be found in all pharmacopeias known to pharmaceutical chemists: Remington's Pharmaceutical Sciences (15th ed. Mack Publishing Company, Easton, Pa. (1975)), particularly Chapter 87: Blaug, Seymour. These formulations comprise, for example, powders, pastes, ointments, gels, waxes, oils, lipids, lipid-containing (cationic or anionic) carriers (e.g., Lipofectin^{™}), DNA conjugates, anhydrous syrups, oil-in-water and water-in-oil emulsions, emulsion polyethylene glycols (polyethylene glycols with various molecular weights), semi-solid gels, and semi-solid mixtures containing polyethylene glycol. Any of the aforementioned mixtures may be suitable for the treatment or therapy according to the present disclosure, provided that the active ingredient in the formulation is not inactivated by the formulation and that the formulation is physiologically compatible and tolerates the route of administration.

In another embodiment, antibodies against MASP-2 may be used in methods known in the art relating to localization and/or quantitation of MASP-2 (e.g., for determining MASP-2 and/or levels of MASP-2 in an appropriate physiological sample, for diagnostic methods, for protein imaging, etc.). In a given embodiment, the antibody comprising an antibody-derived antigen-binding domain specific for MASP-2 or derivatives, fragments, analogs or homologs thereof is used as a pharmaceutically active compound (hereinafter referred to as "therapeutic agent").

In another embodiment, MASP-2 polypeptides may be isolated using antibodies specific for MASP-2 by standard techniques such as immunoaffinity, chromatography, or immunoprecipitation. The proteins in a biological sample may be detected with antibodies (or fragments thereof) against the MASP-2 protein. In some embodiments, MASP-2 may be detected in a biological sample as part of a clinical testing procedure, for example, to determine the efficacy of a given therapeutic regimen. Detection may be facilitated by coupling (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazine aminofluorescein, dansyl chloride, or phycoerythrin; one example of luminescent materials includes luminol; examples of bioluminescent materials include luciferase, luciferin and aequorin, and examples of suitable radioactive materials include ¹²⁵I, ¹³¹I, ³⁵S, or ³H.

In another embodiment, the antibody according to the present disclosure may be used as reagents for detecting the presence of MASP-2 or a protein fragment thereof in a sample. In some embodiments, the antibody comprises a detectable label. The antibody is a polyclonal antibody, or more preferably a monoclonal antibody. Intact antibodies or fragments thereof (e.g., Fab, scFv, or F(ab')₂) are used. The term "labeling" with respect to an antibody is intended to encompass direct labeling of the antibody by coupling (i.e., physically linking) a detectable substance to the antibody, as well as indirect labeling of the antibody by reaction with another reagent that is directly labeled. Examples of indirect labeling include detection of a primary antibody using a fluorescently labeled secondary antibody, and end-labeling of the antibody with biotin to enable detection with fluorescently labeled streptavidin. The term "biological sample" is intended to comprise tissues, cells, and biological fluids isolated from a subject, as well as tissues, cells, and fluids present in a subject. Thus, the term "biological sample" used comprises blood and fractions or components in blood, including serum, plasma, or lymph. In other words, the detection method of the preceding embodiments may be used to detect the mRNA, protein, or genomic DNA analytes in a biological sample both in vitro and in vivo. For example, in vitro techniques for the detection of mRNA analytes include Northern hybridization and in situ hybridization. In vitro techniques for the detection of protein analytes include enzyme-linked immunosorbent assay (ELISA), Western blot, immunoprecipitation, and immunofluorescence. In vitro techniques for the detection of genomic DNA analytes include Southern hybridization. Procedures for performing immunoassay are described, for example, in "ELISA: Theory and Practice: Methods in Molecular Biology", vol. 42, J. R. Crowther (ed.) Human Press, Totowa, N. J. 1995; "Immunoassay", E. Diamandis and T. Christopoulus, Academic Press, Inc., San Diego, Calif., 1996; and "Practice and Theory of Enzyme Immunoassays", P. Tijssen, Elsevier Science Publishers, Amsterdam, 1985. In addition, in vivo techniques for the detection of protein analytes include introducing a labeled anti-analyte protein antibody into a subject. For example, an antibody can be labeled with a radiolabel, and the presence and location of the radiolabel in the subject can then be detected by standard imaging techniques.

The antibodies described herein and derivatives, fragments, analogs, and homologs thereof may be incorporated into pharmaceutical compositions suitable for administration. The principles and considerations involved in preparing such compositions, as well as guidance in selecting components, are well-known in the art.

Such compositions typically comprise the antibody and a pharmaceutically acceptable carrier. When the antibody fragments are used, minimal inhibitory fragments that specifically bind the target protein binding domain may be preferred. For example, based on the variable region sequence of the antibody, peptide molecules can be designed to retain the ability to bind a target protein sequence. Such peptides can be chemically synthesized and/or produced by recombinant DNA technique (see, e.g., Marasco et al., Proc. Natl. Acad. Sci. USA, 90:7889-7893 (1993)).

As used herein, the term "pharmaceutically acceptable carrier" is intended to comprise any and all solvents, dispersion media, coatings, antibacterial and antifungal agents, isotonic agents and absorption delaying agents, etc., compatible with pharmaceutical administration. Suitable pharmaceutically acceptable carriers are described in the latest edition of Remington's Pharmaceutical Sciences, a standard bibliography in the art, which is incorporated herein by reference. Preferred examples of such carriers or diluents include, but are not limited to, water, saline, Ringer's solution, dextrose solution, and 5% human serum albumin. Liposomes and non-aqueous vehicles such as fixed oils can also be used. The use of such media and agents for pharmaceutically active substances is well-known in the art. Except insofar as any conventional media or agent is incompatible with the antibody, its use in the compositions is contemplated.

The pharmaceutical compositions of the preceding embodiments are formulated to be compatible with their intended route of administration. Examples of routes of administration include parenteral, e.g., intravenous, intradermal, subcutaneous, oral (e.g., inhalation), transdermal (i.e., topical), transmucosal, and rectal administration. Solutions or suspensions for parenteral, intradermal or subcutaneous administration may comprise the following components: sterile diluents for injection such as water, saline solutions, fixed oils, polyethylene glycols, glycerol, propylene glycol or other synthetic solvents; antibacterial agents such as benzyl alcohol or methyl p-hydroxybenzoate; antioxidants such as ascorbic acid or sodium bisulfite; chelating agents such as ethylenediamine tetraacetic acid (EDTA); buffers, such as acetates, citrates, or phosphates, and reagents to adjust the osmotic pressure, such as sodium chloride or dextrose. The pH may be adjusted with acids or bases, such as hydrochloric acid or sodium hydroxide. The parenteral preparation may be packaged in an ampoule, disposable syringe, or multiple dose vials made of glass or plastic.

Pharmaceutical compositions suitable for injectable use comprise sterile aqueous solutions (where is water soluble) or dispersions and sterile powders for the extemporaneous preparation of sterile injectable solution or dispersion. For intravenous administration, suitable pharmaceutically acceptable carriers include physiological saline, bacteriostatic water, Cremophor EL^{™} (BASF, Parsippany, N. J.), or phosphate-buffered saline (PBS). In all cases, the composition must be sterile and should be fluid to the extent that easy syringeability exists. It must be stable under the conditions of manufacture and storage and must be preserved against contamination from microorganisms such as bacteria and fungi. The carrier may be a solvent or dispersion medium containing, for example, water, ethanol, polyol (e.g., glycerol, propylene glycol, and liquid polyethylene glycol, etc.), and suitable mixtures thereof. For example, the desired particle size can be maintained in the case of dispersions by the use of coatings such as lecithin, and proper fluidity can be maintained by the use of surfactants. Prevention of the action of microorganisms may be achieved by various antibacterial and antifungal agents, for example, parabens, chlorobutanol, phenol, ascorbic acid, thimerosal, etc. In many cases, it will be preferable to comprise isotonic agents, e.g., sugars, polyalcohols such as mannitol, sorbitol, and sodium chloride in the composition. The absorption of the injectable compositions can be prolonged by encompassing in the aforementioned composition an agent that delays absorption, for example, aluminum monostearate and gelatin.

Sterile injectable solutions can be prepared by incorporating the antibody in the required amount in an appropriate solvent with one or a combination of ingredients enumerated above, as required, followed by filtered sterilization. Generally, dispersions are prepared by incorporating the antibody into a sterile vehicle that contains a basic dispersion medium and the required other ingredients from those enumerated above. In the case of sterile powders for the preparation of sterile injectable solutions, the preparation method is vacuum drying and freeze-drying which yields a powder comprising the active ingredient and any additional desired ingredient from a sterile-filtered solution of those aforementioned ingredients.

For administration by inhalation, the compounds are delivered in the form of an aerosol spray from a pressurized container or dispenser or a nebulizer containing a suitable propellant, e.g., a gas such as carbon dioxide.

Systemic administration may also be carried out by transmucosal or transdermal means. For transmucosal or transdermal administration, penetrants appropriate to permeate the barrier are used in the formulation. Such penetrants are generally known in the art, and include, for example, detergents, bile salts, and fusidic acid derivatives for transmucosal administration. Transmucosal administration may be accomplished through the use of nasal sprays or suppositories. For transdermal administration, one or more of the aforementioned antibodies may be formulated into pastes, ointments, gels, or creams as generally known in the art.

The compounds may also be prepared in the form of suppositories (e.g., with conventional suppository bases such as cocoa butter or other glycerides) or retention enemas for rectal delivery.

In one embodiment, the aforementioned antibodies can be prepared with carriers that prevent their rapid elimination from the body, such as sustained/controlled release formulations, including implants and microencapsulated delivery systems. Biodegradable, biocompatible polymers can be used, such as ethylene-vinyl acetate, polyanhydrides, polyglycolic acid, collagen, polyorthoesters, and polylactic acid. Methods for preparing such formulations will be apparent to those skilled in the art.

It is especially advantageous to formulate parenteral compositions in dosage unit form for ease of administration and uniformity of dosage. Dosage unit form, as used herein, refers to physically discrete units suited as unitary dosages for the subject to be treated; each unit contains a predetermined quantity of one or more of the aforementioned antibodies calculated to produce the desired therapeutic effect in association with the required pharmaceutical carrier. The specification for the dosage unit forms of the preceding embodiments are dictated by and directly dependent on the unique characteristics of antibodies and the specific therapeutic effects to be achieved, and the limitations inherent in the art of formulating such antibodies for treating individuals.

The aforementioned pharmaceutical compositions may be placed in a container, package, or dispenser together with instructions for administration.

The formulations described herein may also comprise more than one of the aforementioned antibodies, preferably those with complementary activities but without adversely affecting each other, depending on the particular situation to be treated. Alternatively or additionally, the compositions may, for example, comprise an agent that enhances its function, such as a cytotoxic agent, a cytokine, a chemotherapeutic agent, or a growth inhibitor. Such molecules are suitably present in combination in amounts effective for the intended purpose. For example, they may be present in combination in a kit or in combination for use.

In one embodiment, one or more of the aforementioned antibodies may be administered in combination therapy, i.e., in combination with other agents, such as therapeutic agents, which can be used to treat pathological conditions or disorders, such as various forms of autoimmune diseases, and inflammatory diseases. The term "in combination" means herein that the agents are administered substantially synchronously, simultaneously, or sequentially. If administered sequentially, the first of the two compounds is still preferably detected at an effective concentration at the treatment site when the second compound is initially administered. In one case, the "combination" can also comprise both the antibody of the present disclosure and another therapeutic agent in the kit.

For example, combination therapy may comprise coformulation and/or coadministration of one or more antibodies described herein with one or more additional therapeutic agents (e.g., one or more cytokine and growth factor inhibitors, immunosuppressants, anti-inflammatory agents, metabolic inhibitors, enzyme inhibitors, and/or cytotoxic or cytostatic agents, as described in more detail below). Such combination therapy may advantageously utilize lower dosages of the administered therapeutic agents, thus avoiding possible toxicities or complications associated with each monotherapy.

Experiments of the present disclosure demonstrate that a variety of monoclonal antibodies and variants thereof provided by the present disclosure that bind to MASP-2 with high affinity and inhibit lectin-mediated complement activation can specifically recognize enzymatic activity domains CCP1-CCP2-SP of human, monkey, mouse and rat MASP-2, and effectively inhibit the production of C4b and C3 convertase C4b2a at a low concentration with a blocking activity 50-200 fold higher than that of the known antibodies. The results of the pre-toxicological study in rhesus monkeys showed that the animals tolerated the high-dose novel MASP-2 antibodies well without any toxic reaction, and its efficacy and safety were superior to those of similar antibodies.

For purposes of clarity and conciseness, features are described herein as part of the same or separate embodiments, however, it will be understood that the scope of the present disclosure may include some embodiments having a combination of all or some of the features described.

### Examples

### Example 1. Lectin pathway MASP-2 activity detection

After recognition of the repeated N-galactosamine or mannose structures by MBL in plasma, serine proteases MASP-1 and MASP-2 can be sequentially activated. The activated MASP-2 cleaves complement components C4 and C2, and the formed C4b is deposited on a well plate, which can be recognized by an anti-C4b antibody; after cleavage of complement component C3 by C3 convertase C4b2a formed by C4 and C2 cleavage products, the formed C3b is deposited on a well plate, which can be recognized by an anti-C3b antibody. Therefore, the inhibitory activity of the MASP-2 antibody against MASP-2 in serum can be demonstrated by detecting the production of C4b or C3b in the lectin-activated reaction.

### C4 cleavage activity assay

20 µg/ml mannan (Sigma, M7504) was added to the ELISA plate and coated overnight at 4°C. After blocking with 2% BSA at room temperature for 60 minutes, 0.5% diluted human serum was added, and overnight at 4°C. After washing, 50 µl/well of different concentrations of MASP-2 antibodies (initial concentration of 30 µg/ml, 5-fold gradient dilution, 9 gradients in total) were added, and pre-incubated at 4°C for 30 min, then 50 µl of 2 µg/ml human C4 protein (CompTech, # A105) was added, and reacted at 37°C for 90 min. After washing the plate, 1:1000 diluted Biotin-labeled human C4b polyclonal antibody (ASSAYPRO, # 11223-05021) was added and incubated at room temperature for 90 min, 1:5000 peroxidase-streptavidin (Jackson ImmunoResearch 016-030-084) was added and incubated at room temperature for 60 min, and then TMB developing solution was added, developed in dark at 37°C for about 10 min. The reaction was stopped and the OD value was read.

### C3 convertase activity assay

20 µg/ml mannan was added to the ELISA plate and coated overnight at 4°C. After blocking with 2% BSA at room temperature for 60 min, MASP-2 antibody diluted with 0.5% human serum (initial concentration of 60 µg/ml, 5-fold gradient dilution, 9 gradients in total) was added and reacted at 37°C for 90 min, 1:500 rabbit anti-human C3/C3b/C3c polyclonal antibody (Proteintech, # 21337-1-AP) was added and incubated at room temperature for 60 min, 1:5000 HRP-labeled goat anti-rabbit secondary antibody (Jackson ImmunoResearch 111-035-144) was added and incubated at room temperature for 60 min, and then TMB developing solution was added, developed in dark at 37°C for about 10 min. The reaction was stopped, and the OD value was read.

### Example 2. Preparation of MASP-2 recombinant proteins

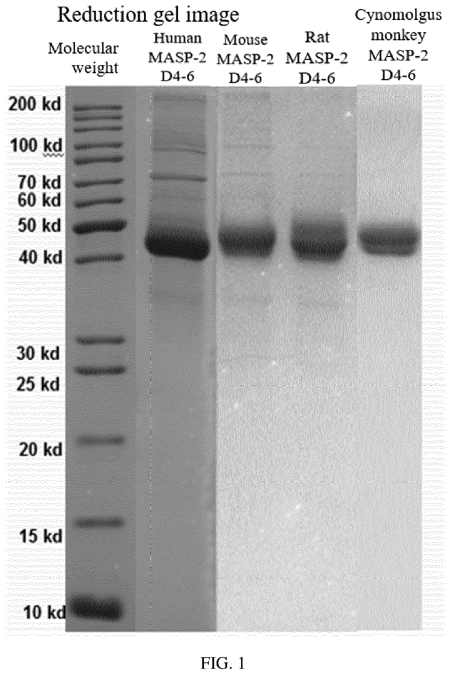

The sequence encoding the enzymatic activity domains CCP1-CCP2-SP of human MASP-2 was cloned into the eukaryotic expression plasmid and fused with his6 tag at N-terminus to facilitate purification. After 7 days of transfection into HEK293E cells, the supernatant of the culture medium was collected and purified by nickel chelating column to obtain the human MASP-2 D4-6 recombinant protein (SEQ ID NO: 1). Similarly, Cynomolgus monkey MASP-2 D4-6 (SEQ ID NO: 2), mouse MASP-2 D4-6 (SEQ ID NO: 3) and rat MASP-2 D4-6 (SEQ ID NO: 4) recombinant proteins were obtained, respectively. FIG. 1 shows the SDS-PAGE result for human, Cynomolgus monkey, mouse and rat MASP-2 D4-6 recombinant proteins.

### Example 3. Immunization of animals and preparation of hybridoma antibodies

BALB/c female mice at 6 weeks of age were selected and immunized subcutaneously or footpad with an equal volume mixture of human or Cynomolgus monkey MASP-2 D4-6 recombinant protein and Freund's adjuvant. Two weeks after the initial immunization, a booster immunization was performed. When the serum titer of the immunized mice reached 1:100000, a shock immunization was performed. Three days later, the spleen and peripheral lymph nodes of the mice are collected and isolated to obtain B cells.

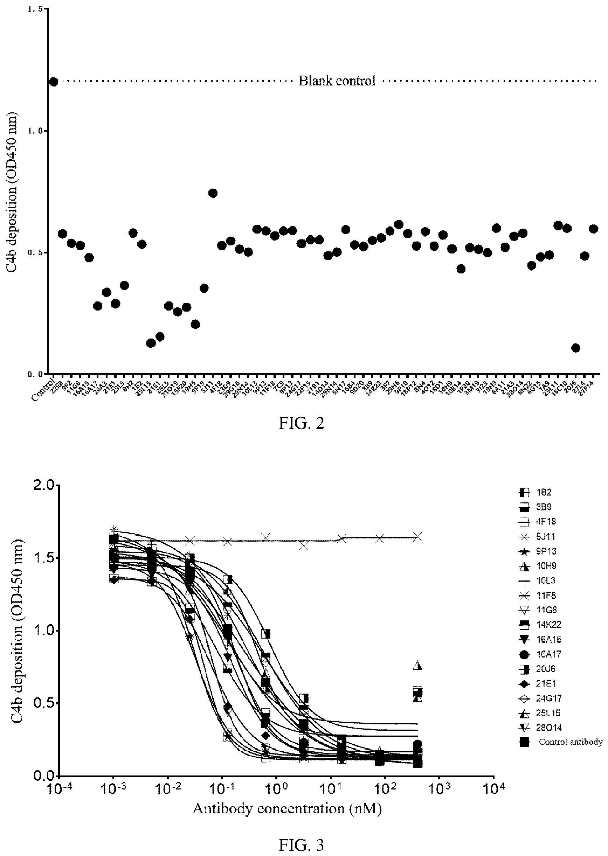

The appropriate amount of B cells and SP2/0 cells were fused and selected in DMEM complete medium containing HAT. The hybridoma cells were cultured at 5% CO₂, 37°C for 5-7 days. Human or Cynomolgus monkey MASP-2 D4-6 recombinant protein (1 µg/mL) was coated and the hybridoma culture supernatant was detected by ELISA. A total of 778 monoclonal antibodies cross-recognized human or Cynomolgus monkey MASP-2. The supernatant was harvested and assayed by C4 lysis activity, of which 61 clones had significant MASP-2 inhibitory activity (as shown in FIG. 2).

Based on the results of hybridoma screening, RNAs of hybridoma monoclonal cell candidates were extracted by TRNzol lysis method, and then single-chain cDNAs were synthesized by reverse transcription, which was used as a template to amplify the variable region sequences of antibodies in the hybridoma monoclonal cells.

The variable region sequences of the MASP-2 antibodies are as follows:

**Table 1. The variable region sequences of MASP-2 murine antibodies**

| MAS P-2 muri ne antib ody | HCVR | | HCD R1 | HCD R2 | HCD R3 | LCVR | | LCD R1 | LCD R2 | LCD R3 |
|---|---|---|---|---|---|---|---|---|---|---|
| | Amin o acid seque nce | Nucle otide sequen ce | Amin o acid seque nce | Amin o acid seque nce | Amin o acid seque nce | Amin o acid seque nce | Nucle otide sequen ce | Amin o acid seque nce | Amin o acid seque nce | Amin oacid seque nce |
| 1B2 | 5 | 6 | 7 | 8 | 9 | 10 | 11 | 12 | 13 | 14 |
| 3B9 | 15 | 16 | 7 | 17 | 18 | 19 | 20 | 21 | 22 | 23 |
| 4F8 | 24 | 25 | 26 | 27 | 28 | 29 | 30 | 31 | 32 | 33 |
| 5J11 | 34 | 35 | 36 | 37 | 38 | 39 | 40 | 41 | 42 | 43 |
| 9P13 | 44 | 45 | 7 | 46 | 47 | 48 | 49 | 50 | 51 | 52 |
| 10H9 | 53 | 54 | 7 | 17 | 18 | 55 | 56 | 21 | 57 | 23 |
| 10L3 | 58 | 59 | 7 | 17 | 60 | 61 | 62 | 50 | 51 | 52 |
| 11F8 | 63 | 64 | 65 | 66 | 67 | 68 | 69 | 70 | 71 | 72 |
| 11G8 | 73 | 74 | 7 | 17 | 47 | 75 | 76 | 50 | 51 | 52 |
| 14K2 2 | 77 | 78 | 7 | 17 | 79 | 80 | 81 | 50 | 51 | 82 |
| 16A1 5 | 83 | 84 | 36 | 85 | 86 | 87 | 88 | 41 | 42 | 43 |
| 16A1 7 | 83 | 84 | 36 | 85 | 86 | 89 | 90 | 41 | 42 | 91 |
| 20J6 | 92 | 93 | 94 | 95 | 96 | 97 | 98 | 99 | 100 | 101 |
| 21E1 | 102 | 103 | 7 | 104 | 105 | 106 | 107 | 108 | 109 | 110 |
| 24G1 7 | 111 | 112 | 26 | 113 | 114 | 115 | 116 | 117 | 118 | 33 |
| 25L1 5 | 119 | 120 | 94 | 95 | 96 | 121 | 122 | 123 | 100 | 101 |
| 28O1 4 | 124 | 125 | 126 | 127 | 128 | 129 | 130 | 131 | 132 | 133 |

| | | | | | | | | | | |
|---|---|---|---|---|---|---|---|---|---|---|
| Clone: 1B2 | | | | | | | | | | |

The amino acid sequence of the heavy chain VH is shown in SEQ ID NO: 5, its encoding nucleic acid is shown in SEQ ID NO: 6, and its CDR1, CDR2 and CDR3 are shown in SEQ ID NO: 7, 8 and 9, respectively.

<-------------FR1----------->        CDR1    <----FR2----->                    CDR2
<--
DVQLQESGPGLVKPSQSLSLTCTVTGYSITSDYAWNWIRQFSGDKLE
WMGYISYSGSTSYNPSLKSRIS
------------FR3-------------->CDR3 <---FR4--->
ITRDTSKNQFFLHLSSVTTEDTATYFCARYWGDYWGHGTTLTVSS

### Nucleotide sequence

GATGTGCAGCTTCAGGAGTCGGGACCTGGCCTGGTGAAACCTTCTCAGTCTCT
GTCCCTCACCTGCACTGTCACTGGCTACTCAATCACCAGTGATTATGCCTGGAA
CTGGATCCGGCAGTTTTCAGGAGACAAACTGGAGTGGATGGGCTACATAAGCT
ACAGTGGTTCCACTAGCTATAACCCATCTCTCAAAAGTCGAATCTCTATCACTCG
AGACACATCCAAGAACCAGTTCTTCCTGCACTTGAGTTCTGTGACTACTGAGG
ACACAGCCACATATTTCTGTGCAAGATATTGGGGCGACTACTGGGGCCACGGCA
CCACTCTCACAGTCTCCTCA

The amino acid sequence of light chain VK is shown in SEQ ID NO: 10, its encoding nucleic acid is shown in SEQ ID NO: 11, and its CDR1, CDR2 and CDR3 are shown in SEQ ID NOs: 12, 13 and 14, respectively.

<----------FR1--------> CDR1 <-----FR2-----> CDR2 <-----------
DSVMTQSQKFMSTSVGDRVSVTCKASHNVGTNVAWFQQKPGQSPK
GLIYSASYRSSGVPDRFTGSGSGT
-----FR3----------> CDR3 <---FR4--->
DFTLTISNVQSEDLAEYFCLQYNSNPLTFGGGTKLEVK

### Nucleotide sequence

GACAGTGTGATGACCCAGTCTCAAAAATTCATGTCCACATCAGTAGGAGACAG
GGTCAGCGTCACCTGCAAGGCCAGTCACAATGTGGGTACTAATGTAGCCTGGTT
TCAACAGAAACCAGGACAATCTCCTAAAGGACTGATTTACTCGGCATCCTACCG
GTCCAGTGGAGTCCCTGATCGCTTCACAGGCAGTGGATCTGGGACAGACTTCA
CTCTCACCATCAGCAATGTGCAGTCTGAAGACTTGGCAGAGTATTTCTGTCTAC

AATATAACAGCAATCCTCTGACGTTCGGTGGAGGCACCAAGCTGGAGGTCAAG

### Clone: 3B9

The amino acid sequence of the heavy chain VH is shown in SEQ ID NO: 15, its encoding nucleic acid is shown in SEQ ID NO: 16, and its CDR1, CDR2 and CDR3 are shown in SEQ ID NOs: 7, 17 and 18, respectively.

<-------------FR1----------->        CDR1    <----FR2----->                    CDR2
<--
DVQLQESGPGLVRPSQSLSLTCTVTGYSITSDYAWNWIRQFPGNKLE
WMGYISYSGRTSYNPSLKSRLS
------------FR3------------->      CDR3    <---FR4--->
ITRDTSKNQFFLQLNSVSSADTATYYCAIISTAVDFDYWGQGTTLTVS
S

### Nucleotide sequence

GATGTACAACTTCAGGAGTCGGGACCTGGCCTGGTGAGACCTTCTCAGTCTCT
GTCCCTCACCTGCACTGTCACTGGCTACTCAATCACCAGTGATTATGCCTGGAA
CTGGATCCGGCAGTTTCCAGGAAACAAACTGGAGTGGATGGGCTACATAAGTT
ACAGTGGTCGCACTAGTTACAACCCCTCTCTCAAAAGTCGACTCTCTATCACTC
GAGACACATCCAAGAACCAGTTCTTCCTGCAGTTGAATTCTGTGAGTTCTGCG
GACACAGCCACGTATTACTGTGCAATTATTTCTACGGCAGTAGACTTTGACTACT
GGGGCCAAGGCACCACTCTCACAGTCTCCTCA

The amino acid sequence of light chain VK is shown in SEQ ID NO: 19, its encoding nucleic acid is shown in SEQ ID NO: 20, and its CDR1, CDR2 and CDR3 are shown in SEQ ID NOs: 21, 22 and 23, respectively.

<-----------FR1--------> CDR1 <-----FR2-----> CDR2 <-----------
QTVLTQSPAIMSASLGERVTMTCTASSSVSSSYLLWYQQKPGSSPKL
WIYSTSKLASGVPVRFSGSGSG
-----FR3-----------> CDR3 <---FR4--->
TSYSLTISSMEADDAATYYCLQYNRSPLTFGAGTKLELK

### Nucleotide sequence

CAAACTGTTCTCACCCAGTCTCCAGCAATCATGTCTGCATCTCTAGGGGAACGG
GTCACCATGACCTGCACTGCCAGCTCAAGTGTAAGTTCCAGTTACTTGCTCTGG
TACCAGCAGAAGCCAGGATCCTCCCCCAAACTCTGGATTTATAGCACATCCAAG
TTGGCTTCTGGAGTCCCAGTTCGCTTCAGTGGCAGTGGGTCTGGGACCTCTTAC
TCTCTCACAATCAGCAGCATGGAGGCTGACGATGCTGCCACTTATTACTGCCTC
CAGTATAATCGTTCCCCGCTCACGTTCGGTGCTGGGACCAAGCTGGAGCTGAA
A

### Clone: 4F8

The amino acid sequence of the heavy chain VH is shown in SEQ ID NO: 24, its encoding nucleic acid is shown in SEQ ID NO: 25, and its CDR1, CDR2 and CDR3 are shown in SEQ ID NOs: 26, 27 and 28, respectively.

<------------FR1----------->      CDR1    <----FR2----->                    CDR2
<--
EVQLQESGPGLVKPSQSLSLACTVTGYSITSDYAWHWIRQFPGNKLE
WMGFISYSGRTNYNPPLTSRIS
------------FR3-------------->    CDR3      <---FR4--->
ITRDTSKNQFFLQLNSVTTEDTATYYCARSYDYWFGYWGQGTLVTV
SA

### Nucleotide sequence

GAGGTCCAGCTGCAGGAGTCGGGACCTGGCCTGGTGAAACCTTCTCAGTCTCT
GTCCCTCGCCTGCACTGTCACTGGCTACTCAATCACCAGTGATTATGCCTGGCA
CTGGATCCGGCAGTTTCCAGGAAACAAACTGGAGTGGATGGGCTTCATAAGCT
ACAGTGGTAGAACTAACTACAACCCACCTCTCACAAGTCGAATCTCTATCACTC
GAGACACATCCAAGAACCAGTTCTTCCTGCAGTTGAATTCTGTGACTACTGAG
GACACAGCCACATATTACTGTGCAAGATCATATGATTACTGGTTTGGTTATTGGG
GCCAAGGGACTCTGGTCACTGTCTCTGCA

The amino acid sequence of light chain VK is shown in SEQ ID NO: 29, its encoding nucleic acid is shown in SEQ ID NO: 30, and its CDR1, CDR2 and CDR3 are shown in SEQ ID NOs: 31, 32 and 33, respectively.

<----------FR1--------> CDR1 <-----FR2-----> CDR2
<-------
NVLMTQTPLSLPVSLGDQASISCRSSQSLVHSNGNTYLEWYMQKPG
QSPKLLIYTVSRRFSGVPDRFSG
-----FR3-----------> CDR3 <---FR4--->
SGSGTDFTLKISRVEAEDLGVYYCFQGSHVPVTFGAGTKLELK

### Nucleotide sequence

AATGTTTTGATGACCCAAACTCCACTCTCCCTGCCTGTCAGTCTTGGAGATCAA
GCCTCCATCTCTTGCAGATCTAGTCAGAGTCTTGTACATAGTAATGGAAACACCT
ATTTAGAATGGTACATGCAGAAACCAGGCCAGTCTCCAAAGCTCCTGATCTACA
CAGTTTCCAGACGATTTTCTGGGGTCCCAGACAGGTTCAGTGGCAGTGGATCA
GGGACAGATTTCACACTCAAGATCAGCAGAGTGGAGGCTGAGGATCTGGGAGT
TTATTATTGCTTTCAAGGTTCACATGTTCCGGTCACGTTCGGTGCTGGGACCAA
GCTGGAGCTGAAA

### Clone: 5J11

The amino acid sequence of the heavy chain VH is shown in SEQ ID NO: 34, its encoding nucleic acid is shown in SEQ ID NO: 35, and its CDR1, CDR2 and CDR3 are shown in SEQ ID NOs: 36, 37 and 38, respectively.

<------------FR1----------->        CDR1    <----FR2----->                    CDR2
<--
DVQLQESGPGLVKPSQSLSLTCTVTGYSITSDYAWKWIRQFPGNKLE

WMGYISYSGSTSYNPSLKGRIS
------------FR3-------------> CDR3 <---FR4--->
ITRDTSKNQFFLHLQSVTIEDTATYYCAILGYFAMDYWGQGTPVTVS
S

### Nucleotide sequence

GATGTACAACTTCAGGAGTCGGGACCTGGCCTGGTGAAACCTTCTCAGTCTCT
GTCCCTCACCTGCACTGTCACTGGCTACTCAATCACCAGTGATTATGCCTGGAA
GTGGATCCGGCAGTTTCCAGGAAACAAACTGGAGTGGATGGGCTACATAAGTT
ACAGTGGCAGCACTAGCTACAACCCTTCTCTCAAAGGTCGAATCTCTATCACTC
GAGACACATCCAAGAACCAGTTTTTCCTGCACTTGCAATCTGTGACTATTGAGG
ACACAGCCACCTATTACTGTGCAATACTAGGTTACTTTGCTATGGACTACTGGGG
GCAAGGAACCCCAGTCACCGTCTCCTCA

The amino acid sequence of light chain VK is shown in SEQ ID NO: 39, its encoding nucleic acid is shown in SEQ ID NO: 40, and its CDR1, CDR2 and CDR3 are shown in SEQ ID NOs: 41, 42 and 43, respectively.

<-----------FR1--------> CDR1 <-----FR2-----> CDR2 <-----------
DIVMTQSQKFMSTSVGDRVSITCKASQNVRVDVAWYQQKSGQSPKS
LIYLASNRHTGVPDRFTGSGSGT
-----FR3----------> CDR3 <---FR4--->
DFTLTISNVQSEDLADYFCLQHWSYPLTFGGGTKLEIK

### Nucleotide sequence

GACATTGTGATGACCCAGTCTCAAAAATTCATGTCCACTTCAGTAGGAGACAGG
GTCAGCATCACCTGCAAGGCCAGTCAGAATGTTCGTGTTGACGTTGCCTGGTAT
CAACAGAAATCAGGGCAGTCTCCTAAATCACTGATTTACTTGGCATCCAACCGG
CACACTGGAGTCCCTGATCGCTTCACAGGCAGTGGATCTGGGACAGATTTCAC
TCTCACCATTAGCAATGTGCAATCTGAAGACCTGGCAGATTATTTCTGTCTACAA
CATTGGAGTTATCCTCTGACGTTCGGTGGAGGCACCAAGCTGGAAATCAAA

### Clone: 9P13

The amino acid sequence of the heavy chain VH is shown in SEQ ID NO: 44, its encoding nucleic acid is shown in SEQ ID NO: 45, and its CDR1, CDR2 and CDR3 are shown in SEQ ID NOs: 7, 46 and 47, respectively.

<-------------FR1----------->        CDR1    <----FR2----->                    CDR2
<--
DVQLQESGPGLVKPSQSLSLTCTVTGYSITSDYAWNWIRQFPGNTLE
WMGYISYSGRTSYNPSLIGRIS
------------FR3------------->    CDR3      <---FR4--->
ITRDTTKNQFFLQLNSVSTEDTATYYCAKLAGGHGMDYWGQGTSV
TVSS

### Nucleotide sequence

GATGTGCAGCTTCAGGAGTCGGGACCTGGCCTGGTGAAACCTTCTCAGTCTCT

GTCCCTCACCTGCACTGTCACTGGCTACTCAATCACCAGTGATTATGCCTGGAA
CTGGATCCGGCAGTTTCCAGGAAACACATTGGAGTGGATGGGCTATATAAGTTA
TAGTGGTAGGACTAGCTACAACCCATCTCTGATAGGTCGAATCTCAATCACTCG
AGACACAACCAAGAACCAGTTCTTTCTGCAGTTGAATTCTGTGTCTACTGAGG
ACACAGCCACATATTACTGTGCAAAATTGGCCGGAGGACATGGGATGGACTACT
GGGGTCAAGGAACCTCAGTCACCGTCTCCTCA

The amino acid sequence of light chain VK is shown in SEQ ID NO: 48, its encoding nucleic acid is shown in SEQ ID NO: 49, and its CDR1, CDR2 and CDR3 are shown in SEQ ID NOs: 50, 51 and 52, respectively.

<-----------FR1--------->        CDR1        <-----FR2----->    CDR2 <------------
NIVMTQSQQFMSTSVGDRVSVTCKASQNVDTNVAWYQQKPGQSPK
ALIYSASYRYSGVPDRFTGSGSGT
-----FR3----------->    CDR3    <---FR4--->
DFTLTISNVQSEDLAEYFCLQYNSYLTFGSGTKLELK

### Nucleotide sequence

AACATTGTGATGACCCAGTCTCAACAATTCATGTCCACATCAGTAGGAGACAGG
GTCAGCGTCACCTGCAAGGCCAGTCAGAATGTGGATACTAATGTAGCCTGGTAT
CAACAGAAACCAGGACAATCTCCTAAAGCACTGATTTACTCGGCATCCTACCGG
TACAGTGGAGTCCCTGATCGCTTCACAGGCAGTGGATCTGGGACAGATTTCACT
CTCACCATCAGCAATGTGCAGTCTGAAGACTTGGCAGAATATTTCTGTCTGCAA
TATAATAGTTACCTCACGTTCGGTTCTGGGACCAAGCTGGAGCTGAAA

### Clone: 10H9

The amino acid sequence of the heavy chain VH is shown in SEQ ID NO: 53, its encoding nucleic acid is shown in SEQ ID NO: 54, and its CDR1, CDR2 and CDR3 are shown in SEQ ID NOs: 7, 17 and 18, respectively.

<-------------FR1----------->        CDR1    <----FR2----->                    CDR2
<--
DVQLQESGPGLVRPSQSLSLTCTVTGYSITSDYAWNWIRQFPGNKLE
WMGYISYSGRTSYNPSLKSRLS
------------FR3------------->    CDR3      <---FR4--->
VTRDTSKNQFFLQLNSVNAADTATYYCAIISTAVDFDYWGQGTTLT
VSS

### Nucleotide sequence

GATGTACAACTTCAGGAGTCGGGACCTGGCCTGGTGAGACCTTCTCAGTCTCT
GTCCCTCACCTGCACTGTCACTGGCTACTCAATCACCAGTGATTATGCCTGGAA
CTGGATCCGGCAGTTTCCAGGAAACAAACTGGAGTGGATGGGCTACATAAGTT
ACAGTGGTCGCACTAGCTACAACCCCTCTCTCAAGAGTCGACTCTCTGTCACTC
GAGACACATCCAAGAACCAGTTCTTCCTGCAGTTGAATTCTGTGAATGCTGCG
GACACAGCCACGTATTACTGTGCAATTATTTCTACGGCAGTAGACTTTGACTACT

GGGGCCAAGGCACCACTCTCACAGTCTCCTCA

The amino acid sequence of light chain VK is shown in SEQ ID NO: 55, its encoding nucleic acid is shown in SEQ ID NO: 56, and its CDR1, CDR2 and CDR3 are shown in SEQ ID NOs: 21, 57 and 23, respectively.

<----------FR1-------->        CDR1        <-----FR2----->    CDR2 <-----------
QIVLTQSPAIMSASLGERVTMTCTASSSVSSSYLLWYQQKPGSSPKL
WIYSSSKLASGVPVRFSGSGSG
-----FR3----------->    CDR3        <---FR4-->
TSYSLTISSMEAEDAATYYCLQYNRSPLTFGAGTKLELK

### Nucleotide sequence

CAAATTGTTCTCACCCAGTCTCCAGCAATCATGTCTGCATCTCTAGGGGAACGG
GTCACCATGACCTGCACTGCCAGCTCAAGTGTAAGTTCCAGTTACTTGCTCTGG
TACCAGCAGAAGCCAGGATCCTCCCCCAAACTCTGGATTTATAGCTCATCCAAG
TTGGCTTCTGGAGTCCCAGTTCGCTTCAGTGGCAGTGGGTCTGGGACCTCTTAC
TCTCTCACAATCAGCAGCATGGAGGCTGAAGATGCTGCCACTTATTACTGCCTC
CAGTATAATCGTTCCCCGCTCACGTTCGGTGCTGGGACCAAGCTGGAGCTGAA
A

### Clone: 10L3

The amino acid sequence of the heavy chain VH is shown in SEQ ID NO: 58, its encoding nucleic acid is shown in SEQ ID NO: 59, and its CDR1, CDR2 and CDR3 are shown in SEQ ID NOs: 7, 17 and 60, respectively.

<-------------FR1----------->        CDR1    <----FR2----->                    CDR2
<--
DVQLQESGPGLVKPSQSLSLTCTVTGYSITSDYAWNWIRQFPGNKLE
WMGYISYSGRTSYNPSLKSRIS
------------FR3------------->      CDR3      <---FR4--->
ITRDTSKNQFFLQLNSVTTEDTATYYCAKLGGGHGMDYWGQGTSV
TVSS

### Nucleotide sequence

GATGTGCAGCTTCAGGAGTCGGGACCTGGCCTGGTGAAACCTTCTCAGTCTCT
GTCCCTCACCTGCACTGTCACTGGCTACTCAATCACCAGTGATTATGCCTGGAA
CTGGATCCGGCAGTTTCCAGGAAACAAATTGGAGTGGATGGGCTACATAAGTTA
TAGTGGTAGGACTAGCTACAACCCATCTCTGAAAAGTCGAATCTCAATCACTCG
AGACACATCCAAGAACCAGTTCTTCCTGCAGTTGAATTCTGTGACTACTGAGG
ACACAGCCACATATTACTGTGCAAAATTGGGCGGAGGACATGGGATGGACTACT
GGGGTCAAGGAACCTCAGTCACCGTCTCCTCA

The amino acid sequence of light chain VK is shown in SEQ ID NO: 61, its encoding nucleic acid is shown in SEQ ID NO: 62, and its CDR1, CDR2 and CDR3 are shown in SEQ ID NOs: 50, 51 and 52, respectively.

<----------FR1--------> CDR1 <-----FR2-----> CDR2 <------------
IVMTQSQKFMSTSVGDRVSVTCKASQNVDTNVAWYQQKPGQSPKA
LIYSASYRYSGVPDRFTGSGSGTD
----FR3----------> CDR3 <---FR4--->
FTLTISNVQSEDLAEYFCLQYNSYLTFGSGTKLELK

### Nucleotide sequence

ACATTGTGATGACCCAGTCTCAAAAATTCATGTCCACATCAGTAGGAGACAGGG
TCAGCGTCACCTGCAAGGCCAGTCAGAATGTGGATACTAATGTAGCCTGGTATC
AACAGAAACCAGGGCAATCTCCTAAAGCGCTGATTTACTCGGCATCCTACCGTT
ACAGTGGAGTCCCTGATCGCTTCACAGGCAGTGGATCTGGGACAGATTTCACT
CTCACCATCAGCAATGTGCAGTCTGAAGACTTGGCAGAGTATTTCTGTCTGCAA
TATAACAGCTACCTCACGTTCGGTTCTGGGACCAAGCTGGAGCTGAAA

### Clone: 11F8

The amino acid sequence of the heavy chain VH is shown in SEQ ID NO: 63, its encoding nucleic acid is shown in SEQ ID NO: 64, and its CDR1, CDR2 and CDR3 are shown in SEQ ID NOs: 65, 66 and 67, respectively.

<-------------FR1----------->        CDR1        <----FR2----->                    CDR2
<-
QVTLKESGPGILKPSQTLILTCSFSGFSLNTSGMGVGWIRQPSGKGLE
WLAHIWWDDDKDYNPSLKSQV
------------FR3-------------->        CDR3        <---FR4--->
TISKDTSTNQVFLKITSVDTADGATYYCVRDTTFLDAMDYWGQGTS
VTVSS

### Nucleotide sequence

CAAGTTACTCTAAAAGAGTCTGGCCCTGGGATATTGAAGCCCTCACAGACCCTC
ATTCTGACTTGTTCTTTCTCTGGGTTTTCACTGAACACTTCTGGTATGGGTGTAG
GCTGGATTCGTCAGCCTTCAGGGAAGGGTCTGGAGTGGCTGGCACACATTTGG
TGGGATGATGATAAGGACTATAACCCATCCCTGAAGAGCCAAGTCACAATCTCC
AAGGATACCTCCACAAACCAGGTGTTCCTCAAGATCACCAGTGTGGACACTGC
AGATGGTGCCACTTACTACTGTGTTCGAGATACTACGTTTTTAGATGCTATGGAC
TACTGGGGTCAAGGAACCTCAGTCACCGTCTCCTCA

The amino acid sequence of light chain VK is shown in SEQ ID NO: 68, its encoding nucleic acid is shown in SEQ ID NO: 69, and its CDR1, CDR2 and CDR3 are shown in SEQ ID NOs: 70, 71 and 72, respectively.

### Nucleotide sequence

GATGTTTTGATGACCCAAACTCCACTCTCCCTGCCTGTCAGTCTTGGAGATCAA
GCCTCCATCTCTTGCAGGTCTAGTCAGAGCATTGTACATCGTAATGGAAACACC
TTTTTAGAATGGTACCTGCAGAAACCAGGCCAGTCTCCAAAGCTCCTGATCTAC
AGGGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
GTTTATTACTGCTTTCAAGGTTCACATGTTCCATTCACGTTCGGCTCGGGGACA
AAGTTGGAAATAAAA

### Clone: 11G8

The amino acid sequence of the heavy chain VH is shown in SEQ ID NO: 73, its encoding nucleic acid is shown in SEQ ID NO: 74, and its CDR1, CDR2 and CDR3 are shown in SEQ ID NOs: 7, 17 and 47, respectively.

<-------------FR1----------->      CDR1    <----FR2----->                    CDR2
<--
DVQLQESGPGLVKPSQSLSLTCTVTGYSITSDYAWNWIRQLPGNKLE
WVGYISYSGRTSYNPSLKSRIS
------------FR3------------->      CDR3      <---FR4--->
ITRDTTKNQFFLQLNSVTTEDTATYYCAKLAGGHGMDYWGQGTSV
TVSS

### Nucleotide sequence

GATGTGCAGCTTCAGGAGTCGGGACCTGGCCTGGTGAAACCTTCTCAGTCTCT
GTCCCTCACCTGCACTGTCACTGGCTACTCAATCACCAGTGATTATGCCTGGAA
CTGGATCCGGCAGTTACCAGGAAACAAATTGGAGTGGGTGGGCTATATAAGTTA
TAGTGGTAGGACTAGCTACAACCCATCTCTGAAAAGTCGAATCTCAATCACTCG
AGACACAACCAAGAACCAGTTCTTCCTGCAGTTGAATTCTGTGACTACTGAGG
ACACAGCCACATATTACTGTGCAAAGTTGGCCGGGGGACATGGGATGGACTAC
TGGGGTCAAGGAACCTCAGTCACCGTCTCCTCA

The amino acid sequence of light chain VK is shown in SEQ ID NO: 75, its encoding nucleic acid is shown in SEQ ID NO: 76, and its CDR1, CDR2 and CDR3 are shown in SEQ ID NOs: 50, 51 and 52, respectively.

<-----------FR1--------> CDR1 <-----FR2-----> CDR2 <-------------
NIVMTQSQKFMSTSVGDRVSVTCKASQNVDTNVAWYQQKPGQSPK
ALIHSASYRYSGVPDRFTGSGSGT
----FR3-----------> CDR3 <---FR4--->
DFILTISNVQSEDLAEYFCLQYNSYLTFGSGTKLELK

### Nucleotide sequence

AACATTGTGATGACCCAGTCTCAAAAATTCATGTCCACATCAGTAGGAGACAGG
GTCAGCGTCACCTGCAAGGCCAGTCAGAATGTGGATACTAATGTAGCCTGGTAT
CAACAGAAACCAGGGCAATCTCCTAAAGCACTGATTCACTCGGCATCCTACCG
GTACAGTGGAGTCCCTGATCGCTTCACAGGCAGTGGATCTGGGACAGATTTCAT

TCTCACCATCAGTAATGTGCAGTCTGAAGACTTGGCAGAGTATTTCTGTCTGCA
ATATAACAGCTACCTCACGTTCGGTTCTGGGACCAAGCTGGAGCTGAAA

### Clone: 14K22

The amino acid sequence of the heavy chain VH is shown in SEQ ID NO: 77, its encoding nucleic acid is shown in SEQ ID NO: 78, and its CDR1, CDR2 and CDR3 are shown in SEQ ID NOs: 7, 17 and 79, respectively.

<------------FR1----------->        CDR1    <----FR2----->                        CDR2
<--
DVQLQESGPGLVKPSQSLSLTCTVTGYSITSDYAWNWIRQFPGNKLE
WMGYISYSGRTSYNPSLKSRIS
-----------FR3-------------->    CDR3 <---FR4--->
ITRDTSKNQFFLHLNSVTTEDTATYFCAVLGLYFDYWGQGTPLTVSS

### Nucleotide sequence

GATGTGCAGCTTCAGGAGTCGGGACCTGGCCTGGTGAAACCTTCTCAGTCTCT
GTCCCTCACCTGCACTGTCACTGGCTACTCAATCACCAGTGATTATGCCTGGAA
CTGGATCCGGCAGTTTCCAGGAAACAAACTGGAGTGGATGGGCTACATAAGCT
ACAGTGGTAGAACTAGCTACAACCCATCTCTCAAAAGTCGAATCTCTATCACTC
GAGACACATCCAAGAACCAGTTCTTCCTGCACTTGAATTCTGTGACTACTGAGG
ACACAGCCACATATTTCTGTGCAGTACTGGGACTCTACTTTGACTACTGGGGCC
AAGGCACCCCTCTCACAGTCTCCTCA

The amino acid sequence of light chain VK is shown in SEQ ID NO: 80, its encoding nucleic acid is shown in SEQ ID NO: 81, and its CDR1, CDR2 and CDR3 are shown in SEQ ID NOs: 50, 51 and 82, respectively.

<----------FR1--------> CDR1 <-----FR2-----> CDR2 <-----------
DIVMTQSQKFMSTSVGDRVSVTCKASQNVDTNVAWYQQKPGQSPK
ALIYSASYRYSGVPDRFTGSGSGT
----FR3-----------> CDR3 <---FR4--->
DFTLTISNVQSEDLAEYFCHQFNSNPLTFGAGTKLELK

### Nucleotide sequence

GACATTGTGATGACCCAGTCTCAAAAATTCATGTCCACATCAGTAGGAGACAGG
GTCAGCGTCACCTGCAAAGCCAGTCAGAATGTGGATACTAATGTAGCCTGGTAT
CAACAGAAACCAGGGCAATCTCCTAAAGCACTGATTTACTCGGCATCCTACCGG
TACAGTGGAGTCCCTGATCGCTTCACAGGCAGTGGATCTGGGACAGATTTCACT
CTCACCATCAGCAATGTGCAGTCTGAAGACTTGGCAGAATATTTCTGTCACCAA
TTTAACAGCAATCCTCTCACGTTCGGTGCTGGGACCAAGCTGGAGCTGAAA

### Clone: 16A15

The amino acid sequence of the heavy chain VH is shown in SEQ ID NO: 83, its encoding nucleic acid is shown in SEQ ID NO: 84, and its CDR1, CDR2 and CDR3 are shown in SEQ ID NOs: 36, 85 and 86, respectively.

<-------------FR1----------->        CDR1    <----FR2----->                    CDR2
<--
DVQLQESGPGLVKPSQSLSLTCTVTGYSITSDYAWKWIRQFPGNKLE
WMGYISYSGSTSYHPSLKGRIS
------------FR3------------->      CDR3 <---FR4--->
ITRDTSKNQFFLQLNSVTADDTATYYCAILGYYAMDYWGQGTSVTV
SS

### Nucleotide sequence

GATGTGCAGCTTCAGGAGTCGGGACCTGGCCTGGTGAAACCTTCTCAGTCTCT
GTCCCTCACCTGCACTGTCACTGGCTACTCAATCACCAGTGATTATGCCTGGAA
ATGGATCCGGCAGTTTCCAGGAAACAAACTGGAATGGATGGGCTACATAAGCT
ACAGTGGTTCCACTAGCTATCACCCATCTCTCAAAGGTCGAATCTCCATCACTC
GAGACACATCCAAGAACCAGTTCTTCCTGCAGTTGAATTCTGTGACTGCTGAC
GACACAGCCACATATTACTGTGCAATATTAGGTTACTATGCTATGGACTACTGGG
GTCAAGGAACCTCAGTCACCGTCTCCTCA

The amino acid sequence of light chain VK is shown in SEQ ID NO: 87, its encoding nucleic acid is shown in SEQ ID NO: 88, and its CDR1, CDR2 and CDR3 are shown in SEQ ID NOs: 41, 42 and 43, respectively.

<-----------FR1--------> CDR1 <-----FR2-----> CDR2 <------------
DIVMTQSQKFMSTSVGDRVSITCKASQNVRVDVAWYQQKSGQSPRA
LIYLASNRHTGVPDRFTGSGSGT
----FR3-----------> CDR3 <---FR4--->
DFTLTITNVQSEDLADYFCLQHWSYPLTFGGGTKLEIK

### Nucleotide sequence

GACATTGTGATGACCCAGTCTCAAAAATTCATGTCCACATCAGTAGGAGACAGG
GTCAGTATCACCTGCAAGGCCAGTCAGAATGTTCGTGTTGACGTAGCCTGGTAT
CAACAGAAATCAGGGCAGTCTCCTAGAGCACTGATTTACTTGGCATCCAACCG
GCACACTGGAGTCCCTGATCGCTTCACAGGCAGTGGATCTGGGACAGATTTCA
CTCTCACCATTACCAATGTGCAATCTGAGGACCTGGCAGATTATTTCTGTCTACA
ACATTGGAGTTATCCTCTGACGTTCGGTGGAGGCACCAAGCTGGAAATCAAA

### Clone: 16A17

The amino acid sequence of the heavy chain VH is shown in SEQ ID NO: 83, its encoding nucleic acid is shown in SEQ ID NO: 84, and its CDR1, CDR2 and CDR3 are shown in SEQ ID NOs: 36, 85 and 86, respectively.

<-------------FR1----------->      CDR1      <----FR2----->                    CDR2
<--
DVQLQESGPGLVKPSQSLSLTCTVTGYSITSDYAWKWIRQFPGNKLE
WMGYISYSGSTSYHPSLKGRIS
------------FR3------------->    CDR3     <---FR4--->
ITRDTSKNQFFLQLNSVTADDTATYYCAILGYYAMDYWGQGTSVTV
SS

### Nucleotide sequence

GATGTGCAGCTTCAGGAGTCGGGACCTGGCCTGGTGAAACCTTCTCAGTCTCT
GTCCCTCACCTGCACTGTCACTGGCTACTCAATCACCAGTGATTATGCCTGGAA
ATGGATCCGGCAGTTTCCAGGAAACAAACTGGAATGGATGGGCTACATAAGCT
ACAGTGGTTCCACTAGCTATCACCCATCTCTCAAAGGTCGAATCTCCATCACTC
GAGACACATCCAAGAACCAGTTCTTCCTGCAGTTGAATTCTGTGACTGCTGAC
GACACAGCCACATATTACTGTGCAATATTAGGTTACTATGCTATGGACTACTGGG
GTCAAGGAACCTCAGTCACCGTCTCCTCA

The amino acid sequence of light chain VK is shown in SEQ ID NO: 89, its encoding nucleic acid is shown in SEQ ID NO: 90, and its CDR1, CDR2 and CDR3 are shown in SEQ ID NOs: 41, 42 and 91, respectively.

<-----------FR1--------> CDR1 <-----FR2-----> CDR2 <------------
DIVMTQSQKFMSTSVGDRVSITCKASQNVRVDVAWYQQKSGQSPRA
LIYLASNRHTGVPDRFTGSGSGT
----FR3-----------> CDR3 <---FR4--->
DFTLTITNVQSEDLADYFCPQHWSYPLTFGGGTKLEIK

### Nucleotide sequence

GACATTGTGATGACCCAGTCTCAAAAATTCATGTCCACATCAGTAGGAGACAGG
GTCAGTATCACCTGCAAGGCCAGTCAGAATGTTCGTGTTGACGTAGCCTGGTAT
CAACAGAAATCAGGGCAGTCTCCTAGAGCACTGATTTACTTGGCATCCAACCG
GCACACTGGAGTCCCTGATCGCTTCACAGGCAGTGGATCTGGGACAGATTTCA
CTCTCACCATTACCAATGTGCAATCTGAGGACCTGGCAGATTATTTCTGTCCACA
ACATTGGAGTTATCCTCTGACGTTCGGTGGAGGCACCAAGCTGGAAATCAAA

### Clone: 20J6

The amino acid sequence of the heavy chain VH is shown in SEQ ID NO: 92, its encoding nucleic acid is shown in SEQ ID NO: 93, and its CDR1, CDR2 and CDR3 are shown in SEQ ID NOs: 94, 95 and 96, respectively.

<-------------FR1-----------> CDR1 <----FR2-----> CDR2 <--
KVKLVESGGDLVKPGGSLKISCTASGFTFSNYAMSWIRQTPDKRLEW
VATVSSGGPYTYYPDSVKGRFT
------------FR3-------------> CDR3 <---FR4--->
ISRDNAENTLYLQMSSLRSDDTAIYYCARQIYSDYDVTYWGQGTLVT
VSA

### Nucleotide sequence

AAAGTGAAGCTGGTGGAGTCTGGGGGAGACTTAGTGAAGCCTGGAGGGTCCC
TGAAAATCTCCTGTACAGCCTCTGGATTCACTTTCAGTAACTATGCCATGTCTTG
GATTCGCCAGACTCCGGACAAGAGGCTGGAGTGGGTCGCAACCGTTAGTAGTG
GTGGTCCTTACACCTACTATCCAGACAGTGTGAAGGGGCGATTCACCATCTCCA
GAGACAATGCCGAAAACACCCTGTACCTGCAAATGAGCAGTCTGAGGTCTGAT

GACACGGCCATATATTACTGTGCAAGGCAGATCTACTCTGATTACGACGTCACTT
ACTGGGGCCAAGGGACTCTGGTCACTGTCTCTGCA

The amino acid sequence of light chain VK is shown in SEQ ID NO: 97, its encoding nucleic acid is shown in SEQ ID NO: 98, and its CDR1, CDR2 and CDR3 are shown in SEQ ID NOs: 99, 100 and 101, respectively.

<----------FR1--------> CDR1 <-----FR2-----> CDR2 <------------
QIVLSQSPAILSASPGETVTMTCRASSSVSFMYWLQQKPGSSPQPWIY
ATSNLASGVPARFSGSGSGTS
----FR3----------> CDR3 <---FR4--->
YSLTITRVEAEDAATYYCQQWSFRPWTFGGGTKLEIK

### Nucleotide sequence

CAAATTGTTCTCTCCCAGTCTCCAGCAATCCTGTCTGCATCTCCAGGGGAGACG
GTCACAATGACTTGCAGGGCCAGTTCAAGTGTAAGTTTCATGTACTGGCTCCAG
CAGAAGCCAGGTTCCTCCCCCCAACCCTGGATTTATGCCACATCCAACCTGGCT
TCTGGAGTCCCTGCTCGCTTCAGTGGCAGTGGGTCTGGGACCTCTTACTCCCTC
ACAATCACCAGAGTGGAGGCTGAAGATGCTGCCACTTATTACTGTCAACAGTG
GAGTTTTAGACCGTGGACGTTCGGTGGAGGCACCAAGCTGGAAATCAAA

### Clone: 21E1

The amino acid sequence of the heavy chain VH is shown in SEQ ID NO: 102, its encoding nucleic acid is shown in SEQ ID NO: 103, and its CDR1, CDR2 and CDR3 are shown in SEQ ID NOs: 7, 104 and 105, respectively.

<-------------FR1-----------> CDR1 <----FR2-----> CDR2
<--
DVQLQESGPGLVKPSQSLSLTCTVTGFSITSDYAWNWIRQFPGNKLE
WMGYISYSGRTNYNPSLKSRFS
------------FR3-------------> CDR3 <---FR4--->
ITRDTSKNQFFLQLNSVTTEDTATYYCARSYDYWFPYWGQGTLVTV
SA

### Nucleotide sequence

GATGTGCAGCTTCAGGAGTCGGGACCTGGCCTGGTGAAACCTTCTCAGTCTCT
GTCCCTCACCTGCACTGTCACTGGCTTCTCAATCACCAGTGATTATGCCTGGAA
CTGGATCCGGCAGTTTCCAGGAAACAAACTGGAGTGGATGGGCTACATAAGCT
ACAGTGGTAGAACTAACTACAACCCATCTCTCAAAAGTCGATTCTCTATCACTC
GAGACACATCCAAGAACCAGTTCTTCCTGCAGTTGAATTCTGTGACTACTGAG
GACACAGCCACATATTACTGTGCAAGATCATATGATTACTGGTTTCCTTACTGGG
GCCAAGGGACTCTGGTCACTGTCTCTGCA

The amino acid sequence of light chain VK is shown in SEQ ID NO: 106, its encoding nucleic acid is shown in SEQ ID NO: 107, and its CDR1, CDR2 and CDR3 are shown in SEQ ID NOs: 108, 109 and 110, respectively.

<----------FR1-------->                    CDR1              <-----FR2----->     CDR2
<-------
DVLMTQTPLSLPVSLGDQASISCRSSQSIVHSNGNTFLEWYLQKPGQ
SPKLLIYKVSNRFSGVPDRFSG
----FR3----------------->    CDR3    <---FR4--->
SGSGTDFTLKISRVEAEDLGVYYCFQGSHIPVTFGAGTKLELK

### Nucleotide sequence

GATGTTTTGATGACCCAAACTCCACTCTCCCTGCCTGTCAGTCTTGGAGATCAA
GCCTCCATCTCTTGCAGATCTAGTCAGAGCATTGTACATAGTAATGGAAACACC
TTTTTGGAATGGTACCTGCAGAAACCAGGCCAGTCTCCAAAGCTCCTGATCTAC
AAAGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
GTTTATTACTGCTTTCAAGGTTCACATATTCCGGTCACGTTCGGTGCTGGGACCA
AGCTGGAGCTGAAA

### Clone: 24G17

The amino acid sequence of the heavy chain VH is shown in SEQ ID NO: 111, its encoding nucleic acid is shown in SEQ ID NO: 112, and its CDR1, CDR2 and CDR3 are shown in SEQ ID NOs: 26, 113 and 114, respectively.

<-------------FR1----------->    CDR1       <----FR2----->                    CDR2
<--
EVQLQESGPGLVKPSQSLSLTCTVTGFSITSDYAWHWIRQFPGNKLE
WMGFINYSGRTNYNPSLTSRIS
------------FR3------------->    CDR3    <---FR4--->
IIRDTSKNQFFLQLNSVTTEDTATYYCARSYDFWFGYWGQGTLVTVS
A

### Nucleotide sequence

GAGGTCCAGCTGCAGGAGTCGGGACCTGGCCTGGTGAAACCTTCTCAGTCTCT
GTCCCTCACCTGCACTGTCACTGGCTTCTCAATCACCAGTGATTATGCCTGGCA
CTGGATCCGGCAGTTTCCAGGAAACAAACTGGAGTGGATGGGCTTCATAAACT
ACAGTGGTAGAACTAACTACAACCCATCTCTCACAAGTCGAATCTCTATCATTC
GGGACACATCCAAGAACCAGTTCTTCCTGCAGTTGAATTCTGTGACTACTGAG
GACACAGCCACATATTACTGTGCAAGATCATATGATTTCTGGTTTGGTTATTGGG
GCCAAGGGACTCTGGTCACTGTCTCTGCA

The amino acid sequence of light chain VK is shown in SEQ ID NO: 115, its encoding nucleic acid is shown in SEQ ID NO: 116, and its CDR1, CDR2 and CDR3 are shown in SEQ ID NOs: 117, 118 and 33, respectively.

<-----------FR1--------> CDR1 <-----FR2-----> CDR2
<-------
NVLMTQSPLSLPVSLGDQASISCRSSQSIVHSNGNTYLEWYMQKPGQ
SPKLLIYTVSKRFSGVPDRFSG
----FR3-----------------> CDR3 <---FR4--->

SGSGTDFTLKISRVEAEDLGVYYCFQGSHVPVTFGAGTKLELK

### Nucleotide sequence

AATGTTTTGATGACCCAAAGTCCACTCTCCCTGCCTGTCAGTCTTGGAGATCAA
GCCTCCATCTCTTGCAGATCTAGTCAGAGTATTGTACATAGTAATGGAAACACCT
ATTTAGAATGGTATATGCAGAAACCAGGCCAGTCTCCAAAGCTCCTGATCTACA
CAGTTTCCAAACGATTTTCTGGGGTCCCAGACAGGTTCAGTGGCAGTGGATCA
GGGACAGATTTCACACTCAAGATCAGCAGAGTGGAGGCTGAGGATCTGGGAGT
TTATTATTGCTTTCAAGGTTCACATGTTCCGGTCACGTTCGGTGCTGGGACCAA
GCTGGAGCTGAAA

### Clone: 25L15

The amino acid sequence of the heavy chain VH is shown in SEQ ID NO: 119, its encoding nucleic acid is shown in SEQ ID NO: 120, and its CDR1, CDR2 and CDR3 are shown in SEQ ID NOs: 94, 95 and 96, respectively.

<-------------FR1----------->    CDR1      <----FR2----->                    CDR2
<--
KVKLVESGGDLVKPGGSLKISCAASGFTFSNYAMSWIRQTPDKRLEW
VATVSSGGPYTYYPDSVKGRFT
------------FR3------------->    CDR3      <---FR4--->
ISRDNAENTLYLQMSSLRSDDTAIYYCARQIYSDYDVTYWGQGTLVT
VSA

### Nucleotide sequence

AAAGTGAAGCTGGTGGAGTCTGGGGGAGACTTAGTGAAGCCTGGAGGGTCCC
TGAAAATCTCCTGTGCAGCCTCTGGATTCACTTTCAGTAACTATGCCATGTCTTG
GATTCGCCAGACTCCGGACAAGAGGCTGGAGTGGGTCGCAACCGTTAGTAGTG
GTGGTCCTTACACCTACTATCCAGACAGTGTGAAGGGGCGATTCACCATCTCCA
GAGACAATGCCGAAAACACCCTGTACCTGCAAATGAGCAGTCTGAGGTCTGAT
GACACGGCCATTTATTACTGTGCAAGGCAGATCTACTCTGATTACGACGTCACTT
ACTGGGGCCAAGGGACTCTGGTCACTGTCTCTGCA

The amino acid sequence of light chain VK is shown in SEQ ID NO: 121, its encoding nucleic acid is shown in SEQ ID NO: 122, and its CDR1, CDR2 and CDR3 are shown in SEQ ID NOs: 123, 100 and 101, respectively.

<-----------FR1--------> CDR1 <-----FR2-----> CDR2 <-------------
QIVLSQSPAILSASPGETVTMTCRVSSSVSYMYWLQQKPGSSPKPWIY
ATSNLASGVPARFSGSGSGTS
----FR3----------> CDR3 <---FR4--->
YSLTITRVEAEDAATYYCQQWSFRPWTFGGGTKLEIK

### Nucleotide sequence

CAAATTGTTCTCTCCCAGTCTCCAGCAATCCTGTCTGCATCTCCAGGGGAGACG
GTCACAATGACTTGCAGGGTCAGTTCAAGTGTAAGTTACATGTACTGGCTCCAA

CAGAAGCCAGGATCCTCCCCCAAACCCTGGATTTATGCCACATCCAACCTGGCT
TCTGGAGTCCCTGCTCGCTTCAGTGGCAGTGGGTCTGGGACCTCTTACTCCCTC
ACAATCACCAGAGTGGAGGCTGAAGATGCTGCCACTTATTACTGTCAGCAGTG
GAGTTTTAGACCGTGGACGTTCGGTGGAGGCACCAAGCTGGAAATCAAA

### Clone: 28014

The amino acid sequence of the heavy chain VH is shown in SEQ ID NO: 124, its encoding nucleic acid is shown in SEQ ID NO: 125, and its CDR1, CDR2 and CDR3 are shown in SEQ ID NOs: 126, 127 and 128, respectively.

<-------------FR1----------> CDR1 <----FR2---->          CDR2          <--
QVQLQQSGTELARPGASVRMSCKASGYTFRSHTMHWVKQRPGQGLEWIGYINPSTGYINYNQKFKDKAT
-----------FR3------------>      CDR3       <---FR4--->
LTADKSSSTAYMQLSSLTSEDSAVYYCARSNYSYYYGMDYWGQGTSVTVSS

### Nucleotide sequence

CAGGTCCAGCTGCAGCAGTCTGGGACTGAACTGGCAAGACCTGGGGCCTCAG
TGAGGATGTCCTGCAAGGCTTCTGGCTACACCTTTAGGAGTCACACGATGCACT
GGGTAAAACAGAGGCCTGGACAGGGTCTGGAATGGATTGGATACATTAATCCTA
GCACTGGTTATATTAATTACAATCAGAAGTTCAAGGACAAGGCCACATTGACTG
CAGACAAATCCTCCAGCACAGCCTACATGCAACTGAGCAGCCTGACATCTGAG
GACTCTGCAGTCTATTACTGTGCAAGAAGTAACTACTCTTATTACTATGGTATGG
ACTACTGGGGTCAAGGAACCTCAGTCACCGTCTCCTCA

The amino acid sequence of light chain VK is shown in SEQ ID NO: 129, its encoding nucleic acid is shown in SEQ ID NO: 130, and its CDR1, CDR2 and CDR3 are shown in SEQ ID NOs: 131, 132 and 133, respectively.

<-----------FR1--------> CDR1 <-----FR2-----> CDR2 <-----------
DIVMTQSHKFMSTSVGDRVTITCKASQDVNTAVAWYQQKPGQSPKLLIYSASYR
YTGVPDRFTGSGSGT
----FR3-----------> CDR3 <---FR4--->
DFTFTISSLQAEDLAVYYCQQHYNIPLTFGAGTKLELK

### Nucleotide sequence

GACATTGTGATGACCCAGTCTCACAAATTCATGTCCACATCAGTGGGAGACAG
GGTCACCATCACCTGCAAGGCCAGTCAGGATGTAAATACTGCTGTAGCCTGGTA
TCAACAGAAACCAGGACAATCTCCTAAACTACTGATTTACTCGGCATCCTACCG
GTACACTGGGGTCCCTGATCGCTTCACTGGCAGTGGATCTGGGACGGATTTCAC
TTTCACCATCAGCAGTTTACAGGCTGAAGACCTGGCAGTTTATTACTGTCAGCA
ACATTATAATATTCCGCTCACGTTCGGTGCTGGGACCAAGCTGGAGCTGAAA

The light chain variable region of the antibody described above was cloned into the vector containing the human light chain constant region, and the heavy chain variable region was cloned into the vector containing the human heavy chain constant region. The plasmid was co-transfected into CHO-S cells. The entire IgG antibody was expressed and secreted into the culture medium. After expression for 7 days at 37°C, 5% CO₂ and 125 rpm, the supernatant was collected and purified by Protein A chromatography to obtain chimeric IgG. The concentration of IgG was determined by spectrophotometry. The control antibody 17D20_3521N11 (OMS646) was synthesized according to the literature US20120282263.

Each chimeric antibody was tested for inhibitory activity against the cleaved C4 response as described in Example 1, and the OD450 nm value was read. After collecting data, the results were calculated using GraphPad Prism 5 software. The results are shown in FIG. 3, in which the chimeric antibodies 10L3, 3B9, 16A17, 4F18, 25L15, 28014 and 1B2 showed significantly greater inhibitory activity against MASP-2 than the control antibody in the C4 cleavage activity assay.

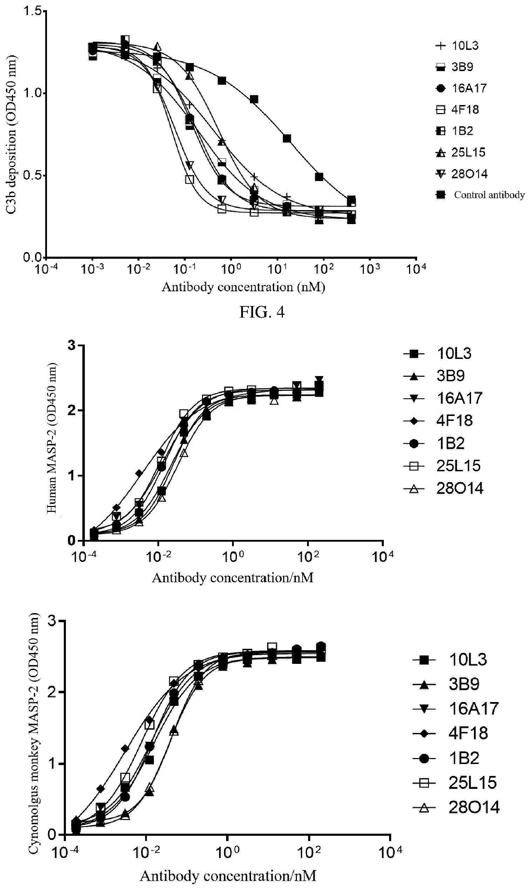

The selected dominant chimeric antibodies were tested for inhibition of C3 convertase activity as described in Example 1. After collecting data, the results were calculated using GraphPad Prism 5 software. The results are shown in FIG. 4, in which the dominant chimeric antibodies 10L3, 3B9, 16A17, 4F18, 25L15, 28014 and 1B2 showed significantly greater inhibitory activity than the control antibody in the C3 convertase activity assay.

### Affinity assessment

Human MASP-2 D4-6 recombinant protein was coated at 1 µg/mL in a 96-well ELISA plate and incubated overnight at 4°C. After blocking with 2% skim milk at room temperature for 1 hour, gradient-diluted MASP-2 antibodies, i.e. chimeric antibodies 10L3, 3B9, 16A17, 4F18, 25L15, 28014 and 1B2 (initial concentration of 30 µg/ml, 1:4 gradient dilution), were added and incubated at room temperature for 60 min. Goat anti-human Fc-HRP diluted 1:4000 was added and incubated at room temperature for 60 min. The color was developed with TMB for 10 min, then the reaction was stopped by adding concentrated sulfuric acid. The OD450 nm was determined, and the results were calculated using Graph Pad Prism 5 software.

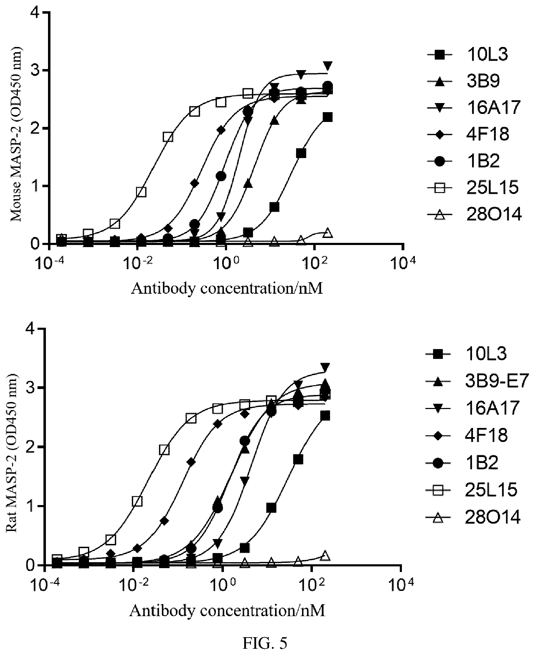

The results are shown in FIG. 5, in which the chimeric antibodies bind to human, Cynomolgus monkey, rat and mouse MASP-2 (Table 2).

**Table 2. Binding of MASP-2 antibodies to each species protein.**

| EC50 (nM) | Human MASP-2 | Cynomolgus monkey MASP-2 | Rat MASP-2 | Mouse MASP-2 |
|---|---|---|---|---|
| 1B2 | 0.015 | 0.014 | 1.439 | 0.947 |
| 3B9 | 0.025 | 0.038 | 1.624 | 4.393 |
| 4F18 | 0.004 | 0.003 | 0.127 | 0.287 |
| 10L3 | 0.022 | 0.016 | 30.19 | 30.90 |
| 16A17 | 0.014 | 0.014 | 4.296 | 1.973 |
| 25L15 | 0.012 | 0.008 | 0.021 | 0.023 |
| 28014 | 0.033 | 0.043 | - | - |

Surface plasmon resonance technology (SPR/Biacore T200) was used to capture 0.1 µg/ml of the antibody on the surface of the Protein A chip, with a capture time of 40 seconds and a flow rate of 10 µl/min. The chip was washed with buffer until the baseline leveled off, then gradient-diluted antigen was flowed through the chip at the flow rate of 30 µl/min, with a binding time of 120 sec and a dissociation time of 600 sec. The equilibrium dissociation constant (KD) was obtained by fitting in Langmuir 1:1 kinetics mode. The affinity of the candidate antibodies with each species of MASP-2 antigen is shown in Table 3.

**Table 3. The affinity of MASP-2 antibodies with each species protein.**

| KD (nM) | Human MASP-2 | Cynomolgus monkey MASP-2 | Rat MASP-2 | Mouse MASP-2 |
|---|---|---|---|---|
| 1B2 | 0.002 | 0.078 | Weak | 632 |
| 3B9 | 0.067 | 0.051 | 1043 | 476 |
| 4F18 | 0.108 | 24.76 | 2471 | 218 |
| 10L3 | 0.070 | 1.290 | Weak | 214 |
| 16A17 | 0.008 | 0.063 | Weak | 487 |
| 25L15 | 0.120 | 0.920 | 167 | 499 |
| 28O14 | 0.090 | 0.010 | 162 | 767 |
| Control antibody | 21.1 | 8.750 | 88.5 | 225 |

### Example 4. Construction and screening of phage antibody library

Total RNA was extracted from the partially immunized mouse B cells obtained in Example 3, and reverse transcribed using light and heavy chain specific primers, respectively, to generate an antibody cDNA library. The variable region fragments of antibody were amplified using variable region primers, and cloned into phage plasmid by enzyme digestion. The phage display library of antibody Fab based on M13 phage was constructed, with the capacity of 1×10¹⁰.

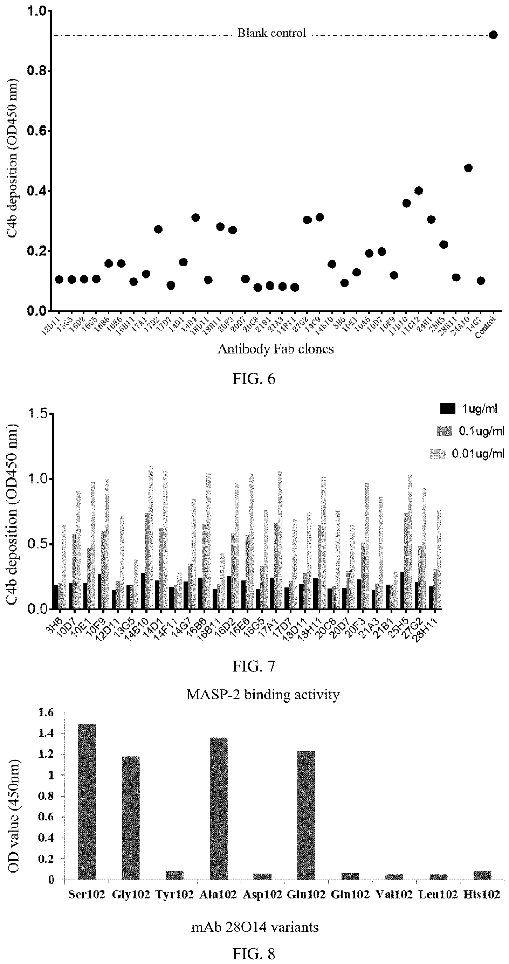

Conventional phage panning was used for library screening. MASP-2 D4-6 recombinant protein was coated onto an Immuno tube, and about 1×10¹² phages were added per screening reaction for incubation and panning. After 2 rounds of panning, single clones were picked and inoculated into a 96-well U-shaped plate for culture and inducible expression. The culture supernatant was taken for ELISA detection of MASP-2 recombinant protein. A total of 494 monoclonal Fab antibodies cross-recognizing human, Cynomolgus monkey, rat and mouse MASP-2 were obtained, of which 34 clones can inhibit C4b deposition catalyzed by MASP-2 (FIG. 6).

After re-inducing the expression of the Fab clone with the inhibitory activity described above, the Fab antibody was purified and the inhibitory activity of the MASP-2 antibody was determined by C4 cleavage activity assay. The results are shown in FIG. 7, in which the monoclonal antibodies 3H6, 14F11, 16B11, 20C8, 21A3 and 21B1 showed the most significant inhibitory activity against C4b deposition catalyzed by MASP-2 at concentrations greater than 0.01 µg/ml.

The surface plasmon resonance (SPR/Biacore T200) technique was used to capture the anti-Flag antibody with Protein A chip. 10 µg/ml purified Fab antibody containing Flag tag was captured on the chip surface, with a capture time of 40 sec and a flow rate of 10 µl/min. The chip was washed with buffer until the baseline leveled off, then gradient-diluted MASP-2 antigens were injected as the analytes successively to determine the affinity between Fab and different species antigens. The data were fitted by Biacore T200 Evaluation software 3.1 software in Langmuir 1:1 kinetics mode. Table 4 shows the affinity of mAbs 16B11 and 14F11 with each species of MASP-2 antigens.

**Table 4. The affinity of MASP-2 antibodies with the antigens.**

| MASP-2 | 16B11 | | | 14F11 | | |
|---|---|---|---|---|---|---|
| | ka (1/Ms) | kd (1/s) | KD (M) | ka (1/Ms) | kd (1/s) | KD (M) |
| Human | 2.15E+06 | 8.75E-05 | 4.07E-11 | 9.48E+05 | 1.63E-04 | 1.72E-10 |
| Cynomolgus | 1.66E+06 | 1.81E-04 | 1.09E-10 | 2.46E+10 | 1.14E+02 | 4.66E-09 |
| Mouse | 4.61E+06 | 8.11E-03 | 1.76E-09 | 6.44E+07 | 1.16E+00 | 1.80E-08 |
| Rat | 1.84E+06 | 9.31E-03 | 5.06E-09 | 3.34E+06 | 1.82E-03 | 5.46E-10 |

The variable region sequences of the Fab antibodies were obtained by Sanger sequencing as follows:

**Table 5. The variable region sequences of MASP-2 murine antibodies**

| MAS P-2 muri ne antib ody | HCVR | | HCD R1 | HCD R2 | HCD R3 | LCVR | | LCD R1 | LCD R2 | LCD R3 |
|---|---|---|---|---|---|---|---|---|---|---|
| | Amin o acid seque nce | Nucle otide sequen ce | Amin o acid seque nce | Amin o acid seque nce | Amin o acid seque nce | Amin o acid seque nce | Nucle otide sequen ce | Amin o acid seque nce | Amin o acid seque nce | Amin o acid seque nce |
| 3H6 | 134 | 135 | 136 | 137 | 138 | 129 | 130 | 131 | 132 | 133 |
| 16B11 | 139 | 140 | 7 | 17 | 141 | 142 | 143 | 144 | 145 | 146 |
| 14F11 | 147 | 148 | 149 | 150 | 151 | 152 | 153 | 154 | 155 | 156 |
| 20C8 | 157 | 158 | 7 | 17 | 9 | 159 | 160 | 161 | 162 | 14 |
| 21A3 | 163 | 164 | 7 | 165 | 9 | 166 | 167 | 144 | 168 | 14 |
| 21B1 | 169 | 170 | 7 | 17 | 9 | 171 | 172 | 144 | 173 | 174 |

### Clone: 3H6

The amino acid sequence of the heavy chain VH is shown in SEQ ID NO: 134, its encoding nucleic acid is shown in SEQ ID NO: 135, and its CDR1, CDR2 and CDR3 are shown in SEQ ID NOs: 136, 137 and 138, respectively.

<-------------FR1-----------> CDR1 <----FR2-----> CDR2 <--
QVQLQQSGTELARPGASVKMSCKASGYTFATYSIHWVKQRPGQGLEWIGYINPS
SGYTNYNQKFRDKAT
------------FR3------------> CDR3 <---FR4--->
LTADRSSNTAYMQLSSLTSEDSAVYYCARVYYTFYHAMDYWGQGTSVTVSS

### Nucleotide sequence

CAGGTCCAGCTGCAGCAGTCTGGGACTGAACTGGCAAGACCTGGGGCCTCAG
TGAAGATGTCCTGCAAGGCTTCTGGCTACACCTTTGCTACCTACTCGATACACT
GGGTAAAACAGAGGCCTGGGCAGGGTCTGGAATGGATTGGATACATTAATCCTA
GCAGTGGTTATACTAATTACAATCAGAAGTTCAGGGACAAGGCCACATTGACTG
CAGACAGATCCTCCAACACAGCCTACATGCAACTGAGCAGCCTGACATCTGAG
GACTCTGCAGTCTATTACTGTGCAAGAGTCTACTATACTTTTTACCATGCTATGG
ACTACTGGGGTCAAGGAACCTCAGTCACCGTCTCCTCA

The amino acid sequence of light chain VK is shown in SEQ ID NO: 129, its encoding nucleic acid is shown in SEQ ID NO: 130, and its CDR1, CDR2 and CDR3 are shown in SEQ ID NOs: 131, 132 and 133, respectively.

<----------FR1--------> CDR1 <-----FR2-----> CDR2 <-----------
DIVMTQSHKFMSTSVGDRVTITCKASQDVNTAVAWYQQKPGQSPKLLIYSASYR
YTGVPDRFTGSGSGT
----FR3----------> CDR3 <---FR4--->
DFTFTISSLQAEDLAVYYCQQHYNIPLTFGAGTKLELK

### Nucleotide sequence

GACATTGTGATGACCCAGTCTCACAAATTCATGTCCACATCAGTGGGAGACAG
GGTCACCATCACCTGCAAGGCCAGTCAGGATGTAAATACTGCTGTAGCCTGGTA
TCAACAGAAACCAGGACAATCTCCTAAACTACTGATTTACTCGGCATCCTACCG
GTACACTGGGGTCCCTGATCGCTTCACTGGCAGTGGATCTGGGACGGATTTCAC
TTTCACCATCAGCAGTTTACAGGCTGAAGACCTGGCAGTTTATTACTGTCAGCA
ACATTATAATATTCCGCTCACGTTCGGTGCTGGGACCAAGCTGGAGCTGAAA

### Clone: 16B11

The amino acid sequence of the heavy chain VH is shown in SEQ ID NO: 139, its encoding nucleic acid is shown in SEQ ID NO: 140, and its CDR1, CDR2 and CDR3 are shown in SEQ ID NOs: 7, 17 and 141, respectively.

<-------------FR1-----------> CDR1 <----FR2-----> CDR2 <--
QMQLQESGPGLVKPSQSLSLTCTVTGYSITSDYAWNWIRQFPGNKLEWMGYISY
SGRTSYNPSLKSRIS
-----------FR3--------------> CDR3<---FR4--->
ITRDTSKNQFFLQLNSVTTEDTATYYCARHYGDYWGQGTTLTVSS

### Nucleotide sequence

CAGATGCAGCTTCAGGAGTCGGGACCTGGCCTGGTGAAACCTTCTCAGTCTCT
GTCCCTCACCTGCACTGTCACTGGCTACTCAATCACCAGTGATTATGCCTGGAA
CTGGATCCGGCAGTTTCCAGGAAACAAACTGGAGTGGATGGGCTACATAAGCT
ACAGTGGTAGGACTAGCTACAACCCATCTCTCAAAAGTCGAATCTCTATCACTC
GAGACACATCCAAGAACCAGTTCTTCCTGCAGTTGAATTCTGTGACTACTGAG
GACACAGCCACATATTACTGTGCAAGACACTACGGAGACTACTGGGGCCAAGG
CACCACTCTCACAGTCTCCTCA

The amino acid sequence of light chain VK is shown in SEQ ID NO: 142, its encoding nucleic acid is shown in SEQ ID NO: 143, and its CDR1, CDR2 and CDR3 are shown in SEQ ID NOs: 144, 145 and 146, respectively.

<----------FR1--------> CDR1 <-----FR2-----> CDR2 <-----------
DIVMTQSQKLMSTSVGDRVSVTCKASQNVGTNVAWYQQKPGQSPKALIYSASY
RNSGVPDRFTGSGSGT
----FR3-----------> CDR3 <---FR4--->
DFTLTISNVQSEDLAEYFCQQFNTNPLTFGSGTKLEIK

### Nucleotide sequence

GACATTGTGATGACTCAGTCTCAAAAACTCATGTCCACATCAGTAGGAGACAG
GGTCAGCGTCACCTGCAAGGCCAGTCAGAATGTGGGTACTAATGTAGCCTGGTA
TCAACAGAAACCAGGGCAATCTCCTAAAGCACTGATTTACTCGGCATCCTACCG
GAACAGTGGAGTCCCTGATCGCTTCACAGGCAGTGGTTCTGGGACAGATTTCA
CTCTCACCATCAGCAATGTGCAGTCTGAAGACTTGGCAGAGTATTTCTGTCAGC
AATTTAACACCAATCCTCTCACGTTCGGCTCGGGGACAAAGTTGGAAATAAAA

### Clone: 14F11

The amino acid sequence of the heavy chain VH is shown in SEQ ID NO: 147, its encoding nucleic acid is shown in SEQ ID NO: 148, and its CDR1, CDR2 and CDR3 are shown in SEQ ID NOs: 149, 150 and 151, respectively.

<-------------FR1-----------> CDR1 <----FR2-----> CDR2 <--
QVQLQQSGPQLLRPGASVKISCKASGFSFTNHWIHWVKQRPGQGLEWIGMIDPS
DSETGLNQKFRDKAT
-----------FR3--------------> CDR3 <---FR4--->
LTVDKSSSTAYMHLNSPTSEDSAVYYCARLGIHDGYYIYAMDFWGQGTSVTVSS

### Nucleotide sequence

CAGGTTCAGCTGCAGCAGTCTGGGCCTCAGCTACTTAGGCCTGGGGCTTCAGT
GAAGATATCCTGCAAGGCTTCTGGTTTCTCATTCACCAACCACTGGATACACTG

GGTGAAACAGAGGCCTGGACAAGGTCTTGAGTGGATTGGCATGATTGATCCTT
CCGATAGTGAAACTGGCCTCAATCAGAAGTTCAGGGACAAGGCCACATTGACT
GTAGACAAGTCCTCCAGCACAGCCTACATGCACCTCAACAGCCCGACATCTGA
GGACTCTGCGGTCTATTACTGTGCAAGATTGGGAATCCATGATGGTTACTACATC
TATGCTATGGACTTTTGGGGTCAAGGAACCTCAGTCACCGTCTCCTCA

The amino acid sequence of light chain VK is shown in SEQ ID NO: 152, its encoding nucleic acid is shown in SEQ ID NO: 153, and its CDR1, CDR2 and CDR3 are shown in SEQ ID NOs: 154, 155 and 156, respectively.

<----------FR1--------> CDR1 <-----FR2-----> CDR2 <-----------
NIVMTQSPKSMSMSVGERVTLSCKASENVGTYVSWYQQKPEQSPKLLIYGASNR
YTGVPDRFTGSGSAT
----FR3----------> CDR3 <---FR4--->
DFTLTISSVQAEDLADYHCGQSYSYPLTFGAGTKLELK

### Nucleotide sequence

AACATTGTAATGACCCAATCTCCCAAATCCATGTCCATGTCAGTAGGAGAGAGG
GTCACCTTGAGCTGCAAGGCCAGTGAGAATGTGGGTACTTATGTATCCTGGTAT
CAACAGAAACCAGAGCAGTCTCCTAAACTGCTGATATACGGGGCATCCAACCG
GTACACTGGGGTCCCCGATCGCTTCACAGGCAGTGGATCTGCAACAGATTTCAC
TCTGACCATCAGCAGTGTGCAGGCTGAAGACCTTGCAGATTATCACTGTGGAC
AGAGTTACAGCTATCCGCTCACGTTCGGTGCTGGGACCAAGCTGGAGCTGAAA

### Clone: 20C8

The amino acid sequence of the heavy chain VH is shown in SEQ ID NO: 157, its encoding nucleic acid is shown in SEQ ID NO: 158, and its CDR1, CDR2 and CDR3 are shown in SEQ ID NOs: 7, 17 and 9, respectively.

<------------FR1----------->    CDR1 <----FR2----->              CDR2                <--
EVQLQESGPGLVKPSQSLSLTCSVTGYSITSDYAWNWIRQFPGNKLEWMGYISYS
GRTSYNPSLKSRIS
-----------FR3--------------> CDR3<---FR4--->
ITRDTSKNQFFLQLISVTTEDTATYYCARYWGDYWGQGTTLTVSS

### Nucleotide sequence

GAGGTGCAGCTTCAGGAGTCGGGACCTGGCCTGGTGAAACCTTCTCAGTCTCT
GTCCCTCACCTGCTCTGTCACTGGCTACTCAATCACCAGTGATTATGCCTGGAA
CTGGATCCGGCAGTTTCCAGGAAACAAGCTGGAGTGGATGGGCTACATAAGTT
ACAGTGGTAGAACTAGCTACAACCCATCTCTCAAAAGTCGAATCTCTATCACTC
GAGACACATCCAAGAACCAGTTCTTCCTGCAGTTGATTTCTGTGACTACTGAGG
ACACAGCCACATATTATTGTGCAAGATATTGGGGCGACTACTGGGGCCAAGGCA
CCACTCTCACAGTCTCCTCA

The amino acid sequence of light chain VK is shown in SEQ ID NO: 159, its encoding nucleic acid is shown in SEQ ID NO: 160, and its CDR1, CDR2 and CDR3 are shown in SEQ ID NOs: 161, 162 and 14, respectively.

<-----------FR1--------> CDR1 <-----FR2-----> CDR2 <-----------
DIVMTQSQKFISTSIGDRVSVTCKASQDVGGHVAWFQQRPGQSPKPLIYSATFRH
SGVPDRFTGSGSGT
----FR3-----------> CDR3 <---FR4--->
DFTLTISNLQSEDLAEYVCLQYNSNPLTFGPGTKLELK

### Nucleotide sequence

GACATTGTGATGACCCAGTCTCAAAAATTCATCTCCACATCAATAGGAGACAGG
GTCAGCGTCACCTGCAAGGCCAGTCAGGATGTGGGTGGACATGTGGCCTGGTT
TCAACAGAGACCCGGGCAATCTCCTAAACCTCTGATTTATTCGGCAACCTTCCG
GCACAGTGGAGTCCCTGATCGCTTCACGGGCAGTGGATCTGGGACAGATTTCA
CTCTCACCATCAGTAATCTACAGTCTGAGGACTTGGCAGAATATGTCTGTCTGC
AATATAACAGTAATCCACTCACGTTCGGTCCTGGGACCAAGCTGGAGCTGAAA

### Clone: 21A3

The amino acid sequence of the heavy chain VH is shown in SEQ ID NO: 163, its encoding nucleic acid is shown in SEQ ID NO: 164, and its CDR1, CDR2 and CDR3 are shown in SEQ ID NOs: 7, 165 and 9, respectively.

<------------FR1----------->    CDR1 <----FR2----->              CDR2              <--
DVQLQESGPGLVKPSQSLSLTCTVTGYSITSDYAWNWIRQFPGNKLEWMGYISYS
GRTSYNPYLKSRIS
-----------FR3------------> CDR3<---FR4--->
ITRDTSKNQFFLQLNSVTTEDTATYYCARYWGDYWGQGTTLTVSS

### Nucleotide sequence

GATGTGCAGCTTCAGGAGTCGGGACCTGGCCTGGTGAAACCTTCTCAGTCTCT
GTCCCTCACCTGCACTGTCACTGGCTACTCAATCACCAGTGATTATGCCTGGAA
CTGGATCCGGCAGTTTCCAGGAAACAAACTGGAGTGGATGGGCTACATAAGCT
ACAGTGGTCGCACTAGCTACAACCCATATCTCAAAAGTCGAATTTCTATCACTC
GAGACACATCCAAGAACCAGTTCTTCCTGCAGTTGAATTCTGTGACTACTGAG
GACACAGCCACATATTACTGTGCAAGATATTGGGGCGACTACTGGGGCCAAGG
CACCACTCTCACAGTCTCCTCA

The amino acid sequence of light chain VK is shown in SEQ ID NO: 166, its encoding nucleic acid is shown in SEQ ID NO: 167, and its CDR1, CDR2 and CDR3 are shown in SEQ ID NOs: 144, 168 and 14, respectively.

<----------FR1--------> CDR1 <-----FR2-----> CDR2 <-----------
DIVMTQSQKFMSTSVGDRVSVTCKASQNVGTNVAWYKQKPGQSPKALIYSASY
RHSGVPDRFTGSGSGT
----FR3----------> CDR3 <---FR4--->
DFTLTISNVQSEDLAEYFCLQYNSNPLTFGAGTKLELK

### Nucleotide sequence

GACATTGTGATGACCCAGTCTCAAAAATTCATGTCCACATCAGTAGGAGACAGG
GTCAGCGTCACCTGCAAGGCCAGTCAGAATGTGGGTACTAATGTAGCCTGGTAT
AAACAGAAACCAGGGCAATCTCCTAAAGCACTGATTTACTCGGCATCCTACCG
GCACAGTGGAGTCCCTGATCGCTTCACAGGCAGTGGATCTGGGACAGATTTCA
CTCTCACCATCAGCAATGTGCAGTCTGAAGACTTGGCAGAGTATTTCTGTCTGC
AATATAACAGCAATCCTCTCACGTTCGGTGCTGGGACCAAGCTGGAGCTGAAA

### Clone: 21B1

The amino acid sequence of the heavy chain VH is shown in SEQ ID NO: 169, its encoding nucleic acid is shown in SEQ ID NO: 170, and its CDR1, CDR2 and CDR3 are shown in SEQ ID NOs: 7, 17 and 9, respectively.

<-------------FR1-----------> CDR1 <----FR2-----> CDR2 <--
DVQLQESGPGLVKPSQSLSLTCTVTGYSITSDYAWNWIRQFPGNKLEWMGYISYS
GRTSYNPSLKSRIS
------------FR3-------------> CDR3<---FR4--->
ITRDTSKNQFFLQLNSVTAEDTATYYCARYWGDYWGQGTTLTVSS

### Nucleotide sequence

GATGTGCAGCTTCAGGAGTCGGGACCTGGCCTGGTGAAACCTTCTCAGTCTCT
GTCCCTCACCTGCACTGTCACTGGCTACTCAATCACCAGTGATTATGCCTGGAA
CTGGATCCGGCAGTTTCCAGGAAACAAACTGGAGTGGATGGGCTACATAAGCT
ACAGTGGTCGCACTAGCTACAACCCATCTCTCAAAAGTCGAATCTCTATCACTC
GAGACACATCCAAGAACCAGTTCTTCCTGCAGTTGAATTCTGTGACTGCTGAG
GACACAGCCACATATTACTGTGCAAGATATTGGGGCGACTACTGGGGCCAAGG
CACCACTCTCACAGTCTCCTCA

The amino acid sequence of light chain VK is shown in SEQ ID NO: 171, its encoding nucleic acid is shown in SEQ ID NO: 172, and its CDR1, CDR2 and CDR3 are shown in SEQ ID NOs: 144, 173 and 174, respectively.

<----------FR1-------->        CDR1        <-----FR2----->    CDR2 <-----------
DIVMTQSQKFMSTSVGDRVSVTCKASQNVGTNVAWFQQKPGQSPKALIYSTSYR
SSGVPDRFTGSGSGT
----FR3----------->    CDR3      <---FR4--->
DFTLTISNVQSEDLAEYFCQQFNSNPLTFGGGTKLELK

### Nucleotide sequence

GACATTGTGATGACACAGTCTCAAAAATTCATGTCCACATCAGTAGGAGACAG
GGTCAGCGTCACCTGCAAGGCCAGTCAGAATGTGGGTACTAATGTAGCCTGGT
TTCAACAGAAACCAGGGCAATCTCCTAAAGCACTGATTTACTCGACATCCTACC
GGTCCAGTGGAGTCCCTGATCGCTTCACAGGCAGTGGATCTGGGACAGATTTC
ACTCTCACCATCAGCAATGTGCAGTCTGAAGACTTGGCAGAGTACTTCTGTCAG
CAATTTAACAGCAATCCTCTCACGTTCGGAGGGGGGACCAAGCTGGAGCTGAA

A

### Example 5. Humanization of MASP-2 antibodies

Murine antibodies 28014, 3H6 and 16B 11 with high affinity and activity were selected for humanization of variable region sequences. The glycosylation site NYS of clone 28014 heavy chain CDR3 was first subjected to site-directed mutagenesis, and the mutant was expressed as Fab in E. coli. The affinity assay showed that the S-A mutant (Ala102) with a serine to alanine mutation at position 102 did not affect MASP-2 binding activity after the removal of the glycosylation site of 28014 heavy chain CDR3 (as shown in FIG. 8).

The sequence of murine antibody was aligned with that of human antibody germline to find out the human germline light chain genes which have good homology, are completely identical with the key amino acid sequences maintaining the antibody structure core and have a high frequency of occurrence in the human body, and murine antibody CDR grafting was conducted; the CDR region and its surrounding framework amino acid sequences were analyzed by computer homology modeling to avoid the concentrated distribution of molecular surface charges or hydrophobic regions. Humanization design yielded a total of three heavy and six light chain 16B 11 variants, four heavy and one light chain 3H6 variants, four heavy and one light chain 28014 variants, and three heavy and six light chain 16B 11 variants.

The humanized sequences are as follows:

**Table 6. The Heavy chain variable region sequences of MASP-2 humanized antibodies**

| MASP-2 humanized antibody | HCVR | | HCDR1 | HCDR2 | HCDR3 |
|---|---|---|---|---|---|
| | Amino acid sequence | Nucleotide sequence | Amino acid sequence | Amino acid sequence | Amino acid sequence |
| h16B11 H1 | 175 | 176 | 7 | 17 | 141 |
| h16B11 H2 | 177 | 178 | 7 | 17 | 141 |
| h16B11 H3 | 179 | 180 | 7 | 17 | 141 |
| h3H6 H1 | 181 | 182 | 136 | 183 | 138 |
| h3H6 H2 | 184 | 185 | 136 | 183 | 138 |
| h3H6 H3 | 186 | 187 | 136 | 183 | 138 |
| h3H6 H4 | 188 | 189 | 136 | 183 | 138 |
| h28O14 H1 | 190 | 191 | 126 | 192 | 128 |
| h28O14 H2 | 193 | 194 | 126 | 192 | 128 |
| h28O14 H3 | 195 | 196 | 126 | 192 | 128 |
| h28O14 H4 | 197 | 198 | 126 | 192 | 128 |

**Table 7. The light chain variable region sequences of MASP-2 humanized antibodies**

| MASP-2 humanized antibody | LCVR | | LCDR1 | LCDR2 | LCDR3 |
|---|---|---|---|---|---|
| | Amino acid sequence | Nucleotide sequence | Amino acid sequence | Amino acid sequence | Amino acid sequence |
| h16B11 K1 | 199 | 200 | 201 | 202 | 146 |
| h16B11 K2 | 203 | 204 | 201 | 202 | 146 |
| h16B11 K3 | 205 | 206 | 201 | 207 | 146 |
| h16B11 K4 | 208 | 209 | 201 | 202 | 146 |
| h16B11 K5 | 210 | 211 | 201 | 202 | 146 |
| h16B11 K6 | 212 | 213 | 201 | 207 | 146 |
| h28O14 K1 | 214 | 215 | 216 | 132 | 133 |
| h3H6 K1 | 214 | 215 | 216 | 132 | 133 |

| | | | | | |
|---|---|---|---|---|---|
| h16B11: h16B11 H1 | | | | | |

The amino acid sequence of h16B11 H1 is shown in SEQ ID NO: 175, its encoding nucleic acid is shown in SEQ ID NO: 176, and its CDR1, CDR2 and CDR3 are shown in SEQ ID NOs: 7, 17 and 141, respectively.

<-------------FR1------------->   CDR1 <-----FR2----->            CDR2            <-------
QMQLQESGPGLVKPSETLSLTCTVSGYSITSDYAWNWIRQPPGKGLEWIGYIS
YSGRTSYNPSLKSRVTISRD
----------FR3-----------> CDR3 <----FR4--->
TSKNQFSLKLSSVTAADTAVYYCARHYGDYWGQGTLVTVSS

### Nucleotide sequence

CAGATGCAGCTGCAAGAGTCTGGACCTGGACTGGTCAAGCCCAGCGAGACAC
TGAGCCTGACCTGTACCGTGTCCGGCTACAGCATCACAAGCGACTACGCCTGG
AACTGGATCAGACAGCCTCCTGGCAAAGGCCTCGAGTGGATCGGCTACATCAG
CTACAGCGGCAGAACCAGCTACAACCCCAGCCTGAAGTCCAGAGTGACCATCA
GCAGAGACACCAGCAAGAACCAGTTCTCCCTGAAGCTGAGCAGCGTGACAGC
CGCCGATACAGCCGTGTACTACTGCGCTAGACACTACGGCGATTACTGGGGCCA
GGGAACCCTGGTCACAGTGTCATCT

### h16B11 H2

The amino acid sequence of h16B11 H2 is shown in SEQ ID NO: 177, its encoding nucleic acid is shown in SEQ ID NO: 178, and its CDR1, CDR2 and CDR3 are shown in SEQ ID NOs: 7, 17 and 141, respectively.

<-------------FR1-------------> CDR1 <-----FR2-----> CDR2 <-------
QLQLQESGPGLVKPSETLSLTCTVSGYSITSDYAWNWIRQPPGKGLEWIGYISYSG
RTSYNPSLKSRVTISRD
----------FR3-------------->CDR3<----FR4--->
TSKNQFSLKLSSVTAADTAVYYCARHYGDYWGQGTLVTVSS

### Nucleotide sequence

CAGCTCCAGCTGCAAGAGTCTGGACCTGGACTGGTCAAGCCCAGCGAGACAC
TGAGCCTGACCTGTACCGTGTCCGGCTACAGCATCACAAGCGACTACGCCTGG
AACTGGATCAGACAGCCTCCTGGCAAAGGCCTCGAGTGGATCGGCTACATCAG
CTACAGCGGCAGAACCAGCTACAACCCCAGCCTGAAGTCCAGAGTGACCATCA
GCAGAGACACCAGCAAGAACCAGTTCTCCCTGAAGCTGAGCAGCGTGACAGC

CGCCGATACAGCCGTGTACTACTGCGCTAGACACTACGGCGATTACTGGGGCCA
GGGAACCCTGGTCACAGTGTCATCT

### h16B11 H3

The amino acid sequence of h16B11 H3 is shown in SEQ ID NO: 179, its encoding nucleic acid is shown in SEQ ID NO: 180, and its CDR1, CDR2 and CDR3 are shown in SEQ ID NOs: 7, 17 and 141, respectively.

<-------------FR1-------------> CDR1 <-----FR2-----> CDR2 <-------
QLQLQESGPGLVKPSETLSLTCTVSGYSITSDYAWNWIRQPPGKGLEWIGYISYSG
RTSYNPSLKSRVTISVD
----------FR3------------->CDR3<----FR4--->
TSKNQFSLKLSSVTAADTAVYYCARHYGDYWGQGTLVTVSS

### Nucleotide sequence

CAGCTCCAGCTGCAAGAGTCTGGACCTGGACTGGTCAAGCCCAGCGAGACAC
TGAGCCTGACCTGTACCGTGTCCGGCTACAGCATCACAAGCGACTACGCCTGG
AACTGGATCAGACAGCCTCCTGGCAAAGGCCTCGAGTGGATCGGCTACATCAG
CTACAGCGGCAGAACCAGCTACAACCCCAGCCTGAAGTCCAGAGTGACCATCA
GCGTAGACACCAGCAAGAACCAGTTCTCCCTGAAGCTGAGCAGCGTGACAGC
CGCCGATACAGCCGTGTACTACTGCGCTAGACACTACGGCGATTACTGGGGCCA
GGGAACCCTGGTCACAGTGTCATCT

### h3H6:

### h3H6 H1

The amino acid sequence of h3H6 H1 is shown in SEQ ID NO: 181, its encoding nucleic acid is shown in SEQ ID NO: 182, and its CDR1, CDR2 and CDR3 are shown in SEQ ID NOs: 136, 183 and 138, respectively.

<-------------FR1------------->    CDR1 <-----FR2----->            CDR2            <-------
QVQLVQSGAEVKKPGSSVKVSCKASGYTFATYSIHWVRQAPGQGLEWMGYINP
SSGYTNYNQKFQGRVTLTAD
--------FR3-------------->            CDR3      <----FR4--->
KSTSTAYMELSSLRSEDTAVYYCARVYYTFYHAMDYWGQGTTVTVSS

### Nucleotide sequence

CAGGTTCAGCTGGTTCAGTCTGGCGCCGAAGTGAAGAAACCTGGCAGCAGCG
TGAAGGTGTCCTGCAAGGCTAGCGGCTACACCTTCGCCACCTACAGCATCCACT
GGGTCCGACAGGCTCCAGGACAGGGACTTGAGTGGATGGGCTACATCAACCCC
AGCTCTGGCTACACCAACTACAACCAGAAATTCCAGGGCCGCGTGACCCTCAC
AGCCGACAAGTCTACAAGCACCGCCTACATGGAACTGAGCAGCCTGAGAAGC
GAGGACACCGCCGTGTACTACTGCGCCAGAGTGTACTACACATTCTACCACGCC
ATGGACTACTGGGGCCAGGGCACAACAGTGACCGTTAGCTCT

### h3H6 H2

The amino acid sequence of h3H6 H2 is shown in SEQ ID NO: 184, its encoding nucleic acid is shown in SEQ ID NO: 185, and its CDR1, CDR2 and CDR3 are shown in SEQ ID NOs: 136, 183 and 138, respectively.

<-------------FR1------------->    CDR1<-----FR2----->            CDR2            <-------
QVQLVQSGAEVKKPGSSVKVSCKASGYTFATYSIHWVRQAPGQGLEWMGYINP
SSGYTNYNQKFQGRVTLTAD
--------FR3-------------->            CDR3        <----FR4--->
ESTSTAYMELSSLRSEDTAVYYCARVYYTFYHAMDYWGQGTTVTVSS

### Nucleotide sequence

CAGGTTCAGCTGGTTCAGTCTGGCGCCGAAGTGAAGAAACCTGGCAGCAGCG
TGAAGGTGTCCTGCAAGGCTAGCGGCTACACCTTCGCCACCTACAGCATCCACT
GGGTCCGACAGGCTCCAGGACAGGGACTTGAGTGGATGGGCTACATCAACCCC
AGCTCTGGCTACACCAACTACAACCAGAAATTCCAGGGCCGCGTGACCCTCAC
AGCCGACGAGTCTACAAGCACCGCCTACATGGAACTGAGCAGCCTGAGAAGC
GAGGACACCGCCGTGTACTACTGCGCCAGAGTGTACTACACATTCTACCACGCC
ATGGACTACTGGGGCCAGGGCACAACAGTGACCGTTAGCTCT

### h3H6 H3

The amino acid sequence of h3H6 H3 is shown in SEQ ID NO: 186, its encoding nucleic acid is shown in SEQ ID NO: 187, and its CDR1, CDR2 and CDR3 are shown in SEQ ID NOs: 136, 183 and 138, respectively.

<-------------FR1------------->    CDR1<-----FR2----->            CDR2            <-------
QVQLVQSGAEVKKPGSSVKVSCKASGYTFATYSIHWVRQAPGQGLEWMGYINP
SSGYTNYNQKFQGRVTITAD
--------FR3-------------->            CDR3        <----FR4--->
KSTSTAYMELSSLRSEDTAVYYCARVYYTFYHAMDYWGQGTTVTVSS

### Nucleotide sequence

CAGGTTCAGCTGGTTCAGTCTGGCGCCGAAGTGAAGAAACCTGGCAGCAGCG
TGAAGGTGTCCTGCAAGGCTAGCGGCTACACCTTCGCCACCTACAGCATCCACT
GGGTCCGACAGGCTCCAGGACAGGGACTTGAGTGGATGGGCTACATCAACCCC
AGCTCTGGCTACACCAACTACAACCAGAAATTCCAGGGCCGCGTGACCATCAC
AGCCGACAAGTCTACAAGCACCGCCTACATGGAACTGAGCAGCCTGAGAAGC
GAGGACACCGCCGTGTACTACTGCGCCAGAGTGTACTACACATTCTACCACGCC
ATGGACTACTGGGGCCAGGGCACAACAGTGACCGTTAGCTCT

### h3H6 H4

The amino acid sequence of h3H6 H4 is shown in SEQ ID NO: 188, its encoding nucleic acid is shown in SEQ ID NO: 189, and its CDR1, CDR2 and CDR3 are shown in SEQ ID NOs: 136, 183 and 138, respectively.

<------------FR1------------>   CDR1<-----FR2----->          CDR2          <-------
QVQLVQSGAEVKKPGSSVKVSCKASGYTFATYSIHWVRQAPGQGLEWMGYINP

SSGYTNYNQKFQGRVTITAD
--------FR3--------------> CDR3 <----FR4--->
ESTSTAYMELSSLRSEDTAVYYCARVYYTFYHAMDYWGQGTTVTVSS

### Nucleotide sequence

CAGGTTCAGCTGGTTCAGTCTGGCGCCGAAGTGAAGAAACCTGGCAGCAGCG
TGAAGGTGTCCTGCAAGGCTAGCGGCTACACCTTCGCCACCTACAGCATCCACT
GGGTCCGACAGGCTCCAGGACAGGGACTTGAGTGGATGGGCTACATCAACCCC
AGCTCTGGCTACACCAACTACAACCAGAAATTCCAGGGCCGCGTGACCATCAC
AGCCGACGAGTCTACAAGCACCGCCTACATGGAACTGAGCAGCCTGAGAAGC
GAGGACACCGCCGTGTACTACTGCGCCAGAGTGTACTACACATTCTACCACGCC
ATGGACTACTGGGGCCAGGGCACAACAGTGACCGTTAGCTCT

### h28O14:

### h28O14 H1

The amino acid sequence of h28O14 H1 is shown in SEQ ID NO: 190, its encoding nucleic acid is shown in SEQ ID NO: 191, and its CDR1, CDR2 and CDR3 are shown in SEQ ID NOs: 126, 192 and 128, respectively.

<-------------FR1-------------> CDR1<-----FR2-----> CDR2 <-------
QVQLVQSGAEVKKPGSSVKVSCKASGYTFRSHTMHWVRQAPGQGLEWMGYIN
PSTGYINYNQKFQGRVTLTAD
--------FR3--------------> CDR3 <----FR4--->
KSTSTAYMELSSLRSEDTAVYYCARSNYAYYYGMDYWGQGTTVTVSS

### Nucleotide sequence

CAGGTTCAGCTGGTTCAGTCTGGCGCCGAAGTGAAGAAACCTGGCAGCAGCG
TGAAGGTGTCCTGCAAGGCTAGCGGCTACACCTTCAGATCCCACACCATGCAC
TGGGTCCGACAGGCTCCAGGACAGGGACTTGAGTGGATGGGCTACATCAACCC
CAGCACCGGCTATATCAACTACAACCAGAAATTCCAGGGCCGCGTGACCCTCA
CAGCCGACAAGTCTACAAGCACCGCCTACATGGAACTGAGCAGCCTGAGAAG
CGAGGACACCGCCGTGTACTACTGCGCCAGAAGCAACTACGCCTACTACTACG
GCATGGACTACTGGGGCCAGGGCACCACAGTGACAGTCTCTTCT

### h28O14 H2

The amino acid sequence of h28O14 H2 is shown in SEQ ID NO: 193, its encoding nucleic acid is shown in SEQ ID NO: 194, and its CDR1, CDR2 and CDR3 are shown in SEQ ID NOs: 126, 192 and 128, respectively.

<------------FR1------------>   CDR1<-----FR2----->            CDR2            <-------
QVQLVQSGAEVKKPGSSVKVSCKASGYTFRSHTMHWVRQAPGQGLEWMGYIN
PSTGYINYNQKFQGRVTLTAD
--------FR3------------->          CDR3       <----FR4--->
ESTSTAYMELSSLRSEDTAVYYCARSNYAYYYGMDYWGQGTTVTVSS

### Nucleotide sequence

CAGGTTCAGCTGGTTCAGTCTGGCGCCGAAGTGAAGAAACCTGGCAGCAGCG
TGAAGGTGTCCTGCAAGGCTAGCGGCTACACCTTCAGATCCCACACCATGCAC
TGGGTCCGACAGGCTCCAGGACAGGGACTTGAGTGGATGGGCTACATCAACCC
CAGCACCGGCTATATCAACTACAACCAGAAATTCCAGGGCCGCGTGACCCTCA
CAGCCGACGAGTCTACAAGCACCGCCTACATGGAACTGAGCAGCCTGAGAAG
CGAGGACACCGCCGTGTACTACTGCGCCAGAAGCAACTACGCCTACTACTACG
GCATGGACTACTGGGGCCAGGGCACCACAGTGACAGTCTCTTCT

### h28O14 H3

The amino acid sequence of h28O14 H3 is shown in SEQ ID NO: 195, its encoding nucleic acid is shown in SEQ ID NO: 196, and its CDR1, CDR2 and CDR3 are shown in SEQ ID NOs: 126, 192 and 128, respectively.

<-------------FR1-------------> CDR1<-----FR2-----> CDR2 <-------
QVQLVQSGAEVKKPGSSVKVSCKASGYTFRSHTMHWVRQAPGQGLEWMGYIN
PSTGYINYNQKFQGRVTITAD
--------FR3--------------> CDR3 <----FR4--->
KSTSTAYMELSSLRSEDTAVYYCARSNYAYYYGMDYWGQGTTVTVSS

### Nucleotide sequence

CAGGTTCAGCTGGTTCAGTCTGGCGCCGAAGTGAAGAAACCTGGCAGCAGCG
TGAAGGTGTCCTGCAAGGCTAGCGGCTACACCTTCAGATCCCACACCATGCAC
TGGGTCCGACAGGCTCCAGGACAGGGACTTGAGTGGATGGGCTACATCAACCC
CAGCACCGGCTATATCAACTACAACCAGAAATTCCAGGGCCGCGTGACCATCAC
AGCCGACAAGTCTACAAGCACCGCCTACATGGAACTGAGCAGCCTGAGAAGC
GAGGACACCGCCGTGTACTACTGCGCCAGAAGCAACTACGCCTACTACTACGG
CATGGACTACTGGGGCCAGGGCACCACAGTGACAGTCTCTTCT

### h28O14 H4

The amino acid sequence of h28O14 H4 is shown in SEQ ID NO: 197, its encoding nucleic acid is shown in SEQ ID NO: 198, and its CDR1, CDR2 and CDR3 are shown in SEQ ID NOs: 126, 192 and 128, respectively.

<-------------FR1------------->    CDR1<-----FR2----->            CDR2            <-------
QVQLVQSGAEVKKPGSSVKVSCKASGYTFRSHTMHWVRQAPGQGLEWMGYIN
PSTGYINYNQKFQGRVTITAD
--------FR3-------------->            CDR3        <----FR4--->
ESTSTAYMELSSLRSEDTAVYYCARSNYAYYYGMDYWGQGTTVTVSS

### Nucleotide sequence

CAGGTTCAGCTGGTTCAGTCTGGCGCCGAAGTGAAGAAACCTGGCAGCAGCG
TGAAGGTGTCCTGCAAGGCTAGCGGCTACACCTTCAGATCCCACACCATGCAC
TGGGTCCGACAGGCTCCAGGACAGGGACTTGAGTGGATGGGCTACATCAACCC

CAGCACCGGCTATATCAACTACAACCAGAAATTCCAGGGCCGCGTGACCATCAC
AGCCGACGAGTCTACAAGCACCGCCTACATGGAACTGAGCAGCCTGAGAAGC
GAGGACACCGCCGTGTACTACTGCGCCAGAAGCAACTACGCCTACTACTACGG
CATGGACTACTGGGGCCAGGGCACCACAGTGACAGTCTCTTCT

### h16B11:

### h16B11 K1

The amino acid sequence of h16B1 K1 is shown in SEQ ID NO: 199, its encoding nucleic acid is shown in SEQ ID NO: 200, and its CDR1, CDR2 and CDR3 are shown in SEQ ID NOs: 201, 202 and 146, respectively.

<----------FR1---------> CDR1 <-----FR2------> CDR2 <----------------
DIQMTQSPSSLSASVGDRVTITCRASQNVGTNVAWYQQKPGKAPKALIYSASYR
ESGVPSRFSGSGSGTDFTL
--FR3----------> CDR3 <---FR4--->
TISSLQPEDFATYFCQQFNTNPLTFGGGTKVEIK

### Nucleotide sequence

GACATCCAGATGACACAGAGCCCTAGCAGCCTGTCTGCCAGCGTGGGAGACAG
AGTGACCATCACCTGTAGAGCCAGCCAGAACGTGGGCACAAACGTGGCCTGGT
ATCAGCAGAAGCCTGGCAAGGCTCCCAAGGCGCTGATCTACAGCGCCAGCTAC
AGAGAGAGCGGCGTGCCATCTAGATTCAGCGGCTCTGGCTCTGGCACCGACTT
CACCCTGACAATCTCTAGCCTGCAGCCTGAGGACTTCGCTACCTACTTCTGCCA
GCAGTTCAACACAAACCCTCTGACCTTCGGCGGAGGCACCAAGGTGGAAATC
AAG

### h16B11 K2

The amino acid sequence of h16B11 K2 is shown in SEQ ID NO: 203, its encoding nucleic acid is shown in SEQ ID NO: 204, and its CDR1, CDR2 and CDR3 are shown in SEQ ID NOs: 201, 202 and 146, respectively.

<----------FR1---------> CDR1 <-----FR2------> CDR2 <----------------
DIQMTQSPSSLSASVGDRVTITCRASQNVGTNVAWYQQKPGKAPKLLIYSASYRE
SGVPSRFSGSGSGTDFTL
--FR3----------> CDR3 <---FR4--->
TISSLQPEDFATYFCQQFNTNPLTFGGGTKVEIK

### Nucleotide sequence

GACATCCAGATGACACAGAGCCCTAGCAGCCTGTCTGCCAGCGTGGGAGACAG
AGTGACCATCACCTGTAGAGCCAGCCAGAACGTGGGCACAAACGTGGCCTGGT
ATCAGCAGAAGCCTGGCAAGGCTCCCAAGCTGCTGATCTACAGCGCCAGCTAC
AGAGAGAGCGGCGTGCCATCTAGATTCAGCGGCTCTGGCTCTGGCACCGACTT
CACCCTGACAATCTCTAGCCTGCAGCCTGAGGACTTCGCTACCTACTTCTGCCA
GCAGTTCAACACAAACCCTCTGACCTTCGGCGGAGGCACCAAGGTGGAAATC

AAG

### h16B11 K3

The amino acid sequence of h16B11 K3 is shown in SEQ ID NO: 205, its encoding nucleic acid is shown in SEQ ID NO: 206, and its CDR1, CDR2 and CDR3 are shown in SEQ ID NOs: 201, 207 and 146, respectively.

<----------FR1---------> CDR1 <-----FR2------> CDR2 <---------------
DIQMTQSPSSLSASVGDRVTITCRASQNVGTNVAWYQQKPGKAPKALIYSASYR
QSGVPSRFSGSGSGTDFTL
--FR3----------> CDR3 <---FR4--->
TISSLQPEDFATYFCQQFNTNPLTFGGGTKVEIK

### Nucleotide sequence

GACATCCAGATGACACAGAGCCCTAGCAGCCTGTCTGCCAGCGTGGGAGACAG
AGTGACCATCACCTGTAGAGCCAGCCAGAACGTGGGCACAAACGTGGCCTGGT
ATCAGCAGAAGCCTGGCAAGGCTCCCAAGGCGCTGATCTACAGCGCCAGCTAC
AGACAGAGCGGCGTGCCATCTAGATTCAGCGGCTCTGGCTCTGGCACCGACTT
CACCCTGACAATCTCTAGCCTGCAGCCTGAGGACTTCGCTACCTACTTCTGCCA
GCAGTTCAACACAAACCCTCTGACCTTCGGCGGAGGCACCAAGGTGGAAATC
AAG

### h16B11 K4

The amino acid sequence of h16B11 K4 is shown in SEQ ID NO: 208, its encoding nucleic acid is shown in SEQ ID NO: 209, and its CDR1, CDR2 and CDR3 are shown in SEQ ID NOs: 201, 202 and 146, respectively.

<----------FR1---------> CDR1 <-----FR2------> CDR2 <---------------
EIVMTQSPATLSVSPGERATLSCRASQNVGTNVAWYQQKPGQAPRALIYSASYR
ESGIPARFSGSGSGTEFTL
--FR3----------> CDR3 <---FR4--->
TISSLQSEDFAVYFCQQFNTNPLTFGGGTKVEIK

### Nucleotide sequence

GAGATCGTGATGACACAGAGCCCCGCCACACTGAGTGTGTCTCCAGGCGAAAG
AGCTACCCTGAGCTGTAGAGCCTCTCAGAACGTGGGCACAAACGTGGCCTGGT
ATCAGCAGAAGCCTGGACAGGCTCCCAGAGCGCTGATCTACAGCGCCAGCTAC
AGAGAGTCTGGCATCCCTGCTAGATTCAGCGGCTCTGGCTCTGGCACCGAGTTC
ACCCTGACAATCAGCAGCCTGCAGAGCGAGGACTTCGCCGTGTACTTCTGCCA
GCAGTTCAACACAAACCCTCTGACCTTCGGCGGAGGCACCAAGGTGGAAATC
AAG

### h16B11 K5

The amino acid sequence of h16B11 K5 is shown in SEQ ID NO: 210, its encoding nucleic acid is shown in SEQ ID NO: 211, and its CDR1, CDR2 and CDR3 are shown in SEQ ID NOs: 201, 202 and 146, respectively.

<----------FR1--------->        CDR1        <-----FR2------>    CDR2    <----------------
EIVMTQSPATLSVSPGERATLSCRASQNVGTNVAWYQQKPGQAPRLLIYSASYRE
SGIPARFSGSGSGTEFTL
--FR3---------->        CDR3        <---FR4--->
TISSLQSEDFAVYFCQQFNTNPLTFGGGTKVEIK

### Nucleotide sequence

GAGATCGTGATGACACAGAGCCCCGCCACACTGAGTGTGTCTCCAGGCGAAAG
AGCTACCCTGAGCTGTAGAGCCTCTCAGAACGTGGGCACAAACGTGGCCTGGT
ATCAGCAGAAGCCTGGACAGGCTCCCAGACTGCTGATCTACAGCGCCAGCTAC
AGAGAGTCTGGCATCCCTGCTAGATTCAGCGGCTCTGGCTCTGGCACCGAGTTC
ACCCTGACAATCAGCAGCCTGCAGAGCGAGGACTTCGCCGTGTACTTCTGCCA
GCAGTTCAACACAAACCCTCTGACCTTCGGCGGAGGCACCAAGGTGGAAATC
AAG

### h16B11 K6

The amino acid sequence of h16B1 K6 is shown in SEQ ID NO: 212, its encoding nucleic acid is shown in SEQ ID NO: 213, and its CDR1, CDR2 and CDR3 are shown in SEQ ID NOs: 201, 207 and 146, respectively.

<----------FR1---------> CDR1 <-----FR2------> CDR2 <---------------
EIVMTQSPATLSVSPGERATLSCRASQNVGTNVAWYQQKPGQAPRALIYSASYR
QSGIPARFSGSGSGTEFTL
--FR3---------> CDR3 <---FR4--->
TISSLQSEDFAVYFCQQFNTNPLTFGGGTKVEIK

### Nucleotide sequence

GAGATCGTGATGACACAGAGCCCCGCCACACTGAGTGTGTCTCCAGGCGAAAG
AGCTACCCTGAGCTGTAGAGCCTCTCAGAACGTGGGCACAAACGTGGCCTGGT
ATCAGCAGAAGCCTGGACAGGCTCCCAGAGCGCTGATCTACAGCGCCAGCTAC
AGACAGTCTGGCATCCCTGCTAGATTCAGCGGCTCTGGCTCTGGCACCGAGTTC
ACCCTGACAATCAGCAGCCTGCAGAGCGAGGACTTCGCCGTGTACTTCTGCCA
GCAGTTCAACACAAACCCTCTGACCTTCGGCGGAGGCACCAAGGTGGAAATC
AAG

### h28O14:

### h28O14 K1

The amino acid sequence of h28O14 K1 is shown in SEQ ID NO: 214, its encoding nucleic acid is shown in SEQ ID NO: 215, and its CDR1, CDR2 and CDR3 are shown in SEQ ID NOs: 216, 132 and 133, respectively.



--FR3---------->      CDR3      <---FR4--->
TISSLQPEDFATYYCQQHYNIPLTFGGGTKVEIK

### Nucleotide sequence

GACATCCAGATGACACAGAGCCCTAGCAGCCTGTCTGCCAGCGTGGGAGACAG
AGTGACCATCACCTGTAGAGCCAGCCAGGACGTGAACACAGCCGTGGCTTGGT
ATCAGCAGAAGCCTGGCAAGGCCCCTAAGCTGCTGATCTACAGCGCCAGCTAC
AGATACACCGGCGTGCCCTCTAGATTCAGCGGCTCTGGCTCTGGCACCGACTTC
ACCCTGACAATCTCTAGCCTGCAGCCTGAGGACTTCGCCACCTACTACTGCCAG
CAGCACTACAACATCCCTCTGACCTTCGGCGGAGGCACCAAGGTGGAAATCAA
G

### h3H6 K:

### h3H6 K1

The amino acid sequence of h3H6 K1 is shown in SEQ ID NO: 214, its encoding nucleic acid is shown in SEQ ID NO: 215, and its CDR1, CDR2 and CDR3 are shown in SEQ ID NOs: 216, 132 and 133, respectively.

<-----------FR1---------->        CDR1        <-----FR2------>    CDR2    <-----------------
DIQMTQSPSSLSASVGDRVTITCRASQDVNTAVAWYQQKPGKAPKLLIYSASYRY
TGVPSRFSGSGSGTDFTL
--FR3----------->        CDR3        <---FR4--->
TISSLQPEDFATYYCQQHYNIPLTFGGGTKVEIK

### Nucleotide sequence

GACATCCAGATGACACAGAGCCCTAGCAGCCTGTCTGCCAGCGTGGGAGACAG
AGTGACCATCACCTGTAGAGCCAGCCAGGACGTGAACACAGCCGTGGCTTGGT
ATCAGCAGAAGCCTGGCAAGGCCCCTAAGCTGCTGATCTACAGCGCCAGCTAC
AGATACACCGGCGTGCCCTCTAGATTCAGCGGCTCTGGCTCTGGCACCGACTTC
ACCCTGACAATCTCTAGCCTGCAGCCTGAGGACTTCGCCACCTACTACTGCCAG
CAGCACTACAACATCCCTCTGACCTTCGGCGGAGGCACCAAGGTGGAAATCAA
G

After full-sequence synthesis of the light and heavy chains, respectively, they were cloned into eukaryotic expression vectors containing antibody kappa chain constant region Ckappa or human IgG4 constant region CH1-CH3. The light and heavy chain plasmids were pair-matched, then transfected into CHO-S cells and expressed at 37°C for 5-6 days. The culture supernatant was collected and purified by the Protein A column.

### Example 6. MASP-2 inhibitory activity of humanized antibodies

### C4 cleavage activity assay

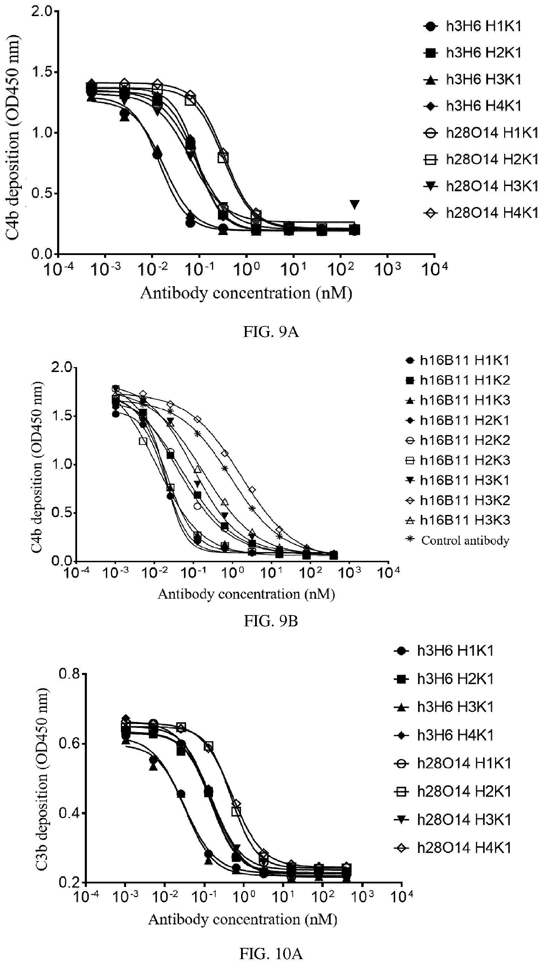

According to the C4 cleavage assay in Example 1, different concentrations of MASP-2 antibodies (initial concentration of 30 µg/ml, 5-fold gradient dilution, 9 gradients in total) were added to the reaction wells at 50 µl per well and allowed to react at 4°C for 30 min. 50 µl of 2 µg/ml C4 pure protein (CompTech) was added, and reacted in a constant temperature incubator at 37°C for 90 min, then C4b antibody was added to detect C4b deposition. As can be seen from Figures 9A, 9B and Table 8, the humanized antibodies can significantly inhibit MASP-2 activity.

**Table 8. Inhibitory activity of C4b deposition by MASP-2 humanized antibodies**

| Humanized antibody | Heavy chain variable region | Light chain variable region | IC50 (nM) |
|---|---|---|---|
| h3H6 H1K1 | h3H6 H1 | h3H6 K1 | 0.014 |
| h3H6 H2K1 | h3H6 H2 | h3H6 K1 | 0.086 |
| h3H6 H3K1 | h3H6 H3 | h3H6 K1 | 0.017 |
| h3H6 H4K1 | h3H6 H4 | h3H6 K1 | 0.090 |
| h28O14 H1K1 | h28O14 H1 | h28O14 K1 | 0.083 |
| h28O14 H2K1 | h28O14 H2 | h28O14 K1 | 0.324 |
| h28O14 H3K1 | h28O14 H3 | h28O14 K1 | 0.064 |
| h28O14 H4K1 | h28O14 H4 | h28O14 K1 | 0.338 |
| h16B11 H1K1 | h16B11 H1 | h16B11 K1 | 0.020 |
| h16B11 H1K2 | h16B11 H1 | h16B11 K2 | 0.051 |
| h16B11 H1K3 | h16B11 H1 | h16B11 K3 | 0.021 |
| h16B11 H2K1 | h16B11 H2 | h16B11 K1 | 0.020 |
| h16B11 H2K2 | h16B11 H2 | h16B11 K2 | 0.032 |
| h16B11 H2K3 | h16B11 H2 | h16B11 K3 | 0.012 |
| h16B11 H3K1 | h16B11 H3 | h16B11 K1 | 0.098 |
| h16B11 H3K2 | h16B11 H3 | h16B11 K2 | 1.691 |
| h16B11 H3K3 | h16B11 H3 | h16B11 K3 | 0.440 |
| Control antibody | | | 0.909 |

### C3 convertase activity assay

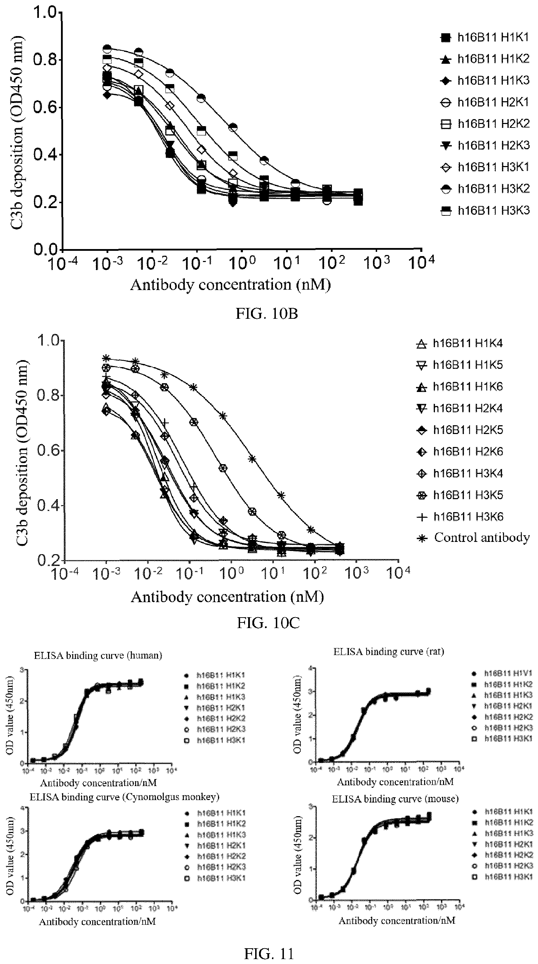

According to the C3 convertase activity assay in Example 1, different concentrations of MASP-2 antibodies (initial concentration of 30 µg/ml, 5-fold gradient dilution, 9 gradients in total) were added to the reaction wells at 50 µl per well and reacted at 37°C for 90 min. C3b antibody was added at 1:500 to detect C3b deposition. As can be seen in Figures 10A, 10B, 10C and Table 9, each humanized antibody can inhibit the deposition of C3b.

**Table 9. Inhibitory activity of C3b deposition by MASP-2 humanized antibodies**

| Humanized antibody | Heavy chain variable region | Light chain variable region | IC50 (nM) |
|---|---|---|---|
| h3H6 H1K1 | h3H6 H1 | h3H6 K1 | 0.030 |
| h3H6 H2K1 | h3H6 H2 | h3H6 K1 | 0.151 |
| h3H6 H3K1 | h3H6 H3 | h3H6 K1 | 0.034 |
| h3H6 H4K1 | h3H6 H4 | h3H6 K1 | 0.151 |
| h28O14 H1K1 | h28O14 H1 | h28O14 K1 | 0.146 |
| h28O14 H2K1 | h28O14 H2 | h28O14 K1 | 0.483 |
| h28O14 H3K1 | h28O14 H3 | h28O14 K1 | 0.161 |
| h28O14 H4K1 | h28O14 H4 | h28O14 K1 | 0.506 |
| h16B11 H1K1 | h16B11 H1 | h16B11 K1 | 0.014 |
| h16B11 H1K2 | h16B11 H1 | h16B11 K2 | 0.040 |
| h16B11 H1K3 | h16B11 H1 | h16B11 K3 | 0.023 |
| h16B11 H2K3 | h16B11 H2 | h16B11 K3 | 0.018 |
| h16B11 H2K1 | h16B11 H2 | h16B11 K1 | 0.017 |
| h16B11 H2K2 | h16B11 H2 | h16B11 K2 | 0.028 |
| h16B11 H3K1 | h16B11 H3 | h16B11 K1 | 0.060 |
| h16B11 H3K2 | h16B11 H3 | h16B11 K2 | 0.399 |
| h16B11 H3K3 | h16B11 H3 | h16B11 K3 | 0.112 |
| h16B11 H1K4 | h16B11 H1 | h16B11 K4 | 0.015 |
| h16B11 H1K5 | h16B11 H1 | h16B11 K5 | 0.024 |
| h16B11 H1K6 | h16B11 H1 | h16B11 K6 | 0.018 |
| h16B11 H2K4 | h16B11 H2 | h16B11 K4 | 0.015 |
| h16B11 H2K5 | h16B11 H2 | h16B11 K5 | 0.032 |
| h16B11 H2K6 | h16B11 H2 | h16B11 K6 | 0.018 |
| h16B11 H3K4 | h16B11 H3 | h16B11 K4 | 0.052 |
| h16B11 H3K5 | h16B11 H3 | h16B11 K5 | 0.440 |
| h16B11 H3K6 | h16B11 H3 | h16B11 K6 | 0.071 |
| Control antibody | | | 3.692 |

### Example 7. Affinity of MASP-2 humanized antibodies

(1) ELISA detection of the affinity of humanized MASP-2 antibodies with each species antigen

Human MASP-2 D4-6 recombinant protein was coated at 1 µg/mL in a 96-well ELISA plate and incubated overnight at 4°C. After blocking with 2% skim milk at room temperature for 1 h, gradient-diluted MASP-2 antibodies (initial concentration of 30 µg/ml, 1:4 gradient dilution) were added and incubated at room temperature for 60 min. Goat anti-human Fc-HRP diluted 1:4000 was added and incubated at room temperature for 60 min. The color was developed with TMB for 10 min, then the reaction was stopped by adding concentrated sulfuric acid. The OD450 nm was determined, and the results were calculated using Graph Pad Prism 5 software.

The results are shown in FIG. 11, in which the MASP-2 humanized antibodies bind to human, Cynomolgus monkey, rat and mouse MASP-2 with comparable affinity.

### (2) BIAcore detection of the affinity of humanized antibodies with mouse MASP-2

The antibody of 0.1 µg/ml was captured on the surface of the Protein A chip, with a capture time of 40 sec and a flow rate of 10 µl/min. The chip was washed with buffer until the baseline leveled off, then gradient diluted antigen was flowed through the chip at the flow rate of 30 µl/min, with a binding time of 120 sec and a dissociation time of 600 sec. The equilibrium dissociation constant (KD) was obtained by fitting. The affinity measurements are shown in Table 10.

**Table 10. The affinity of MASP-2 antibodies with MASP-2**

| MASP-2 | Human MASP-2-D4-6 | | | Mouse MASP-2-D4-6 | | |
|---|---|---|---|---|---|---|
| | ka | kd (1/s) | KD (M) | ka | kd (1/s) | KD (M) |
| h16B11 | 3.26E+0 | 3.77E-0 | 1.16E-1 | 4.66E+0 | 1.38E-0 | 2.95E-0 |
| h16B11 | 5.00E+0 | 1.46E-0 | 2.92E-1 | 9.36E+0 | 1.18E-0 | 1.26E-0 |
| h16B11 | 6.35E+0 | 1.91E-0 | 3.01E-1 | 6.88E+0 | 3.71E-0 | 5.39E-0 |
| h16B11 | 4.33E+0 | 4.97E-0 | 1.15E-1 | 5.00E+0 | 8.47E-0 | 1.69E-0 |
| h16B11 | 3.81E+0 | 1.40E-0 | 3.69E-1 | 6.24E+0 | 6.42E-0 | 1.03E-0 |
| h16B11 | 4.60E+0 | 5.74E-0 | 1.25E-1 | 5.88E+0 | 2.16E-0 | 3.68E-0 |
| h16B11 | 4.91E+0 | 3.35E-0 | 6.83E-1 | 4.75E+0 | 1.44E-0 | 3.04E-0 |
| h16B11 | 4.66E+0 | 1.41E-0 | 3.02E-1 | 6.91E+0 | 8.71E-0 | 1.26E-0 |
| h16B11 | 5.23E+0 | 5.04E-0 | 9.63E-1 | 5.54E+0 | 3.42E-0 | 6.18E-0 |
| h16B11 | 4.23E+0 | 5.14E-0 | 1.22E-1 | 4.78E+0 | 8.63E-0 | 1.81E-0 |
| h16B11 | 3.88E+0 | 1.34E-0 | 3.46E-1 | 6.10E+0 | 5.95E-0 | 9.76E-0 |
| h16B11 | 4.67E+0 | 6.30E-0 | 1.35E-1 | 5.44E+0 | 2.25E-0 | 4.13E-0 |
| h16B11 | 3.61E+0 | 1.21E-0 | 3.34E-1 | 6.97E+0 | 6.73E-0 | 9.65E-0 |
| h16B11 | 3.51E+0 | 6.77E-0 | 1.93E-1 | 1.43E+0 | 2.76E+0 | 1.93E-0 |
| h16B11 | 3.63E+0 | 1.66E-0 | 4.57E-1 | 1.23E+0 | 1.90E-0 | 1.54E-0 |
| h16B11 | 2.97E+0 | 1.20E-0 | 4.03E-1 | 5.26E+0 | 6.06E-0 | 1.15E-0 |
| h16B11 | 3.04E+0 | 5.66E-0 | 1.87E-1 | 2.76E+0 | 9.55E-0 | 3.47E-0 |
| h16B11 | 3.07E+0 | 1.74E-0 | 5.66E-1 | 8.07E+0 | 1.80E-0 | 2.22E-0 |

### Example 8. Shiga toxin 2 induced hemolytic uremic syndrome model in mice

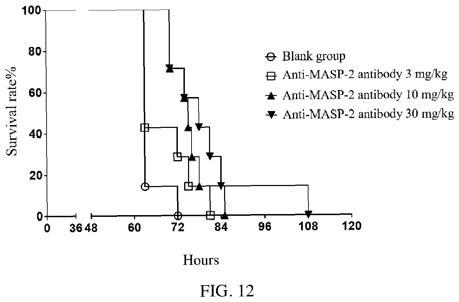

cDNA plasmid with STX2 (Sino Biological, cat. No: EG40019-G) was used as a template for PCR amplification of the STX2A (Arg23-Lys319) and STX2B (Ala20-Asp89) gene fragments of E. coli O157:H7, and cloned into a prokaryotic expression plasmid, in which the N-terminus of STX2A protein was fused with twin strep tag to facilitate purification, and an enterokinase cleavage site, DDDDK, was added between them. The constructed STX2 expression plasmid was transformed into TG1 E. coli competent cells, then a single clone was picked into bacterial culture medium and added IPTG to induce and cultured overnight. The bacteria were collected, and PPB and 5 mM MgSO4 solution were added successively. The bacterial cell wall was lysed, and the protein expression solution in the periplasmic cavity was collected by centrifugation. Shiga toxin STX2 protein was purified by Strep-TactinX (Sino HaiGene, cat. No: C0402), and EK enterokinase was added to the protein collection solution and incubated overnight at 25°C to cleave the twin strep tag. About 18 g C57BL/6 female mice were selected and divided into 4 dose groups of MASP-2 antibodies 0 mg/kg, 3 mg/kg, 10 mg/kg and 30 mg/kg, with 7 mice in each group. The anti-MASP-2 antibody h16B11 H2K4 (100 µl) was injected via tail vein 2 hours in advance, followed by intraperitoneal injection of LPS (5 µg/animal, L6136-Sigma) and STX2 (10 ng/animal). Mice were monitored for survival rate beginning 60 hours after dosing and sacrificed upon reaching the lethargic phase of the HUS pathology. The results are shown in FIG. 12, in which anti-MASP-2 antibodies significantly prolonged the survival time of mice in a dose-dependent manner.

## Claims

1. An antibody or antigen-binding portion thereof that binds to MASP-2, comprising heavy chain CDRs selected from the group consisting of amino acid sequences SEQ ID NOs: 7, 8, 9, 17, 18, 26, 27, 28, 36, 37, 38, 46, 47, 60, 65, 66, 67, 79, 85, 86, 94, 95, 96, 104, 105, 113, 114, 126, 127, 128, 136, 137, 138, 141, 149, 150, 151, 165, 183, 192 or any variant thereof, and/or light chain CDRs selected from the group consisting of amino acid sequences SEQ ID NOs: 12, 13, 14, 21, 22, 23, 31, 32, 33, 41, 42, 43, 50, 51, 52, 57, 70, 71, 72, 82, 91, 108, 109, 110, 117, 118, 123, 131, 132, 133, 144, 145, 146, 154, 155, 156, 161, 162, 168, 173, 174, 201, 202, 207, 216 or any variant thereof.

2. The antibody or antigen-binding portion thereof of claim 1, comprising a heavy chain CDR1 selected from the group consisting of amino acid sequences SEQ ID NOs: 7, 26, 36, 65, 94, 126, 136, 149 or any variant thereof, a heavy chain CDR2 selected from the group consisting of amino acid sequences SEQ ID NOs: 8, 17, 27, 37, 46, 66, 85, 95, 104, 113, 127, 137, 150, 165, 183, 192 or any variant thereof, and a heavy chain CDR3 selected from the group consisting of amino acid sequences SEQ ID NOs: 9, 18, 28, 38, 47, 60, 67, 79, 86, 96, 105, 114, 128, 138, 141, 151 or any variant thereof; and/or a light chain CDR1 selected from the group consisting of amino acid sequences SEQ ID NOs: 12, 21, 31, 41, 50, 70, 99, 108, 117, 123, 131, 144, 154, 161, 201, 216 or any variant thereof, a light chain CDR2 selected from the group consisting of amino acid sequences SEQ ID NOs: 13, 22, 32, 42, 51, 57, 71, 100, 109, 118, 132, 145, 155, 162, 168, 173, 202, 207 or any variant thereof, and a light chain CDR3 selected from the group consisting of amino acid sequences SEQ ID NOs: 14, 23, 33, 43, 52, 72, 82, 91, 101, 110, 133, 146, 156, 174 or any variant thereof.

3. The antibody or antigen-binding portion thereof of claim 1 or 2, comprising a combination of heavy and light chain CDRs selected from the group consisting of:
(1) the heavy chain CDR1, CDR2 and CDR3 sequences comprising SEQ ID NOs: 7, 8 and 9, respectively, and/or the light chain CDR1, CDR2 and CDR3 sequences comprising SEQ ID NOs: 12, 13 and 14, respectively;
(2) the heavy chain CDR1, CDR2 and CDR3 sequences comprising SEQ ID NOs: 7, 17 and 18, respectively, and/or the light chain CDR1, CDR2 and CDR3 sequences comprising SEQ ID NOs: 21, 22 and 23, respectively;
(3) the heavy chain CDR1, CDR2 and CDR3 sequences comprising SEQ ID NOs: 26, 27 and 28, respectively, and/or the light chain CDR1, CDR2 and CDR3 sequences comprising SEQ ID NOs: 31, 32 and 33, respectively;
(4) the heavy chain CDR1, CDR2 and CDR3 sequences comprising SEQ ID NOs: 36, 37 and 38, respectively, and/or the light chain CDR1, CDR2 and CDR3 sequences comprising SEQ ID NOs: 41, 42 and 43, respectively;
(5) the heavy chain CDR1, CDR2 and CDR3 sequences comprising SEQ ID NOs: 7, 46 and 47, respectively, and/or the light chain CDR1, CDR2 and CDR3 sequences comprising SEQ ID NOs: 50, 51 and 52, respectively;
(6) the heavy chain CDR1, CDR2 and CDR3 sequences comprising SEQ ID NOs: 7, 17 and 18, respectively, and/or the light chain CDR1, CDR2 and CDR3 sequences comprising SEQ ID NOs: 21, 57 and 23, respectively;
(7) the heavy chain CDR1, CDR2 and CDR3 sequences comprising SEQ ID NOs: 7, 17 and 60, respectively, and/or the light chain CDR1, CDR2 and CDR3 sequences comprising SEQ ID NOs: 50, 51 and 52, respectively;
(8) the heavy chain CDR1, CDR2 and CDR3 sequences comprising SEQ ID NOs: 65, 66 and 67, respectively, and/or the light chain CDR1, CDR2 and CDR3 sequences comprising SEQ ID NOs: 70, 71 and 72, respectively;
(9) the heavy chain CDR1, CDR2 and CDR3 sequences comprising SEQ ID NOs: 7, 17 and 47, respectively, and/or the light chain CDR1, CDR2 and CDR3 sequences comprising SEQ ID NOs: 50, 51 and 52, respectively;
(10) the heavy chain CDR1, CDR2 and CDR3 sequences comprising SEQ ID NO: 7, 17 and 79, respectively, and/or the light chain CDR1, CDR2 and CDR3 sequences comprising SEQ ID NO: 50, 51 and 82, respectively;
(11) the heavy chain CDR1, CDR2 and CDR3 sequences comprising SEQ ID NOs: 36, 85 and 86, respectively, and/or the light chain CDR1, CDR2 and CDR3 sequences comprising SEQ ID NOs: 41, 42 and 43, respectively;
(12) the heavy chain CDR1, CDR2 and CDR3 sequences comprising SEQ ID NOs: 36, 85 and 86, respectively, and/or the light chain CDR1, CDR2 and CDR3 sequences comprising SEQ ID NOs: 41, 42 and 91, respectively;
(13) the heavy chain CDR1, CDR2 and CDR3 sequences comprising SEQ ID NO: 94, 95 and 96, respectively, and/or the light chain CDR1, CDR2 and CDR3 sequences comprising SEQ ID NO: 99, 100 and 101, respectively;
(14) the heavy chain CDR1, CDR2 and CDR3 sequences comprising SEQ ID NOs: 7, 104 and 105, respectively, and/or the light chain CDR1, CDR2 and CDR3 sequences comprising SEQ ID NOs: 108, 109 and 110, respectively;
(15) the heavy chain CDR1, CDR2 and CDR3 sequences comprising SEQ ID NOs: 26, 113 and 114, respectively, and/or the light chain CDR1, CDR2 and CDR3 sequences comprising SEQ ID NOs: 117, 118 and 33, respectively;
(16) the heavy chain CDR1, CDR2 and CDR3 sequences comprising SEQ ID NOs: 94, 95 and 96, respectively, and/or the light chain CDR1, CDR2 and CDR3 sequences comprising SEQ ID NOs: 123, 100 and 101, respectively;
(17) the heavy chain CDR1, CDR2 and CDR3 sequences comprising SEQ ID NOs: 126, 127 and 128, respectively, and/or the light chain CDR1, CDR2 and CDR3 sequences comprising SEQ ID NOs: 131, 132 and 133, respectively;
(18) the heavy chain CDR1, CDR2 and CDR3 sequences comprising SEQ ID NOs: 136, 137 and 138, respectively, and/or the light chain CDR1, CDR2 and CDR3 sequences comprising SEQ ID NOs: 131, 132 and 133, respectively;
(19) the heavy chain CDR1, CDR2 and CDR3 sequences comprising SEQ ID NOs: 7, 17 and 141, respectively, and/or the light chain CDR1, CDR2 and CDR3 sequences comprising SEQ ID NOs: 144, 145 and 146, respectively;
(20) the heavy chain CDR1, CDR2 and CDR3 sequences comprising SEQ ID NOs: 149, 150 and 151, respectively, and/or the light chain CDR1, CDR2 and CDR3 sequences comprising SEQ ID NOs: 154, 155 and 156, respectively;
(21) the heavy chain CDR1, CDR2 and CDR3 sequences comprising SEQ ID NOs: 7, 17 and 9, respectively, and/or the light chain CDR1, CDR2 and CDR3 sequences comprising SEQ ID NOs: 161, 162 and 14, respectively;
(22) the heavy chain CDR1, CDR2 and CDR3 sequences comprising SEQ ID NOs: 7, 165 and 9, respectively, and/or the light chain CDR1, CDR2 and CDR3 sequences comprising SEQ ID NOs: 144, 168 and 14, respectively;
(23) the heavy chain CDR1, CDR2 and CDR3 sequences comprising SEQ ID NOs: 7, 17 and 9, respectively, and/or the light chain CDR1, CDR2 and CDR3 sequences comprising SEQ ID NOs: 144, 173 and 174, respectively;
(24) the heavy chain CDR1, CDR2 and CDR3 sequences comprising SEQ ID NOs: 136, 183 and 138, respectively, and/or the light chain CDR1, CDR2 and CDR3 sequences comprising SEQ ID NOs: 216, 132 and 133, respectively;
(25) the heavy chain CDR1, CDR2 and CDR3 sequences comprising SEQ ID NOs: 126, 192 and 128, respectively, and/or the light chain CDR1, CDR2 and CDR3 sequences comprising SEQ ID NOs: 216, 132 and 133, respectively;
(26) the heavy chain CDR1, CDR2 and CDR3 sequences comprising SEQ ID NOs: 7, 17 and 141, respectively, and/or the light chain CDR1, CDR2 and CDR3 sequences comprising SEQ ID NOs: 201, 202 and 146, respectively;
(27) the heavy chain CDR1, CDR2 and CDR3 sequences comprising SEQ ID NOs: 7, 17 and 141, respectively, and/or the light chain CDR1, CDR2 and CDR3 sequences comprising SEQ ID NOs: 201, 207 and 146, respectively.

4. The antibody or antigen-binding portion thereof of claim 1 or 2, comprising a heavy chain variable region selected from the group consisting of amino acid sequences SEQ ID NOs: 5, 15, 24, 34, 44, 53, 58, 63, 73, 77, 83, 92, 102, 111, 119, 124, 134, 139, 147, 157, 163, 169, 175, 177, 179, 181, 184, 186, 188, 190, 193, 195, 197 or any variant thereof, and/or a light chain variable region selected from the group consisting of amino acid sequences SEQ ID NOs: 10, 19, 29, 39, 48, 55, 61, 68, 75, 80, 87, 89, 97, 106, 115, 121, 129, 142, 152, 159, 166, 171, 199, 203, 205, 208, 210, 212, 214 or any variant thereof;
preferably, comprising the heavy chain variable region of the amino acid sequence SEQ ID NO: 5 or any variant thereof, and the light chain variable region of the amino acid sequence SEQ ID NO: 10 or any variant thereof;
preferably, comprising the heavy chain variable region of the amino acid sequence SEQ ID NO: 15 or any variant thereof, and the light chain variable region of the amino acid sequence SEQ ID NO: 19 or any variant thereof;
preferably, comprising the heavy chain variable region of the amino acid sequence SEQ ID NO: 24 or any variant thereof, and the light chain variable region of the amino acid sequence SEQ ID NO: 29 or any variant thereof;
preferably, comprising the heavy chain variable region of the amino acid sequence SEQ ID NO: 34 or any variant thereof, and the light chain variable region of the amino acid sequence SEQ ID NO: 39 or any variant thereof;
preferably, comprising the heavy chain variable region of the amino acid sequence SEQ ID NO: 44 or any variant thereof, and the light chain variable region of the amino acid sequence SEQ ID NO: 48 or any variant thereof;
preferably, comprising the heavy chain variable region of the amino acid sequence SEQ ID NO: 53 or any variant thereof, and the light chain variable region of the amino acid sequence SEQ ID NO: 55 or any variant thereof;
preferably, comprising the heavy chain variable region of the amino acid sequence SEQ ID NO: 58 or any variant thereof, and the light chain variable region of the amino acid sequence SEQ ID NO: 61 or any variant thereof;
preferably, comprising the heavy chain variable region of the amino acid sequence SEQ ID NO: 63 or any variant thereof, and the light chain variable region of the amino acid sequence SEQ ID NO: 68 or any variant thereof;
preferably, comprising the heavy chain variable region of the amino acid sequence SEQ ID NO: 73 or any variant thereof, and the light chain variable region of the amino acid sequence SEQ ID NO: 75 or any variant thereof;
preferably, comprising the heavy chain variable region of the amino acid sequence SEQ ID NO: 77 or any variant thereof, and the light chain variable region of the amino acid sequence SEQ ID NO: 80 or any variant thereof;
preferably, comprising the heavy chain variable region of the amino acid sequence SEQ ID NO: 83 or any variant thereof, and the light chain variable region of the amino acid sequence SEQ ID NO: 87 or any variant thereof;
preferably, comprising the heavy chain variable region of the amino acid sequence SEQ ID NO: 83 or any variant thereof, and the light chain variable region of the amino acid sequence SEQ ID NO: 89 or any variant thereof;
preferably, comprising the heavy chain variable region of the amino acid sequence SEQ ID NO: 92 or any variant thereof, and the light chain variable region of the amino acid sequence SEQ ID NO: 97 or any variant thereof;
preferably, comprising the heavy chain variable region of the amino acid sequence SEQ ID NO: 102 or any variant thereof, and the light chain variable region of the amino acid sequence SEQ ID NO: 106 or any variant thereof;
preferably, comprising the heavy chain variable region of the amino acid sequence SEQ ID NO: 111 or any variant thereof, and the light chain variable region of the amino acid sequence SEQ ID NO: 115 or any variant thereof;
preferably, comprising the heavy chain variable region of the amino acid sequence SEQ ID NO: 119 or any variant thereof, and the light chain variable region of the amino acid sequence SEQ ID NO: 121 or any variant thereof;
preferably, comprising the heavy chain variable region of the amino acid sequence SEQ ID NO: 124 or any variant thereof, and the light chain variable region of the amino acid sequence SEQ ID NO: 129 or any variant thereof;
preferably, comprising the heavy chain variable region of the amino acid sequence SEQ ID NO: 134 or any variant thereof, and the light chain variable region of the amino acid sequence SEQ ID NO: 129 or any variant thereof;
preferably, comprising the heavy chain variable region of the amino acid sequence SEQ ID NO: 139 or any variant thereof, and the light chain variable region of the amino acid sequence SEQ ID NO: 142 or any variant thereof;
preferably, comprising the heavy chain variable region of the amino acid sequence SEQ ID NO: 147 or any variant thereof, and the light chain variable region of the amino acid sequence SEQ ID NO: 152 or any variant thereof;
preferably, comprising the heavy chain variable region of the amino acid sequence SEQ ID NO: 157 or any variant thereof, and the light chain variable region of the amino acid sequence SEQ ID NO: 159 or any variant thereof;
preferably, comprising the heavy chain variable region of the amino acid sequence SEQ ID NO: 163 or any variant thereof, and the light chain variable region of the amino acid sequence SEQ ID NO: 166 or any variant thereof;
preferably, comprising the heavy chain variable region of the amino acid sequence SEQ ID NO: 169 or any variant thereof, and the light chain variable region of the amino acid sequence SEQ ID NO: 171 or any variant thereof;
preferably, comprising the heavy chain variable region of the amino acid sequence SEQ ID NO: 181 or any variant thereof, and the light chain variable region of the amino acid sequence SEQ ID NO: 214 or any variant thereof;
preferably, comprising the heavy chain variable region of the amino acid sequence SEQ ID NO: 184 or any variant thereof, and the light chain variable region of the amino acid sequence SEQ ID NO: 214 or any variant thereof;
preferably, comprising the heavy chain variable region of the amino acid sequence SEQ ID NO: 186 or any variant thereof, and the light chain variable region of the amino acid sequence SEQ ID NO: 214 or any variant thereof;
preferably, comprising the heavy chain variable region of the amino acid sequence SEQ ID NO: 188 or any variant thereof, and the light chain variable region of the amino acid sequence SEQ ID NO: 214 or any variant thereof;
preferably, comprising the heavy chain variable region of the amino acid sequence SEQ ID NO: 190 or any variant thereof, and the light chain variable region of the amino acid sequence SEQ ID NO: 214 or any variant thereof;
preferably, comprising the heavy chain variable region of the amino acid sequence SEQ ID NO: 193 or any variant thereof, and the light chain variable region of the amino acid sequence SEQ ID NO: 214 or any variant thereof;
preferably, comprising the heavy chain variable region of the amino acid sequence SEQ ID NO: 195 or any variant thereof, and the light chain variable region of the amino acid sequence SEQ ID NO: 214 or any variant thereof;
preferably, comprising the heavy chain variable region of the amino acid sequence SEQ ID NO: 197 or any variant thereof, and the light chain variable region of the amino acid sequence SEQ ID NO: 214 or any variant thereof;
preferably, comprising the heavy chain variable region of the amino acid sequence SEQ ID NO: 175 or any variant thereof, and the light chain variable region of the amino acid sequence SEQ ID NO: 199 or any variant thereof;
preferably, comprising the heavy chain variable region of the amino acid sequence SEQ ID NO: 175 or any variant thereof, and the light chain variable region of the amino acid sequence SEQ ID NO: 203 or any variant thereof;
preferably, comprising the heavy chain variable region of the amino acid sequence SEQ ID NO: 175 or any variant thereof, and the light chain variable region of the amino acid sequence SEQ ID NO: 205 or any variant thereof;
preferably, comprising the heavy chain variable region of the amino acid sequence SEQ ID NO: 177 or any variant thereof, and the light chain variable region of the amino acid sequence SEQ ID NO: 205 or any variant thereof;
preferably, comprising the heavy chain variable region of the amino acid sequence SEQ ID NO: 177 or any variant thereof, and the light chain variable region of the amino acid sequence SEQ ID NO: 199 or any variant thereof;
preferably, comprising the heavy chain variable region of the amino acid sequence SEQ ID NO: 177 or any variant thereof, and the light chain variable region of the amino acid sequence SEQ ID NO: 203 or any variant thereof;
preferably, comprising the heavy chain variable region of the amino acid sequence SEQ ID NO: 179 or any variant thereof, and the light chain variable region of the amino acid sequence SEQ ID NO: 199 or any variant thereof;
preferably, comprising the heavy chain variable region of the amino acid sequence SEQ ID NO: 179 or any variant thereof, and the light chain variable region of the amino acid sequence SEQ ID NO: 203 or any variant thereof;
preferably, comprising the heavy chain variable region of the amino acid sequence SEQ ID NO: 179 or any variant thereof, and the light chain variable region of the amino acid sequence SEQ ID NO: 205 or any variant thereof;
preferably, comprising the heavy chain variable region of the amino acid sequence SEQ ID NO: 175 or any variant thereof, and the light chain variable region of the amino acid sequence SEQ ID NO: 208 or any variant thereof;
preferably, comprising the heavy chain variable region of the amino acid sequence SEQ ID NO: 175 or any variant thereof, and the light chain variable region of the amino acid sequence SEQ ID NO: 210 or any variant thereof;
preferably, comprising the heavy chain variable region of the amino acid sequence SEQ ID NO: 175 or any variant thereof, and the light chain variable region of the amino acid sequence SEQ ID NO: 212 or any variant thereof;
preferably, comprising the heavy chain variable region of the amino acid sequence SEQ ID NO: 177 or any variant thereof, and the light chain variable region of the amino acid sequence SEQ ID NO: 208 or any variant thereof;
preferably, comprising the heavy chain variable region of the amino acid sequence SEQ ID NO: 177 or any variant thereof, and the light chain variable region of the amino acid sequence SEQ ID NO: 210 or any variant thereof;
preferably, comprising the heavy chain variable region of the amino acid sequence SEQ ID NO: 177 or any variant thereof, and the light chain variable region of the amino acid sequence SEQ ID NO: 212 or any variant thereof;
preferably, comprising the heavy chain variable region of the amino acid sequence SEQ ID NO: 179 or any variant thereof, and the light chain variable region of the amino acid sequence SEQ ID NO: 208 or any variant thereof;
preferably, comprising the heavy chain variable region of the amino acid sequence SEQ ID NO: 179 or any variant thereof, and the light chain variable region of the amino acid sequence SEQ ID NO: 210 or any variant thereof;
preferably, comprising the heavy chain variable region of the amino acid sequence SEQ ID NO: 179 or any variant thereof, and the light chain variable region of the amino acid sequence SEQ ID NO: 212 or any variant thereof.

5. A bispecific or multispecific molecule, comprising the antibody or antigen-binding portion thereof that binds to MASP-2 of any one of claims 1-4.

6. The antibody or antigen-binding portion thereof of any one of claims 1-4 or the bispecific or multispecific molecule of claim 5, wherein the antibody or antigen-binding portion thereof is humanized.

7. A nucleic acid encoding the antibody or antigen-binding portion thereof of any one of claims 1-4, the bispecific or multispecific molecule of claim 5, or the antibody or antigen-binding portion thereof or the bispecific or multispecific molecule of claim 6; preferably, the nucleic acid comprises an antibody heavy chain variable region nucleotide sequence selected from the group consisting of SEQ ID NOs: 6, 16, 25, 35, 45, 54, 59, 64, 74, 78, 84, 93, 103, 112, 120, 125, 135, 140, 148, 158, 164, 170, 176, 178, 180, 182, 185, 187, 189, 191, 194, 196, 198 or any variant thereof, and/or an antibody light chain variable region nucleotide sequence selected from the group consisting of SEQ ID NOs: 11, 20, 30, 40, 49, 56, 62, 69, 76, 81, 88, 90, 98, 107, 116, 122, 130, 143, 153, 160, 167, 172, 200, 204, 206, 209, 211, 213, 215 or any variant thereof;
more preferably, the nucleic acid comprises the heavy chain variable region nucleotide sequence of the nucleotide sequence SEQ ID NO: 6 or any variant thereof, and the light chain variable region nucleotide sequence of the nucleotide sequence SEQ ID NO: 11 or any variant thereof;
more preferably, the nucleic acid comprises the heavy chain variable region nucleotide sequence of the nucleotide sequence SEQ ID NO: 16 or any variant thereof, and the light chain variable region nucleotide sequence of the nucleotide sequence SEQ ID NO: 20 or any variant thereof;
more preferably, the nucleic acid comprises the heavy chain variable region nucleotide sequence of the nucleotide sequence SEQ ID NO: 25 or any variant thereof, and the light chain variable region nucleotide sequence of the nucleotide sequence SEQ ID NO: 30 or any variant thereof;
more preferably, the nucleic acid comprises the heavy chain variable region nucleotide sequence of the nucleotide sequence SEQ ID NO: 35 or any variant thereof, and the light chain variable region nucleotide sequence of the nucleotide sequence SEQ ID NO: 40 or any variant thereof;
more preferably, the nucleic acid comprises the heavy chain variable region nucleotide sequence of the nucleotide sequence SEQ ID NO: 45 or any variant thereof, and the light chain variable region nucleotide sequence of the nucleotide sequence SEQ ID NO: 49 or any variant thereof;
more preferably, the nucleic acid comprises the heavy chain variable region nucleotide sequence of the nucleotide sequence SEQ ID NO: 54 or any variant thereof, and the light chain variable region nucleotide sequence of the nucleotide sequence SEQ ID NO: 56 or any variant thereof;
more preferably, the nucleic acid comprises the heavy chain variable region nucleotide sequence of the nucleotide sequence SEQ ID NO: 59 or any variant thereof, and the light chain variable region nucleotide sequence of the nucleotide sequence SEQ ID NO: 62 or any variant thereof;
more preferably, the nucleic acid comprises the heavy chain variable region nucleotide sequence of the nucleotide sequence SEQ ID NO: 64 or any variant thereof, and the light chain variable region nucleotide sequence of the nucleotide sequence SEQ ID NO: 69 or any variant thereof;
more preferably, the nucleic acid comprises the heavy chain variable region nucleotide sequence of the nucleotide sequence SEQ ID NO: 74 or any variant thereof, and the light chain variable region nucleotide sequence of the nucleotide sequence SEQ ID NO: 76 or any variant thereof;
more preferably, the nucleic acid comprises the heavy chain variable region nucleotide sequence of the nucleotide sequence SEQ ID NO: 78 or any variant thereof, and the light chain variable region nucleotide sequence of the nucleotide sequence SEQ ID NO: 81 or any variant thereof;
more preferably, the nucleic acid comprises the heavy chain variable region nucleotide sequence of the nucleotide sequence SEQ ID NO: 84 or any variant thereof, and the light chain variable region nucleotide sequence of the nucleotide sequence SEQ ID NO: 88 or any variant thereof;
more preferably, the nucleic acid comprises the heavy chain variable region nucleotide sequence of the nucleotide sequence SEQ ID NO: 84 or any variant thereof, and the light chain variable region nucleotide sequence of the nucleotide sequence SEQ ID NO: 90 or any variant thereof;
more preferably, the nucleic acid comprises the heavy chain variable region nucleotide sequence of the nucleotide sequence SEQ ID NO: 93 or any variant thereof, and the light chain variable region nucleotide sequence of the nucleotide sequence SEQ ID NO: 98 or any variant thereof;
more preferably, the nucleic acid comprises the heavy chain variable region nucleotide sequence of the nucleotide sequence SEQ ID NO: 103 or any variant thereof, and the light chain variable region nucleotide sequence of the nucleotide sequence SEQ ID NO: 107 or any variant thereof;
more preferably, the nucleic acid comprises the heavy chain variable region nucleotide sequence of the nucleotide sequence SEQ ID NO: 112 or any variant thereof, and the light chain variable region nucleotide sequence of the nucleotide sequence SEQ ID NO: 116 or any variant thereof;
more preferably, the nucleic acid comprises the heavy chain variable region nucleotide sequence of the nucleotide sequence SEQ ID NO: 120 or any variant thereof, and the light chain variable region nucleotide sequence of the nucleotide sequence SEQ ID NO: 122 or any variant thereof;
more preferably, the nucleic acid comprises the heavy chain variable region nucleotide sequence of the nucleotide sequence SEQ ID NO: 125 or any variant thereof, and the light chain variable region nucleotide sequence of the nucleotide sequence SEQ ID NO: 130 or any variant thereof;
more preferably, the nucleic acid comprises the heavy chain variable region nucleotide sequence of the nucleotide sequence SEQ ID NO: 135 or any variant thereof, and the light chain variable region nucleotide sequence of the nucleotide sequence SEQ ID NO: 130 or any variant thereof;
more preferably, the nucleic acid comprises the heavy chain variable region nucleotide sequence of the nucleotide sequence SEQ ID NO: 140 or any variant thereof, and the light chain variable region nucleotide sequence of the nucleotide sequence SEQ ID NO: 143 or any variant thereof;
more preferably, the nucleic acid comprises the heavy chain variable region nucleotide sequence of the nucleotide sequence SEQ ID NO: 148 or any variant thereof, and the light chain variable region nucleotide sequence of the nucleotide sequence SEQ ID NO: 153 or any variant thereof;
more preferably, the nucleic acid comprises the heavy chain variable region nucleotide sequence of the nucleotide sequence SEQ ID NO: 158 or any variant thereof, and the light chain variable region nucleotide sequence of the nucleotide sequence SEQ ID NO: 160 or any variant thereof;
more preferably, the nucleic acid comprises the heavy chain variable region nucleotide sequence of the nucleotide sequence SEQ ID NO: 164 or any variant thereof, and the light chain variable region nucleotide sequence of the nucleotide sequence SEQ ID NO: 167 or any variant thereof;
more preferably, the nucleic acid comprises the heavy chain variable region nucleotide sequence of the nucleotide sequence SEQ ID NO: 170 or any variant thereof, and the light chain variable region nucleotide sequence of the nucleotide sequence SEQ ID NO: 172 or any variant thereof;
more preferably, the nucleic acid comprises the heavy chain variable region nucleotide sequence of the nucleotide sequence SEQ ID NO: 182 or any variant thereof, and the light chain variable region nucleotide sequence of the nucleotide sequence SEQ ID NO: 215 or any variant thereof;
more preferably, the nucleic acid comprises the heavy chain variable region nucleotide sequence of the nucleotide sequence SEQ ID NO: 185 or any variant thereof, and the light chain variable region nucleotide sequence of the nucleotide sequence SEQ ID NO: 215 or any variant thereof;
more preferably, the nucleic acid comprises the heavy chain variable region nucleotide sequence of the nucleotide sequence SEQ ID NO: 187 or any variant thereof, and the light chain variable region nucleotide sequence of the nucleotide sequence SEQ ID NO: 215 or any variant thereof;
more preferably, the nucleic acid comprises the heavy chain variable region nucleotide sequence of the nucleotide sequence SEQ ID NO: 189 or any variant thereof, and the light chain variable region nucleotide sequence of the nucleotide sequence SEQ ID NO: 215 or any variant thereof;
more preferably, the nucleic acid comprises the heavy chain variable region nucleotide sequence of the nucleotide sequence SEQ ID NO: 191 or any variant thereof, and the light chain variable region nucleotide sequence of the nucleotide sequence SEQ ID NO: 215 or any variant thereof;
more preferably, the nucleic acid comprises the heavy chain variable region nucleotide sequence of the nucleotide sequence SEQ ID NO: 194 or any variant thereof, and the light chain variable region nucleotide sequence of the nucleotide sequence SEQ ID NO: 215 or any variant thereof;
more preferably, the nucleic acid comprises the heavy chain variable region nucleotide sequence of the nucleotide sequence SEQ ID NO: 196 or any variant thereof, and the light chain variable region nucleotide sequence of the nucleotide sequence SEQ ID NO: 215 or any variant thereof;
more preferably, the nucleic acid comprises the heavy chain variable region nucleotide sequence of the nucleotide sequence SEQ ID NO: 198 or any variant thereof, and the light chain variable region nucleotide sequence of the nucleotide sequence SEQ ID NO: 215 or any variant thereof;
more preferably, the nucleic acid comprises the heavy chain variable region nucleotide sequence of the nucleotide sequence SEQ ID NO: 176 or any variant thereof, and the light chain variable region nucleotide sequence of the nucleotide sequence SEQ ID NO: 200 or any variant thereof;
more preferably, the nucleic acid comprises the heavy chain variable region nucleotide sequence of the nucleotide sequence SEQ ID NO: 176 or any variant thereof, and the light chain variable region nucleotide sequence of the nucleotide sequence SEQ ID NO: 204 or any variant thereof;
more preferably, the nucleic acid comprises the heavy chain variable region nucleotide sequence of the nucleotide sequence SEQ ID NO: 176 or any variant thereof, and the light chain variable region nucleotide sequence of the nucleotide sequence SEQ ID NO: 206 or any variant thereof;
more preferably, the nucleic acid comprises the heavy chain variable region nucleotide sequence of the nucleotide sequence SEQ ID NO: 178 or any variant thereof, and the light chain variable region nucleotide sequence of the nucleotide sequence SEQ ID NO: 206 or any variant thereof;
more preferably, the nucleic acid comprises the heavy chain variable region nucleotide sequence of the nucleotide sequence SEQ ID NO: 178 or any variant thereof, and the light chain variable region nucleotide sequence of the nucleotide sequence SEQ ID NO: 200 or any variant thereof;
more preferably, the nucleic acid comprises the heavy chain variable region nucleotide sequence of the nucleotide sequence SEQ ID NO: 178 or any variant thereof, and the light chain variable region nucleotide sequence of the nucleotide sequence SEQ ID NO: 204 or any variant thereof;
more preferably, the nucleic acid comprises the heavy chain variable region nucleotide sequence of the nucleotide sequence SEQ ID NO: 180 or any variant thereof, and the light chain variable region nucleotide sequence of the nucleotide sequence SEQ ID NO: 200 or any variant thereof;
more preferably, the nucleic acid comprises the heavy chain variable region nucleotide sequence of the nucleotide sequence SEQ ID NO: 180 or any variant thereof, and the light chain variable region nucleotide sequence of the nucleotide sequence SEQ ID NO: 204 or any variant thereof;
more preferably, the nucleic acid comprises the heavy chain variable region nucleotide sequence of the nucleotide sequence SEQ ID NO: 180 or any variant thereof, and the light chain variable region nucleotide sequence of the nucleotide sequence SEQ ID NO: 206 or any variant thereof;
more preferably, the nucleic acid comprises the heavy chain variable region nucleotide sequence of the nucleotide sequence SEQ ID NO: 176 or any variant thereof, and the light chain variable region nucleotide sequence of the nucleotide sequence SEQ ID NO: 209 or any variant thereof;
more preferably, the nucleic acid comprises the heavy chain variable region nucleotide sequence of the nucleotide sequence SEQ ID NO: 176 or any variant thereof, and the light chain variable region nucleotide sequence of the nucleotide sequence SEQ ID NO: 211 or any variant thereof;
more preferably, the nucleic acid comprises the heavy chain variable region nucleotide sequence of the nucleotide sequence SEQ ID NO: 176 or any variant thereof, and the light chain variable region nucleotide sequence of the nucleotide sequence SEQ ID NO: 213 or any variant thereof;
more preferably, the nucleic acid comprises the heavy chain variable region nucleotide sequence of the nucleotide sequence SEQ ID NO: 178 or any variant thereof, and the light chain variable region nucleotide sequence of the nucleotide sequence SEQ ID NO: 209 or any variant thereof;
more preferably, the nucleic acid comprises the heavy chain variable region nucleotide sequence of the nucleotide sequence SEQ ID NO: 178 or any variant thereof, and the light chain variable region nucleotide sequence of the nucleotide sequence SEQ ID NO: 211 or any variant thereof;
more preferably, the nucleic acid comprises the heavy chain variable region nucleotide sequence of the nucleotide sequence SEQ ID NO: 178 or any variant thereof, and the light chain variable region nucleotide sequence of the nucleotide sequence SEQ ID NO: 213 or any variant thereof;
more preferably, the nucleic acid comprises the heavy chain variable region nucleotide sequence of the nucleotide sequence SEQ ID NO: 180 or any variant thereof, and the light chain variable region nucleotide sequence of the nucleotide sequence SEQ ID NO: 209 or any variant thereof;
more preferably, the nucleic acid comprises the heavy chain variable region nucleotide sequence of the nucleotide sequence SEQ ID NO: 180 or any variant thereof, and the light chain variable region nucleotide sequence of the nucleotide sequence SEQ ID NO: 211 or any variant thereof;
more preferably, the nucleic acid comprises the heavy chain variable region nucleotide sequence of the nucleotide sequence SEQ ID NO: 180 or any variant thereof, and the light chain variable region nucleotide sequence of the nucleotide sequence SEQ ID NO: 213 or any variant thereof.

8. A vector, comprising the nucleic acid of claim 7.

9. A cell, comprising the nucleic acid of claim 7 or the vector of claim 8.

10. A composition, comprising the antibody or antigen-binding portion thereof of any one of claims 1-4, the bispecific or multispecific molecule of claim 5, the antibody or antigen-binding portion thereof or the bispecific or multispecific molecule of claim 6, the nucleic acid of claim 7, the vector of claim 8 and/or the cell of claim 9.

11. The composition of claim 10, further comprising one or more additional therapeutic agents; the additional therapeutic agents being selected from the group consisting of anti-inflammatory and/or analgesic agents, anti-restenosis agents, other complement inhibitors, chondroprotective agents, and/or spasm inhibitors.

12. A kit, comprising the antibody or antigen-binding portion thereof of any one of claims 1-4, the bispecific or multispecific molecule of claim 5, the antibody or antigen-binding portion thereof or the bispecific or multispecific molecule of claim 6, the nucleic acid molecule of claim 7, the vector of claim 8, the cell of claim 9 and/or the composition of claim 10 or 11.

13. Use of the antibody or antigen-binding portion thereof of any one of claims 1-4, the bispecific or multispecific molecule of claim 5, the antibody or antigen-binding portion thereof or the bispecific or multispecific molecule of claim 6, the nucleic acid of claim 7, the vector of claim 8, the cell of claim 9 and/or the composition of claim 10 or 11 in the preparation of a drug or kit for diagnosing, treating or preventing diseases associated with lectin pathway of complement activation;
preferably, the diseases associated with MASP-2 dependent complement activation are selected from the group consisting of MASP-2 dependent complement-mediated vascular conditions, ischemia-reperfusion injury, atherosclerosis, inflammatory gastrointestinal tract disorders, lung conditions, in vitro reperfusion processes, skeletal muscle conditions, kidney conditions, skin conditions, organ or tissue transplantation, neurological disorders or injuries, blood disorders, urogenital tract conditions, diabetes, chemotherapy or radiation therapy, malignancies, endocrine disorders, coagulation disorders, or ophthalmic conditions; injuries and diseases related to complement activation caused by bacterial, fungal or viral infections;
preferably, the diseases associated with MASP-2 dependent complement activation are autoimmune inflammatory conditions or autoimmune diseases; preferably, the autoimmune inflammatory conditions are selected from the group consisting of multiple sclerosis (myelin coated on neurites), type I diabetes (pancreas), Hashimoto's thyroiditis (thyroid), pernicious anemia (stomach), Addison' s disease (adrenal gland), myasthenia gravis (acetylcholine receptors on neuromuscular junctions), rheumatoid arthritis, uveitis (eye), psoriasis (skin), Guillain-Barre syndrome (nerve cells), Grave's disease (thyroid), systemic lupus erythematosus, glomeronephritis, dermatomyositis, asthma, eczema, atopicaldermatitis, contact dermatitis, other eczematous dermatitises, seborrheic dermatitis, rhinitis, lichen planus, pemplugus, bullous pemphigoid, epidermolysis bullosa, urticaria, angioedema, vasculitis, erythema, cutaneous eosinophilia, alopecia areata, arteriosclerosis, primary biliary cirrhosis and nephrotic syndrome,
preferably, the diseases associated with MASP-2 dependent complement activation are selected from the group consisting of enteritis, such as coeliac disease, proctitis, eosinophilic gastroenteritis, mastocytosis, inflammatory bowel disease, Chrohn's disease, ulcerative colitis, and food-related allergies.
